(19) Europäisches Patentamt
European Patent Office
Office européen des brevets

(11) EP 4 056 558 A1

# (12) EUROPEAN PATENT APPLICATION
published in accordance with Art. 153(4) EPC

(43) Date of publication:
**14.09.2022 Bulletin 2022/37**

(21) Application number: **20884968.7**

(22) Date of filing: **04.11.2020**

(51) International Patent Classification (IPC):
***C07D 231/56** (2006.01)* ***C07D 471/00** (2006.01)*
***C07D 471/04** (2006.01)* ***C07D 487/04** (2006.01)*
***A61K 31/416** (2006.01)* ***A61P 35/00** (2006.01)*

(52) Cooperative Patent Classification (CPC):
**A61K 31/416; A61P 35/00; C07D 231/56; C07D 471/00; C07D 471/04; C07D 487/04**

(86) International application number:
**PCT/CN2020/126448**

(87) International publication number:
**WO 2021/088859 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.11.2019 CN 201911077994**

(71) Applicant: **Jinan University**
**Tianhe District**
**Guangzhou**
**Guangdong 510632 (CN)**

(72) Inventors:
- **DING, Ke**
  **Guangzhou, Guangdong 510632 (CN)**
- **CHAN, Shingpan**
  **Guangzhou, Guangdong 510632 (CN)**
- **ZHANG, Zhang**
  **Guangzhou, Guangdong 510632 (CN)**
- **DUAN, Yunxin**
  **Guangzhou, Guangdong 510632 (CN)**
- **WANG, Jie**
  **Guangzhou, Guangdong 510632 (CN)**
- **REN, Xiaomei**
  **Guangzhou, Guangdong 510632 (CN)**
- **TU, Zhengchao**
  **Guangzhou, Guangdong 510632 (CN)**

(74) Representative: **Haseltine Lake Kempner LLP**
**Redcliff Quay**
**120 Redcliff Street**
**Bristol BS1 6HU (GB)**

# (54) INDAZOLE COMPOUND, PHARMACEUTICAL COMPOSITION OF SAME, AND APPLICATIONS THEREOF

(57) The present invention provides indazole derivatives or their pharmaceutically acceptable salts or stereoisomers having the structure shown in formula (I), and their pharmaceutical compositions and applications thereof. Such compounds can be used as protein kinase inhibitors, which can effectively inhibit the activity of tropomyosin receptor kinase (TRK) protein kinase and can inhibit the proliferation, migration and invasion of a variety of tumor cells.

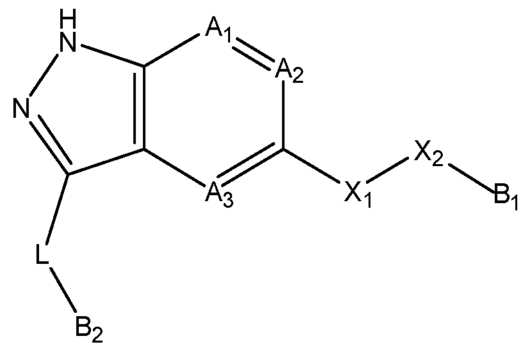

(I)

EP 4 056 558 A1

## Description

### Technical Field

The present disclosure relates to the technical field of chemical medicine, in particular to indazole compounds and their pharmaceutical composition and applications thereof.

### Background

Tropomyosin receptor kinase (TRK), which belongs to the receptor tyrosine kinase (RTK) family, has three subtypes TRKA, TRKB, and TRKC, which are encoded by NTRK1, NTRK2, and NTRK3 genes respectively. TRK is a class of transmembrane proteins consisting of an extracellular ligand-binding region, a transmembrane domain (TM) and an intracellular region, and activates mainly by binding neurotrophic factors (NTs). Neurotrophic factors are a class of protein molecules produced by nerve-innervated tissues (e.g., muscles) and astrocytes that are essential for neuronal growth and survival. At present, four major neurotrophic factors have been discovered, namely NGFs (nerve growth factors), brain-derived neurotrophic factors (BDNFs), neurotrophic factors 3 (NT-3) and neurotrophic factors 4 (NT-4), wherein NGF binds to TRKA, BDNF and NT-4 bind to TRKB, and NT-3 can bind to all three TRK proteins, but it binds more strongly to TRKC. When activated by signal induction, TRK activates downstream signaling pathways sequentially through self-dimerization and phosphorylation to achieve various cellular physiological functions. The downstream signaling pathways of TRK include MAPK, PI3K/AKT, and PLCγ/PKC pathways. These signaling pathways regulate cell proliferation, differentiation, migration, apoptosis and other physiological processes, as well as a variety of neuron-related physiological activities, such as neurosynaptic flexibility, neural dendrite growth and repair, prevention and repair of neuronal degradation and maintenance of sensory neurons.

Numerous studies have shown that TRK overexpression, gene fusions, and mononucleotide alterations are closely related to the occurrence and development of various types of tumors, such as non-small cell lung cancer, breast cancer, colon cancer, prostate cancer, thyroid cancer, malignant melanoma, neuroblastoma, and mammary analog secretory carcinoma. Among mechanisms of abnormal TRK activation, the most prevalent mechanism is the gene fusion of TRK. The earliest NTRK fusion gene discovered in medical research was the TPM3-NTRK1 fusion gene found in colon cancer samples. With the deepening of research, researchers successively discovered multiple types of fusion genes, such as CD74-NTRK1, ETV6-NTRK2, QKI-NTRK2, and ETV6-NTRK3. The TRK fusion proteins expressed by NTRK fusion genes can continuously activate downstream signaling pathways independent of binding to ligands, thereby inducing abnormal cell proliferation and promoting the occurrence and development of tumors. Therefore, TRK is considered to be an effective target for anticancer therapy.

At present, a selective TRK inhibitor, Larotrectinib, developed by LOXO Inc. in USA was approved by the FDA in 2018; and a TRK inhibitor, Entrectinib, developed by Roche Pharmaceuticals Inc. was launched in Japan in June 2019. Belizatinib developed by TESARO Inc. is undergoing clinical study. In addition, multi-target inhibitors such as Cabozanitinib, Sitravatinib and Altiratinib also have good TRK inhibitory activity.

The NTRK gene point mutation caused by continuous use of TRK inhibitors is the key factor of drug resistance in tumors. Clinical studies have successively discovered mutations on G595R, G667C, F589L, and G667S in NTRK1, and on G623R and G696A in NTRK3. There is currently no inhibitor targeting these mutations on the market, and the second-generation TRK inhibitors, LOXO-195, TPX-0005, and ONO-5390556 are under clinical investigation.

### Summary

In view of the above problems, the present disclosure provides an indazole derivative or its pharmaceutically acceptable salt or its stereoisomer, and such compounds can be used as protein kinase inhibitors, which can effectively inhibit the activity of TRK protein kinase and inhibit the proliferation, migration and invasion of a variety of tumor cells.

The specific technical solutions are as follows:

indazole derivatives or their pharmaceutically acceptable salts or their stereoisomers, having the structure shown in formula (I):

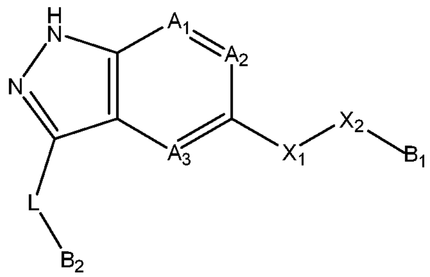

(I)

wherein,

0-2 of $A_1$, $A_2$, and $A_3$ are selected from N, and the rest are CH;

$X_1$ is selected from: $-(CR_1R_2)_n-$, -O-, -S-, -S(O)-, $-S(O_2)-$, -C(O)-, $-N(R_3)-$, -CH=CH-, -C=C-, $-C(O)N(R_3)-$, or none;

$X_2$ is selected from: $-(CR_1R_2)_n-$, -O-, -S-, -S(O)-, $-S(O_2)-$, -C(O)-, $-N(R_3)-$, -CH=CH-, -C=C-, $-C(O)N(R_3)-$, or none;

alternatively, $X_1$ and $X_2$ together form one or more $R_1$ substituted or unsubstituted $C_3$-$C_8$ cycloalkyl, or one or more $R_1$ substituted or unsubstituted $C_3$-$C_8$ heterocyclyl;

$B_1$ is selected from: one or more $R_4$ substituted or unsubstituted $C_6$-$C_{10}$ aryl, one or more $R_4$ substituted or unsubstituted $C_5$-$C_{10}$ heteroaryl;

L is selected from: -CH=CH-, -C=C-;

$B_2$ is selected from: one or more $R_5$ substituted or unsubstituted $C_6$-$C_{10}$ aryl, one or more $R_5$ substituted or unsubstituted $C_5$-$C_{10}$ heteroaryl, $-C(O)N(R_6R_7)$;

$R_1$ and $R_2$ are each independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl; when eithe $X_1$ or $X_2$ is selected from -O- and -S- and the other is selected from $-(CR_1R_2)_n-$, both $R_1$ and $R_2$ are hydrogen;

each $R_3$ is independently selected from: hydrogen and $C_1$-$C_6$ alkyl;

each $R_4$ is independently selected from: hydrogen, $C_1$-$C_6$ alkyl, cyano, halogen, halogenated $C_1$-$C_6$ alkyl, hydroxyl, nitro, amino, mercapto, $C_1$-$C_6$ alkoxy, halogenated $C_1$-$C_6$ alkoxy, $C_1$-$C_6$ alkylamino, $C_1$-$C_6$ alkylamido, $C_1$-$C_6$ alkylthio, $C_1$-$C_6$ alkoxy $C_1$-$C_6$ alkyl, $C_1$-$C_6$ alkylsulfonyl, $C_1$-$C_6$ alkylsulfonamido;

each $R_5$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$;

$R_6$ and $R_7$ are each independently selected from: hydrogen and $C_1$-$C_{10}$ alkyl, or $R_6$ and $R_7$ together with a nitrogen atom which they are attached to, form one or more $R_5$ substituted or unsubstituted 3-8 membered heterocyclyl;

each $R_5$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$; and

n is selected from: an integer between 1-8.

In some of the embodiments, both $A_1$ and $A_2$ are CH, and $A_3$ is N or CH.

In some of the embodiments, $A_1$ is N or CH, and both $A_2$ and $A_3$ are CH.

In some of the embodiments, $X_1$-$X_2$ formed by $X_1$ and $X_2$ together is selected from: $-(CR_1R_2)_n-$, -O-, -S-, -S(O)-, $-S(O_2)-$, -C(O)-, $-N(C_1$-$C_6$ alkyl)-, $-(CH_2)_n-O-$, $-O-(CH_2)_n-$, $-(CR_1R_2)_n-N(R_3)-$, $-N(R_3)-(CR_1R_2)_n-$, $-(CH_2)_n-S-$, -S-(CH_2)n-, $-S(O_2)N(R_3)-$, $-N(R_3)S(O_2)-$, $-N(R_3)C(O)N(R_3)-$, one or more $R_1$ substituted or unsubstituted $C_3$-$C_8$ cycloalkyl, and one or more $R_1$ substituted or unsubstituted $C_3$-$C_8$ heterocycloalkyl;

$R_1$ and $R_2$ are each independently selected from: hydrogen, $C_1$-$C_6$ alkyl;

each $R_3$ is independently selected from: H, $C_1$-$C_6$ alkyl;

n is selected from: an integer between 1-4.

In some of the embodiments, $X_1$-$X_2$ formed by $X_1$ and $X_2$ together is selected from: $-(CR_1R_2)_n-$, -O-, -S-, $-S(O_2)-$, $-N(C_1$-$C_3$ alkyl)-, $-(CH_2)_n-O-$, $-O-(CH_2)_n-$, $-(CR_1R_2)_n-N(R_3)-$, $-NR_3-(CR_1R_2)_n-$, $-S(O_2)N(R_3)-$, $-N(R_3)S(O_2)$, and $C_5$-$C_6$ heterocycloalkyl;

$R_1$ and $R_2$ are each independently selected from: hydrogen, $C_1$-$C_3$ alkyl;

each $R_3$ is independently selected from: H, $C_1$-$C_3$ alkyl;

n is selected from: an integer between 1-2.

In some of the embodiments, $X_1$-$X_2$ formed by $X_1$ and $X_2$ together is selected from: $-CH_2-$, -O-, -S-, $-S(O_2)-$, $-OCH_2-$, $-N(CH_3)-$, $-CH_2O-$, $-CH_2NH-$, $-CH_2N(CH_3)-$, $-NH-S(O_2)-$, $-S(O_2)-NH-$, $-NHCH_2-$, $-NHCH(CH_3)-$, $-CH(CH_3)NH-$, $-N(CH_3)CH_2-$, $-CH_2CH_2-$,

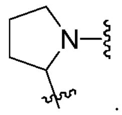

.

In some of the embodiments, $X_1$-$X_2$ formed by $X_1$ and $X_2$ together is selected from: $-S(O_2)-$, $-CH_2CH_2-$; or $X_1$ is -O- and $X_2$ is $CH_2$-.

In some of the embodiments, $X_1$ is -NH- and $X_2$ is selected from: $-S(O_2)-$, $-CH_2-$, $-CH(CH_3)-$.

In some of the embodiments, the indazole derivative hasthe structure shown in formula (II) or formula (III).

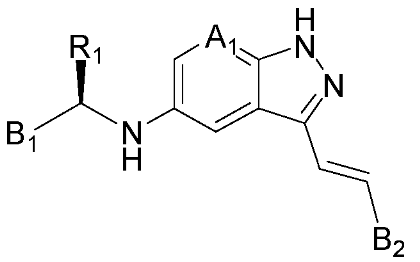

(II)

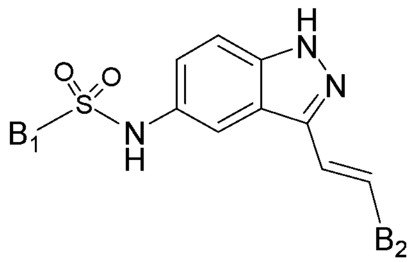

(III).

In some of the embodiments, $B_1$ is selected from: one or more $R_4$ substituted or unsubstituted phenyl, one or more $R_4$ substituted or unsubstituted pyridinyl, one or more $R_4$ substituted or unsubstituted pyrimidinyl, one or more $R_4$ substituted or unsubstituted naphthyl, one or more $R_4$ substituted or unsubstituted quinolinyl, one or more $R_4$ substituted or unsubstituted pyrazolyl, one or more $R_4$ substituted or unsubstituted pyrrolyl, one or more $R_4$ substituted or unsubstituted thienyl, one or more $R_4$ substituted or unsubstituted furanyl, one or more $R_4$ substituted or unsubstituted pyrazinyl.

In some of the embodiments, each $R_4$ is independently selected from: hydrogen, $C_1$-$C_3$ alkyl, halogen, halogenated $C_1$-$C_3$ alkyl, cyano, $C_1$-$C_3$ alkoxy, $C_1$-$C_4$ alkyl sulfonyl, $C_1$-$C_3$ alkylsulfonamido.

In some of the embodiments, each $R_4$ is independently selected from: hydrogen, halogen, nitro, amino, hydroxyl, mercapto, trifluoromethyl, cyano, formamido, methylamino, trifluoromethoxy, difluoromethoxy, difluoromethyl, methyl, ethyl, n-propyl, isopropyl, n-butyl, isobutyl, tert-butyl, n-pentyl, isopentyl, neopentyl, methoxy, ethoxy, isopropoxy, tert-butoxy, methoxyethyl, ethoxyethyl, methylthio, ethylthio, isopropylthio, tert-butylthio, methylsulfonyl, ethylsulfonyl, isopropylsulfonyl, tert-butyl sulfonyl, methylsulfonamido.

In some of the embodiments, $B_1$ is selected from:

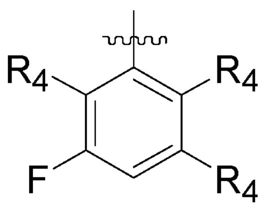

,

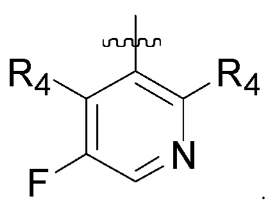

.

In some of the embodiments, $B_1$ is selected from:

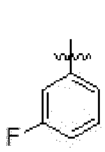

,

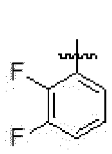

,

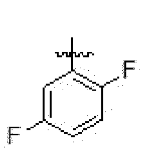

,

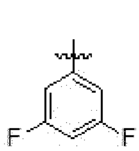

,

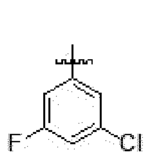

,

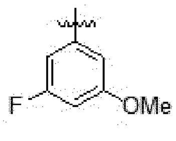, 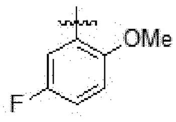, 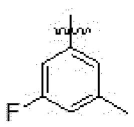, 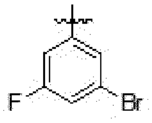, 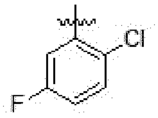,

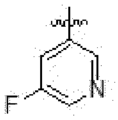, 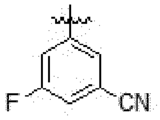, 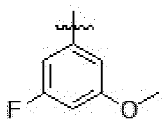, 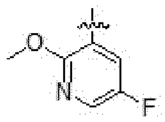, 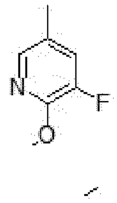,

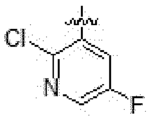, 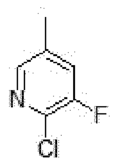

In some of the embodiments, $B_2$ is selected from: one or more $R_5$ substituted or unsubstituted phenyl, one or more $R_5$ substituted or unsubstituted naphthyl, one or more $R_5$ substituted or unsubstituted quinolinyl, one or more $R_5$ substituted or unsubstituted pyrazolyl, one or more $R_5$ substituted or unsubstituted pyrrolyl, one or more $R_5$ substituted or unsubstituted thienyl, one or more $R_5$ substituted or unsubstituted furanyl, one or more $R_5$ substituted or unsubstituted pyridyl, one or more $R_5$ substituted or unsubstituted pyrimidinyl, one or more $R_5$ substituted or unsubstituted pyrazinyl, one or more $R_5$ substituted or unsubstituted triazolyl, $-C(O)NR_6R_7$.

In some of the embodiments, $B_2$ is selected from:

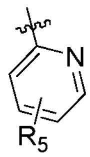, 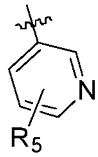, 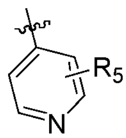, 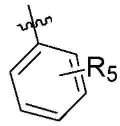, 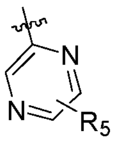, 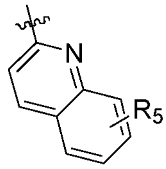, 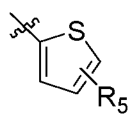, 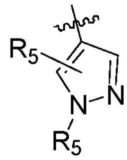,

$-C(O)NR_6R_7$.

In some of the embodiments, $B_2$ is selected from:

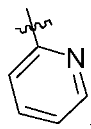, 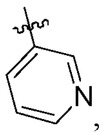, 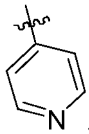,

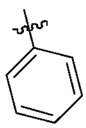, 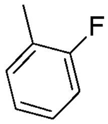, 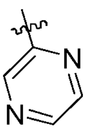, 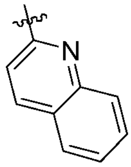, 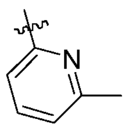, 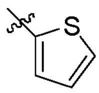, 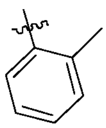, 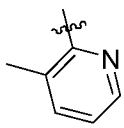,

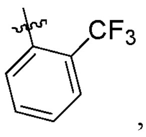, 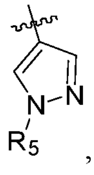,

$-C(O)NR_6R_7$.

In some of the embodiments, each $R_5$ is independently selected from: hydrogen, $C_1$-$C_3$ alkyl, halogen, halogenated $C_1$-$C_3$ alkyl, cyano, $C_1$-$C_3$ alkoxy, hydroxyl-substituted $C_1$-$C_3$ alkyl, cyano-substituted $C_1$-$C_3$ alkyl, $C_1$-$C_3$ alkoxy $C_1$-$C_3$ alkyl, -C(O)-$C(R_3)_3$, cyclopropyl, $N(R_3)_2C(O)$-$(C(R_3)_2)_n$-, wherein each $R_3$ is independently selected from: H, $C_1$-$C_3$ alkyl.

In some of the embodiments, each $R_5$ is independently selected from: hydrogen, hydroxyl-substituted $C_1$-$C_3$ alkyl, $C_1$-$C_3$ alkyl, cyano-substituted $C_1$-$C_3$ alkyl, cyclopropyl, $C_1$-$C_3$ alkoxy $C_1$-$C_3$ alkyl.

In some of the embodiments, each $R_5$ is independently selected from: hydrogen, hydroxyl, amino, mercapto, halogen, trifluoromethyl, cyano, trifluoromethoxy, difluoromethoxy, methyl, ethyl, n-propyl, isopropyl, n-butyl, isobutyl, tert-butyl, n-pentyl, isopentyl, neopentyl, methoxy, ethoxy, isopropoxy, tert-butoxy, methylthio, ethylthio, isopropylthio, tert-butylthio, methoxyethyl, ethoxyethyl, hydroxylmethyl, hydroxylethyl, hydroxylpropyl, cyano-substituted methyl, cyano-substituted ethyl, acetyl, 2,2,2-trifluoroethyl, cyclopropyl, 2,2-difluoroethyl, N,N-dimethylacetamido, difluoromethyl, isopropoxy-substituted ethyl, N-methylacetamido.

In some of the embodiments, $R_6$ and $R_7$ are each independently selected from: hydrogen, and $C_1$-$C_6$ alkyl, or $R_6$ and $R_7$ together with a nitrogen atom, which they are attached to, form one or more $R_8$ substituted or unsubstituted 4-8 membered heterocyclyl.

In some of the embodiments, $R_6$ and $R_7$ are each independently selected from: hydrogen and $C_1$-$C_3$ alkyl, or $R_6$ and $R_7$ together with a nitrogen atom, which they are attached to, form heterocyclyl with following structure :

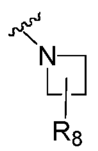 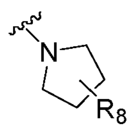 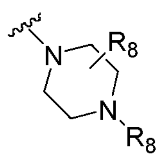 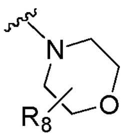 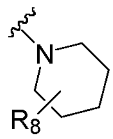 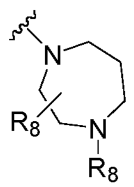 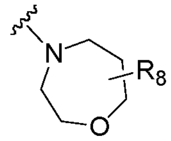 ;

wherein each $R_8$ is independently selected from: hydrogen, hydroxyl, $C_1$-$C_3$ alkyl, halogen, halogenated $C_1$-$C_3$ alkyl, $C_1$-$C_3$ alkoxy.

In some of the embodiments, $R_6$ and $R_7$ are each independently selected from: hydrogen and methyl, or $R_6$ and $R_7$ with a nitrogen atom, which they are attached to, form heterocyclyl with following structure:

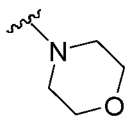, 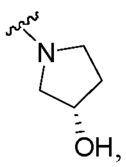, 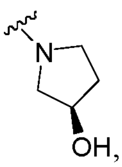, 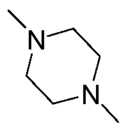.

In some of the embodiments, the indazole derivative has the structure shown in formula (II).

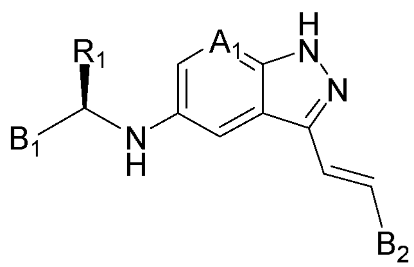

(II)

wherein $A_1$ is selected from: CH, N;

$R_1$ is selected from: H and methyl;

$B_1$ is selected from:

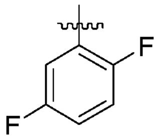, 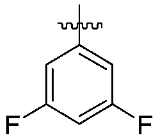, 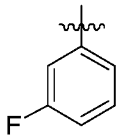, 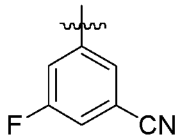,

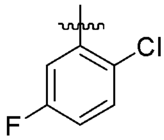, 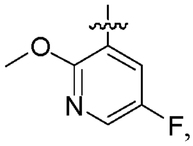, 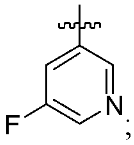;

$B_2$ is selected from:

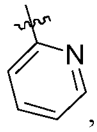, 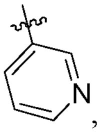, 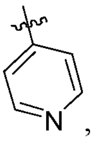, 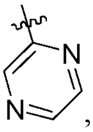, 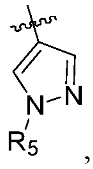,

$-C(O)NR_6R_7$;

$R_5$ is selected from: hydrogen, methyl, ethyl, hydroxylethyl;

$R_6$ and $R_7$ are each independently selected from: hydrogen and methyl, or $R_6$ and $R_7$ with a nitrogen atom, which they are attached to, form heterocyclyl with following structure:

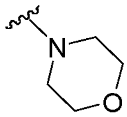, 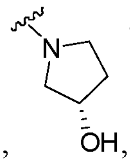, 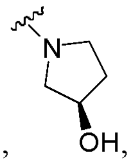, 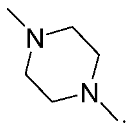.

In some of the embodiments, the indazole derivative has the structure shown in formula (III).

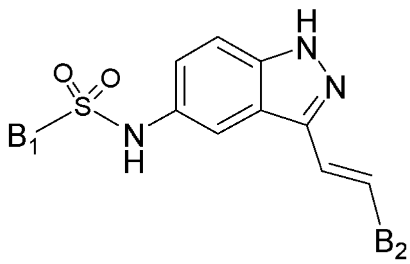

(III)

$B_1$ is selected from:

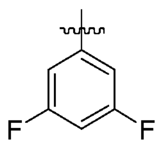, 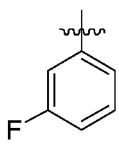, 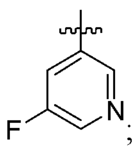;

$B_2$ is selected from:

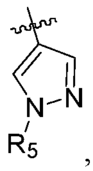, 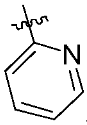, 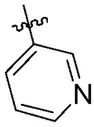;

$R_5$ is selected from: hydrogen, cyano-substituted methyl, acetyl, 2,2,2-trifluoroethyl, cyclopropyl, 2,2-difluoroethyl, isopropyl, difluoromethyl, isopropoxyethyl, methoxyethyl, hydroxylethyl, methyl, ethyl.

The present disclosure also provides applications of the above indazole derivatives or their pharmaceutically acceptable salts or stereoisomers.

The specific technical solutions are as follows.

Provided is an application of the above indazole derivatives or their pharmaceutically acceptable salts or their stereoisomers in the preparation of tropomyosin receptor kinase (TRK) inhibitors.

Provided is an application of the above indazole derivatives or their pharmaceutically acceptable salts or stereoisomers in the manufacture of drug mediated by tropomyosin receptor kinase (TRK) tyrosine kinases for preventing or treating diseases.

In some of the embodiments, the diseases mediated by the TRK tyrosine kinases are tumors, preferably non-small cell lung cancer, breast cancer, colon cancer, prostate cancer, thyroid cancer, malignant melanoma, neuroblastoma, and mammary analog secretory carcinoma.

The present disclosure also provides pharmaceutical compositions for preventing or treating tumors.

The specific technical solutions are as follows.

Provided is pharmaceutical compositions for preventing or treating tumors. It's prepared from active ingredient and pharmaceutically acceptable excipient, wherein the active ingredient includes the said indazole derivatives or their pharmaceutically acceptable salt or their stereoisomers.

Based on the above technical solutions, the present disclosure has following beneficial effects.

After extensive and intensive research, the inventors of the present disclosure surprisingly developed a structurally novel indazole derivative. Such compounds have strong inhibitory activity against TRK tyrosine kinase. Thus, such compounds can be used as an effective inhibitor of TRK tyrosine kinase, and can be used to prevent or treat related diseases mediated by TRK tyrosine kinase, such as a variety of tumors (non-small cell lung cancer, breast cancer, colon cancer, prostate cancer, thyroid cancer, malignant melanoma, neuroblastoma and mammary analog secretory carcinoma).

## Detailed Description

In the compounds of the present disclosure, when any variable (e.g. R, etc.) occurs more than once in any component, its definition at each occurrence is independent of the definitions at each other occurrence. Similarly, combinations of substituents and variables are allowed as long as such combinations stabilize the compounds. A line drawn from a substituent into a ring system indicates that the bond referred to can be attached to any substitutable ring atom. If the ring system is polycyclic, it means that such bonds are attached only to any appropriate carbon atom adjacent to the ring. It is understood that a person of ordinary skill in the art may select the substituents and substitution patterns of the compounds of the present disclosure to provide compounds that are chemically stable and readily synthesized from readily available raw materials by the technology in the field and the methods set forth below. If the substituents themselves are substituted by more than one group, it is understood that these groups may be on the same carbon atom or on different carbon atoms, as long as the structure is stabilized.

As used herein, the phrases such as "$R_f$-substituted" and "R-substituted" are considered as equivalent to the phrases "substituted with at least one substituent", and in this case the preferred embodiments will have 1-4 substituents.

As used herein, the term "alkyl" is intended to include branched and linear saturated aliphatic hydrocarbyl having a specific number of carbon atoms. For example, the definition of "$C_1$-$C_6$" in "$C_1$-$C_6$ alkyl" includes groups having 1, 2, 3, 4, 5 or 6 carbon atoms in a linear or branched chain arrangement. For example, "$C_1$-$C_6$ alkyl" specifically includes methyl, ethyl, n-propyl, isopropyl, n-butyl, tert-butyl, isobutyl, pentyl and hexyl. The term "cycloalkyl" refers to a monocyclic saturated aliphatic hydrocarbyl having a specific number of carbon atoms. For example, "cycloalkyl" includes cyclopropyl, cyclobutyl, cyclopentyl or cyclohexyl, etc. The term "alkoxy" refers to a group having an -O-alkyl structure, such as -OCH3, $-OCH_2CH_3$, $-OCH_2CH_2CH_3$, $-O-CH_2CH(CH_3)_2$, $-OCH_2CH_2CH_2CH_3$, and $-O-CH(CH_3)_2$ and the like. The term "heterocyclyl" is a saturated or partially unsaturated monocyclic or polycyclic substituent, in which one or more ring atoms are selected from a heteroatom of N, O or S(O)m (wherein m is an integer from 0 to 2), and the remaining ring atoms are carbon, such as tetrahydropyrrolyl, etc. The term "heteroaryl" refers to an aromatic ring containing 1, 2 or 3

heteroatoms selected from O, N or S. Heteroaryl groups within the scope of the present disclosure include, but are not limited to: quinolinyl, pyrazolyl, pyrrolyl, thienyl, furanyl, pyridinyl, pyrimidinyl, pyrazinyl, triazolyl, imidazolyl, oxazolyl, isoxazolyl, and pyridazinyl. As understood by those skilled in the art, "halo" or "halogen" as used herein means chlorine, fluorine, bromine and iodine.

Provided are indazole derivatives or their pharmaceutically acceptable salts or stereoisomer having the structure shown in formula (I):

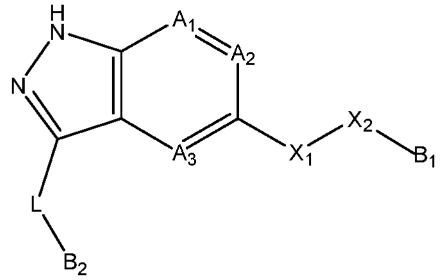

(I)

wherein,

0-2 of $A_1$-$A_3$ are selected from N and the rest are CH;
$X_1$ is selected from: $-(CR_1R_2)_n-$, -O-, -S-, -S(O)-, $-S(O_2)-$, -C(O)-, $-N(R_3)-$, -CH=CH-, -C=C-, $-C(O)N(R_3)-$, or none;
$X_2$ is selected from: $-(CR_1R_2)_n-$, -O-, -S-, -S(O)-, $-S(O_2)-$, -C(O)-, $-N(R_3)-$, -CH=CH-, -C=C-, $-C(O)N(R_3)-$, or none;
alternatively, $X_1$ and $X_2$ together form one or more $R_1$ substituted or unsubstituted $C_3$-$C_8$ cycloalkyl, or one or more $R_1$ substituted or unsubstituted $C_3$-$C_8$ heterocyclyl;
$B_1$ is selected from: one or more $R_4$ substituted or unsubstituted $C_6$-$C_{10}$ aryl, one or more $R_4$ substituted or unsubstituted $C_5$-$C_{10}$ heteroaryl;
L is selected from: -CH=CH-, -C=C-;
$B_2$ is selected from: one or more $R_5$ substituted or unsubstituted $C_6$-$C_{10}$ aryl, one or more $R_5$ substituted or unsubstituted $C_5$-$C_{10}$ heteroaryl $-C(O)N(R_6R_7)$;
$R_1$ and $R_2$ are each independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy, $C_1$-$C_6$ alkyl; when one of $X_1$ and $X_2$ is selected from -O-, -S-, and the other is selected from $-(CR_1R_2)_n-$, both $R_1$ and $R_2$ are hydrogen;
each $R_3$ is independently selected from: hydrogen $C_1$-$C_6$ alkyl.
each $R_4$ is independently selected from: hydrogen, $C_1$-$C_6$ alkyl, cyano, halogen, halogenated $C_1$-$C_6$ alkyl, hydroxyl, nitro, amino, mercapto, $C_1$-$C_6$ alkoxy, halogenated $C_1$-$C_6$ alkoxy, $C_1$-$C_6$ alkylamino, $C_1$-$C_6$ alkylamido, $C_1$-$C_6$ alkylthio, $C_1$-$C_6$ alkoxy $C_1$-$C_6$ alkyl, $C_1$-$C_6$ alkylsulfonyl, $C_1$-$C_6$ alkylsulfonamidyl;
each $R_5$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$;
$R_6$ and $R_7$ are each independently selected from: hydrogen, $C_1$-$C_{10}$ alkyl, or $R_6$ and $R_7$ together with a nitrogen atom connected thereto form one or more $R_8$ substituted or unsubstituted 3-8 membered heterocyclyl;
each $R_8$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$.

The present disclosure includes free forms of compounds of formula (I), formula (II) and formula (III), as well as their pharmaceutically acceptable salts and stereoisomers. Some of the specific exemplary compounds herein are protonated salts of amine compounds. The term "free form" refers to the amine compound in a non-salt form. The included pharmaceutically acceptable salts include not only exemplary salts of the specific compounds described herein, but also typical pharmaceutically acceptable salts of the free forms of all compounds of formula (I), formula (II) and formula (III). The free forms of specific salts of the compounds can be isolated by using techniques known in the art. For example, the free forms can be regenerated by treating the salt with an appropriate dilute aqueous base solution such as dilute aqueous NaOH, dilute aqueous potassium carbonate, dilute aqueous ammonia and dilute aqueous sodium bicarbonate. The free forms differ somewhat from their respective salt forms in certain physical properties, such as solubility in polar solvents, but for the purposes of the present disclosure such acid salts and alkaline salts are otherwise pharmaceutically equivalent to their respective free forms.

The pharmaceutically acceptable salts of the present disclosure can be synthesized from compounds of the present disclosure containing either a basic or acidic portion by conventional chemical methods. Typically, salts of basic compounds are prepared by ion exchange chromatography or by a reaction of a free base and a stoichiometric amount or excess of an inorganic or organic acid in the desired salt form in a suitable solvent or a combination of solvents. Similarly, salts of acidic compounds are formed by a reaction with a suitable inorganic or organic base.

Thus, pharmaceutically acceptable salts of the compounds of the present disclosure include conventional non-toxic salts of the compounds of the present disclosure formed by a reaction of a basic compound of the present disclosure with an inorganic or organic acid. For example, the conventional non-toxic salts include salts obtained from inorganic acids such as hydrochloric acid, hydrobromic acid, sulfuric acid, sulfamic acid, phosphoric acid, nitric acid, etc., as well as organic acids such as acetic acid, propionic acid, succinic acid, glycolic acid, stearic acid, lactic acid, malic acid, tartaric acid, citric acid, ascorbic acid, embonic acid, maleic acid, hydroxylmaleic acid, phenylacetic acid, glutamic acid, benzoic acid, salicylic acid, p-aminobenzenesulfonic acid, 2-acetoxybenzoic acid, fumaric acid, toluenesulfonic acid, methanesulfonic acid, ethanedisulfonic acid, oxalic acid, hydroxylethyl sulfonic acid, trifluoroacetic acid, etc.

If the compounds of the disclosure are acidic, the appropriate "pharmaceutically acceptable salt" refers to salts prepared from pharmaceutically acceptable non-toxic bases, including inorganic and organic bases. Salts obtained from inorganic bases include aluminum salts, ammonium salts, calcium salts, copper salts, iron salts, ferrous salts, lithium salts, magnesium salts, manganese salts, manganous salts, potassium salts, sodium salts, and zinc salts. Particularly preferred salts are ammonium salts, calcium salts, magnesium salts, potassium salts, and sodium salts. Salts obtained from pharmaceutically acceptable organic non-toxic bases include salts of primary amines, secondary amines and tertiary amines. The substituted amines include naturally occurring substituted amines, cyclic amines, and basic ion-exchanged resins, such as arginine, betaine, caffeine, choline, N,N'-dibenzyl ethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, aminoglucose, histidine, hydroxocobalamin, isopropylamine, lysine, methylglucosamine, morpholine, piperazine, piperidine, quazha, polyamine resin, procaine, purine, theobromine, triethylamine, trimethylamine, tripropylamine, and trometamol.

Berg et al, "Pharmaceutical Salts" J. Pharm. Sci. 1977: 66: 1-19 describes in more details about the preparation of the pharmaceutically acceptable salts described above, and the preperation of other typical pharmaceutically acceptable salts.

The deprotonated acidic portion of a compound such as carboxyl group can be anionic under physiological conditions. This charge can be counterbalanced by protonated or alkylated basic portion with cations inside, such as a tetravalent nitrogen atom. It should be noted that the compounds of the present disclosure are potential internal salts or amphoteric ions.

In one embodiment, the present application provides a method of treating hyperproliferative disease or condition such as tumor in human or other mammals by using the compounds with the structure shown in formula (I), formula (II), and formula (III), and their pharmaceutically acceptable salts.

In one embodiment, the compounds designed in the present application and their pharmaceutically acceptable salts can be used to treat or control non-small cell lung cancer, small cell lung cancer, lung adenocarcinoma, squamous lung cancer, pancreatic cancer, breast cancer, prostate cancer, liver cancer, skin cancer, epithelial cell cancer, gastrointestinal stromal tumor, endometrial cancer, histiocytic lymphoma, nasopharyngeal cancer, head and neck tumor, colon cancer, rectal cancer, glioma, malignant melanoma, kidney cancer, bladder cancer, ovarian cancer, cervical cancer, laryngeal cancer or multiple myeloma, B lymphoma, leukemia, hyperglycemia, diabetes mellitus, obesity, atherosclerosis, metabolic syndrome, liver fibrosis, nonalcoholic fatty liver, gallstones, hyperlipidemia, hypercholesterolemia, hyperlipoproteinemia, hypertriglyceridemia, hypertension, hyperinsulinemia, hyperuricemia, Parkinson's disease, Alzheimer's disease, etc.

## Pharmaceutical compositions

The disclosure also provides pharmaceutical compositions including active ingredients in a safe and effective amount of range, and pharmaceutically acceptable carriers.

The "active ingredients" described in the present disclosure include the compounds of formula (I), formula (II), and formula (III) described in the present disclosure.

The "active ingredients" and the pharmaceutical compositions described in the present disclosure can be used as TRK inhibitors. In another preferred example, the "active ingredients" and the pharmaceutical compositions are used in the manufacture of drugs for the prevention and/or treatment of tumors.

The "safe and effective amount" means that the amount of the active ingredient is sufficient to significantly improve the condition without causing serious side effects.

The "pharmaceutically acceptable carriers" means one or more compatible solid or liquid filler or gel substances that are suitable for human use, and must have enough purity and low toxicity.

"Compatibile" here means that each of components of the compositions can be blended with each other with the active ingredients in the present disclosure without significantly reducing the efficacy of the active ingredient.

Some examples of the pharmaceutically acceptable carriers are cellulose and its derivatives (e.g. sodium carboxymethyl cellulose, sodium ethyl cellulose, cellulose acetate, etc.), gelatin, talc, solid lubricants (e.g. stearic acid, magnesium stearate, etc.), calcium sulfate, vegetable oils (e.g. soybean oil, sesame oil, peanut oil, olive oil, etc.), polyols (e.g. propylene glycol, glycerol, mannitol, sorbitol, etc.), emulsifiers (e.g. Tween®), wetting agents (such as sodium dodecyl sulfate), coloring agents, flavoring agents, stabilizers, antioxidants, preservatives, pyrogen-free water, etc.

In another preferred example, the compounds of formula (I), formula (II), and formula (III) of the present disclosure may form a complex with macromolecular compound or polymer by a non-bonding interaction. In another preferred example, the compounds of formula (I), formula (II), and formula (III) of the present disclosure as small molecules may also be linked by chemical bonds through macromolecular compound or polymer. The macromolecular compounds can be biological macromolecules such as polysaccharides, proteins, nucleic acids, and polypeptides, etc.

The mode of administration of the active ingredients or pharmaceutical compositions of the present disclosure is not particularly limited. The representative modes of administration include (but are not limited to): oral, intratumoral, rectal, parenteral (intravenous, intramuscular or subcutaneous) administration.

Solid dosage forms used for oral administration include capsules, tablets, pills, powders and granules.

In these solid dosage forms, the active ingredient is mixed with at least one of the conventional inert excipients (or carriers), such as sodium citrate or dicalcium phosphate, or the mixture of the following ingredients:

(a) fillers or compatibilizers, such as starch, lactose, sucrose, glucose, mannitol, and silicic acid;
(b) binders, such as hydroxylmethylcellulose, alginate, gelatin, polyvinylpyrrolidone, sucrose, and gum arabic;
(c) humectants, such as glycerin;
(d) disintegrants, such as agar, calcium carbonate, potato starch or tapioca starch, alginic acid, certain complex silicates, and sodium carbonate;
(e) dissolution-slowing agents, such as paraffin wax;
(f) absorption accelerators, such as quaternary amine compounds;
(g) wetting agents, such as cetyl alcohol and glycerol monostearate;
(h) adsorbents, such as kaoline; and
(i) lubricants, such as talc, calcium stearate, magnesium stearate, solid polyethylene glycol, sodium dodecyl sulfate, or the mixtures thereof. In capsules, tablets and pills, the dosage form may also contain buffering agents.

The above-described solid dosage forms may also be prepared using coatings and shell materials, such as enteric coatings and other materials well known in the art. They may contain opacifying agents and the release of the active ingredient in such compositions may be released in a delayed manner at a certain part in the digestive tract. Examples of embedding components that may be employed are polymeric substances and waxes.

Liquid dosage forms for oral administration include pharmaceutically acceptable emulsifiers, solutions, suspensions, syrups, or tinctures. In addition to the active ingredient, the liquid dosage forms may contain inert diluents conventionally employed in the art, such as water or other solvents, solubilizers, and emulsifiers, such as ethanol, isopropyl alcohol, ethyl carbonate, ethyl acetate, propylene glycol, 1,3-butanediol, dimethylformamide, and oils, in particular cottonseed oil, peanut oil, corn germ oil, olive oil, castor oil and sesame oil, or mixtures of these substances. In addition to these inert diluents, the compositions may also contain adjuvants such as wetting agents, emulsifying agents and suspending agents, sweeteners, flavoring agents and perfuming agents.

In addition to the active ingredient, the suspension may contain suspending agents, such as ethoxylated isooctadecanol, polyoxyethylene sorbitol and dehydrated sorbitol esters, microcrystalline cellulose, aluminium methoxide and agar, or mixtures of these substances and the like.

Compositions for parenteral injection may include physiologically acceptable sterile aqueous or anhydrous solutions, dispersions, suspensions or emulsions, and sterile powders for redissolution into sterile injectable solutions or dispersions. Suitable aqueous and anhydrous carriers, diluents, solvents, or excipients include water, ethanol, polyols, and suitable mixtures thereof.

The compounds of the present disclosure can be administered alone or in combination with other therapeutic agents (e.g., hypoglycemic agents).

An application of the pharmaceutical composition is to administer a safe and effective amount of the compound of the present disclosure to a mammal (e.g, a human) in need of treatment, wherein the dose is administered at a pharmacologically effective dose. Of course, the route of administration, the patient’s health status, and other factors should also be considered to determine the specific dosage, which are all within the skill of the skilled practitioner.

Combination Medication

The compounds of formula (I), formula (II), and formula (III) can be used in combination with other drugs known to treat or improve similar conditions. During co-administration, the original drug administration mode and dosage remain unchanged, while the compounds of formula (I), formula (II), and formula (III) are administered simultaneously or subsequently. When the compounds of formula (I), formula (II), and formula (III) are administered simultaneously with one or more other drugs, it is preferable to use a pharmaceutical composition containing one or more known drugs together with the compounds of formula (I), formula (II), and formula (III). Drug combinations also include administration of the compounds of formula (I), formula (II), and formula (III) with one or more other known drugs at overlapping time periods. When the compounds of formula (I), formula (II), and formula (III) are administered in combination with one or more other drugs, it is possible that the dosage of the compounds of formula (I), formula (II), and formula (III) or the known drugs is lower than when they are administered alone.

The drugs or active ingredients that can be used in combination with the compounds of formula (I), formula (II), and formula (III) include, but are not limited to:
estrogen receptor modulators, androgen receptor modulators, retina-like receptor modulators, cytotoxic/cytostatic agents, antiproliferative agents, protein transferase inhibitors, HMG-CoA reductase inhibitors, HIV protein kinase inhibitors, reverse transcriptase inhibitors, angiogenesis inhibitors, inhibitors of cell proliferation and survival signaling, drugs that interfere with cell cycle checkpoints and apoptosis inducers, cytotoxic drugs, tyrosine protein inhibitors, EGFR inhibitors, VEGFR inhibitors, serine/threonine protein inhibitors, Bcr-Abl inhibitors, c-Kit inhibitors, Met inhibitors, Raf inhibitors, MEK inhibitors, MMP inhibitors, topoisomerase inhibitors, histidine deacetylase inhibitors, proteasome inhibitors, CDK inhibitors, Bcl -2 family protein inhibitors, MDM2 family protein inhibitors, IAP family protein inhibitors, STAT family protein inhibitors, PI3K inhibitors, AKT inhibitors, integrin blockers, interferon-$\alpha$, interleukin-12, COX-2 inhibitors, p53, p53 activators, VEGF antibodies, EGF antibodies, etc.

In one embodiment, the drugs or active ingredients that can be used in combination with the compounds of formula (I) include, but are not limited to: aldesleukin, alendronic acid, interferon, alitretinoin, allopurinol, allopurinol sodium, palonosetron hydrochloride, altretamine, aminoglutethimide, amifostine, amrubicin, amsacrine, anastrozole, dolasetron, aranesp, arglabin, arsenic trioxide, aromasin, 5-azacytidine, azathioprine, BCG vaccine or tice BCG vaccine, bestatin, betamethasone acetate, a betamethasone sodium phosphate preparation, bexarotene, bleomycin sulfate, broxuridine, bortezomib, busulfan, calcitonin, alemtuzumab injection, capecitabine, carboplatin, casodex, cefesone, celmoleukin, daunorubicin, chlorambucil, cisplatin, cladribine, cladribine, clodronic acid, cyclophosphamide, cytarabine, dacarbazine, actinomycin D, daunorubicin liposome, dexamethasone, dexamethasone phosphate, estradiol valerate, denileukin diftitox 2, depo-medrol, deslorelin, dexrazoxane, diethylstilbestrol, fluconazole, docetaxel, doxifluridine, adriamycin, dronabinol, a holmium-166-chitosan complex, eligard, rasburicase, epirubicin hydrochloride, aprepitant, epirubicin, epoetin alfa, erythropoietin, eptaplatin, levamisole tablets, estradiol preparations, 17-beta-estradiol, estramustine sodium phosphate, ethinyloestradiol, amifostine, hydroxyphosphoric acid, etopophos, etoposide, fadrozole, tamoxifen preparations, filgrastim, finasteride, filgrastim, fluorouridine, fluconazole, fludarabine, 5-fluorodeoxyuridine monophosphate, 5-fluorouracil, fluoxymesterone, flutamide, formestan, 1-$\beta$-D-arabinofuranosylcytosine-5'-stearyl phosphate, fotemustine, fulvestrant, gammaglobulin, gemcitabine, gemtuzumab, imatinib mesylate, gliadel, goserelin, granisetron hydrochloride, histrelin, hycamtin, hydrocortisone, erythro-hydroxynonyladenine, hydroxyurea, ibritumomab tiuxetan, idarubicin, ifosfamide, interferon alpha , interferon-$\alpha$2, interferon-alpha-2A, interferon-alpha-2B, interferon-alpha-nl, interferon-alpha-n3, interferon-beta, interferon-gamma-la, interleukin-2, intron A, iressa, irinotecan, kytril, lentinan sulfate, letrozole, leucovorin, leuprolide, leuprolide acetate, levamisole, calcium levofolinate, levothyroxine sodium, a levothyroxine sodium preparation, lomustine, lonidamine, dronabinol, chlormethine, mecobalamin, medroxyprogesterone acetate, megestrol acetate, melphalan, esterified estrogen, 6-mercaptopurine, mesna, methotrexate, methyl aminolevulinate, miltefosine, minocycline, mitomycin C, mitotane, mitoxantrone, trilostane, doxorubicin citrate liposome, nedaplatin, pegfilgrastim, oprelvekin, neupogen, nilutamide, tamoxifen, NSC-631570, recombinant human interleukin 1-$\beta$, octreotide, ondansetron hydrochloride, a prednisolone oral solution, oxaliplatin, paclitaxel, a prednisolone sodium phosphate preparation, pegaspargase, Pegasys, pentostatin, picibanil, pilocarpine hydrochloride, pirarubicin, plicamycin, porfimer sodium, prednimustine, prednisolone steaglate, prednisone, premarin, procarbazine, recombinant human erythropoietin, raltitrexed, rebif, rhenium-186 etidronate, rituximab, roferon-A, romurtide, pilocarpine hydrochloride tablets, octreotide, sargramostim, semustine, sizofiran, sobuzoxane, solu-medrol, sparfosic acid, stem-cell therapy, streptozocin, strontium-89 chloride, synthroid, tamoxifen, tamsulosin, tasonermin, tastolactone, taxotere, teceleukin, temozolomide, teniposide, testosterone propionate, methyltestosterone, thioguanine, thiotepa, thyrotropin, tiludronic acid, topotecan, toremifene, tositumomab, trastuzumab, treosulfan, tretinoin, methotrexate tablets, trimethylmelamine, trimetrexate, triptorelin acetate, triptorelin pamoate, UFT, uridine, valrubicin, vesnarinone, vinblastine, vincristine, vindesine, vinorelbine, virulizin, dexrazoxane, zinostatin stimalamer, zofran, paclitaxel protein stabilizers, acolbifene, interferon r-lb, affinitak, aminopterin, arzoxifene, asoprisnil, atamestane, atrasentan, BAY43-9006, avastin, CCI-779, CDC-501, celebrex, cetuximab, crisnatol, cyproterone acetate, decitabine, DN-101, doxorubicin-MTC, dSLIM, dutasteride, edotecarin, eflornithine, exatecan, fenretinide, histamine dihydrochloride, a histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis-retinoic acid, satraplatin, seocalcitol, T-138067, tarceva, paclitaxel docosahexaenoate, thymosin $\alpha$1, tiazofurine, tipifarnib, tirapazamine, TLK-286, toremifene, TransMID-107R, valspodar, vapreotide, vatalanib, verteporfin, vinflunine, Z-100, zoledronic acid, or a combination thereof.

The above-mentioned features in the present disclosure, or the features mentioned in the embodiments, may be combined arbitrarily. All features disclosed in the description of the present disclosure may be used in combination with any composition forms, and each feature disclosed in the description may be replaced by any alternative features that serve the same, equivalentor similar purpose. Thus, unless otherwise stated, the features disclosed are only general examples of equivalent or similar features.

The present disclosure is further described below in connection with specific embodiments. It should be understood that these embodiments are intended to illustrate the invention only and are not intended to limit the scope of the invention. The experimental methods for which specific conditions are not indicated in the following embodiments generally follow conventional conditions such as those described in Sambrook et al., Molecular Cloning: A Laboratory Manual (New York: Cold Spring Harbor Laboratory Press, 1989), or conditions as recommended by the manufacturer. Percentages and portions are calculated by weight unless otherwise stated.

Unless otherwise defined, all professional and scientific terms used herein have the same meaning as those familiar to those skilled in the art. In addition, any methods and materials similar or equivalent to those described herein may be applied to the methods of the present disclosure. The methods and materials of the preferred embodiment described herein are for illustrative purposes only.

The reagents used in the following embodiments are all commercially available.

The following are specific embodiments.

Example 1: Preparation of (E)-5-benzyl-3-(2-(pyridin-2-yl)vinyl)-1H-indazole (designated as DYX-048):

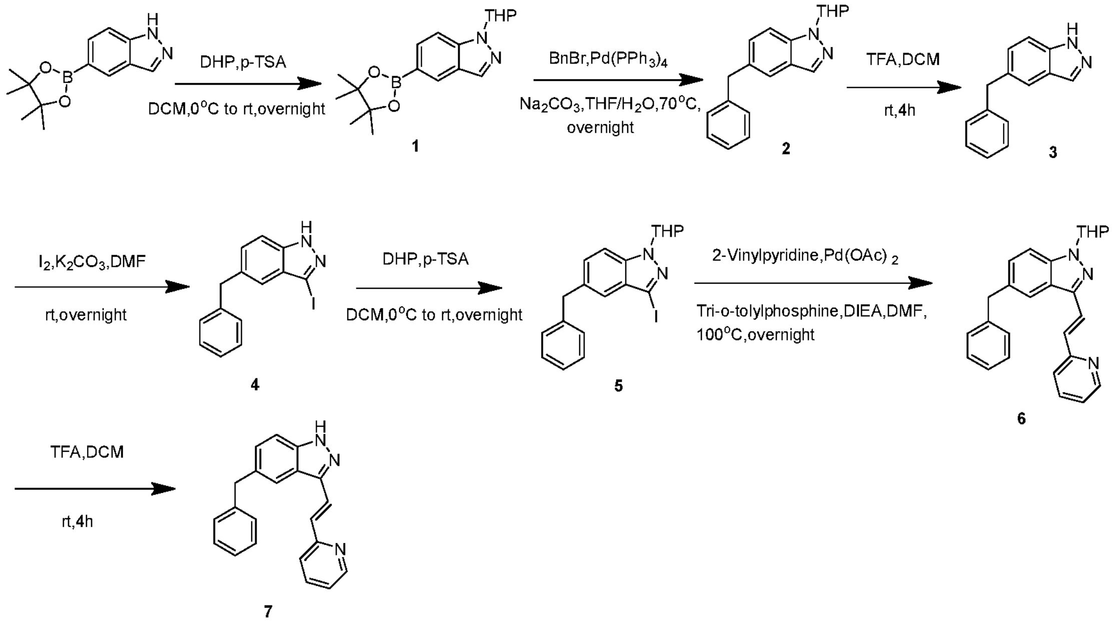


Step 1: Preparation of 1-(tetrahydro-2H-pyran-2-yl)-5-(4,4,5,5-tetramethyl-1,3,2-dioxaborolan-2-yl)-1H-indaz ole (Compound 1)

1H-indazole-5-boronic acid pinacol ester (5 g, 20.5 mmol) and p-toluenesulfonic acid (352 mg, 2.05 mmol) were dissolved in 50 mL of dichloromethane and DHP (5.17 g, 61.5 mmol) was slowly added under ice bath. After addition, the temperature of the mixture was slowly raised to room temperature and stirred overnight. After the reaction was completed, 50 mL of dichloromethane was added for diluting, and the diluted reaction solution was extracted with a saturated sodium bicarbonate solution (100 mL). The organic layer was washed with saturated brine, dried over anhydrous sodium sulfate, filtered by suction and evaporated to dryness. Column chromatography was performed to give 5.5 g of the compound 1 (yield: 81.8%). $^1$H NMR (400 MHz, DMSO-$d_6$) $\delta$ 8.23 - 8.09 (m, 2H), 7.78 - 7.61 (m, 2H), 5.85 (dd, $J$ = 9.7, 2.5 Hz, 1H), 3.93 - 3.83 (m, 1H), 3.73 (ddd, $J$ = 11.4, 8.0, 5.9 Hz, 1H), 2.46 - 2.31 (m, 1H), 2.09 - 1.88

(m, 2H), 1.84 - 1.65 (m, 1H), 1.58 (dq, *J* = 8.8, 5.4, 4.6 Hz, 2H), 1.31 (s, 12H).

LCMS (ESI), m/z: 329.2 $[M+H]^+$

Step 2: Preparation of 5-benzyl-1-(tetrahydro-2H-pyran-2-yl)-1H-indazole (Compound 2)

Compound 1 (1 g, 3 mmol), benzyl bromide (1.05 g, 6 mmol), and sodium carbonate (650 mg, 6 mmol) were dissolved in a mixed solution of 40 mL of tetrahydrofuran and 8 mL of water, replaced with argon three times, and tetrakis(triphenylphosphine)palladium (352 mg, 0.3 mmol) was added, and the mixture was replaced with argon three times again; and the reaction was carried out overnight at 70 °C. After the reaction was completed, THF was removed by rotary evaporation, and ethyl acetate and water were added for extraction. The organic layer was washed with saturated brine, dried over anhydrous sodium sulfate, filtered by suction, and evaporated to dryness,; and column chromatography was performed to give 650 mg of compound 2. (yield: 73.0%). $^1$H NMR (400 MHz, DMSO-$d_6$) δ 8.03 (d, *J* = 0.8 Hz, 1H), 7.65 - 7.60 (m, 1H), 7.58 (q, *J* = 0.8 Hz, 1H), 7.30 - 7.21 (m, 5H), 7.20 - 7.14 (m, 1H), 5.79 (dd, *J* = 9.7, 2.6 Hz, 1H), 4.04 (s, 2H), 3.85 (dtd, *J*= 11.5, 3.8, 1.6 Hz, 1H), 3.70 (ddd, *J*= 11.4, 8.1, 5.9 Hz, 1H), 2.46 - 2.33 (m, 1H), 2.07 - 1.88 (m, 2H), 1.73 (tddd, *J* = 14.6, 12.3, 6.8, 3.8 Hz, 1H), 1.61 - 1.50 (m, 2H). LCMS (ESI), m/z: 293.1 $[M+H]^+$.

Step 3: Preparation of 5-benzyl-1H-indazole (Compound 3)

Compound 2 (500 mg, 1.71 mmol) was dissolved in 10 mL of dichloromethane, 5 mL of trifluoroacetic acid was added thereto and the mixture was stirred for 4 hours at room temperature. After the reaction was completed, the solvent was removed by evaporation, and saturated sodium carbonate solution was added to adjust the pH to be alkaline, followed by extraction with ethyl acetate. The organic layer was washed with saturated brine, dried over anhydrous sodium sulfate, filtered by suction, and evaporated to dryness, and column chromatography was performed to give 276 mg of Compound 3 (yield: 77.5%). $^1$H NMR (400 MHz, DMSO-$d_6$) δ 12.96 (s, 1H), 7.99 (s, 1H), 7.57 (s, 1H), 7.45 (d, *J* = 8.6 Hz, 1H), 7.31 - 7.12 (m, 6H), 4.02 (s, 2H). LCMS (ESI), m/z: 209.1 $[M+H]^+$

Step 4: Preparation of 5-benzyl-3-iodo-1H-indazole (Compound 4)

Compound 3 (250 mg, 1.2 mmol) was dissolved in 20 mL of DMF, and $I_2$ (610 mg, 2.4 mmol) and $K_2CO_3$ (330 mg, 2.4 mmol) were added thereto, and the mixture was stirred at room temperature overnight. After the reaction was completed, a large amount of ice water was added to the reaction flask to precipitate a solid. After stirring for 15 min, suction filtration was performed. The filter residue was washed with water three times and dried to give 380 mg of Compound 4 (yield: 94.7%). $^1$H NMR (400 MHz, DMSO-$d_6$) δ 13.42 (s, 1H), 7.46 (d, *J* = 8.5 Hz, 1H), 7.32 - 7.22 (m, 6H), 7.21 - 7.14 (m, 1H), 4.07 (s, 2H). LCMS (ESI), m/z: 335.0 $[M+H]^+$

Step 5: Preparation of 5-benzyl-3-iodo-1-(tetrahydro-2H-pyran-2-yl)-1H-indazole (Compound 5)

Compound 4 (350 mg, 1.05 mmol), and p-toluenesulfonic acid (18 mg, 0.1 mmol) were dissolved in 10 mL of dichloromethane, and DHP (265 mg, 3.14 mmol) was slowly added thereto under ice bath. After the addition was completed, the temperature of the mixture was slowly raised to room temperature and stirred overnight. After the reaction was completed, 10 mL of dichloromethane was added for diluting, and the diluted reaction solution was extracted with saturated sodium bicarbonate solution (20 mL). The organic layer was washed with saturated brine, dried over anhydrous sodium sulfate, filtered by suction, and evaporated to dryness, and column chromatography was performed to give 333 mg of Compound 5 (yield: 76.2%).

LCMS (ESI), m/z: 419.1 $[M+H]^+$

Step 6: Preparation of (E)-5-benzyl-3-(2-(pyridin-2-yl)vinyl)-1-(tetrahydro-2H-pyran-2-yl)-1H-indazole (Compound 6)

Compound 5 (300 mg, 0.72 mmol) was dissolved in 10 mL of DMF, and 2-vinylpyridine (150 mg, 1.44 mmol) and DIEA (280 mg, 2.16 mmol) were added, performed argon replacement three times. Then, palladium acetate (9 mg, 0.04 mmol) and tris(o-methylphenyl)phosphine (22 mg, 0.07 mmol) were added, performed argon replacement three times again. The reaction was carried out at 100°C overnight. After the reaction was completed, an appropriate amount of water was added, followed by extraction with ethyl acetate three times. The organic layer was washed for four times with water and once with saturated brine, dried over anhydrous sodium sulfate, filtered by suction and evaporated to dryness, and column chromatography was performed to give 154 mg of Compound 6 (yield: 54.1%). $^1$H NMR (400 MHz, DMSO-$d_6$) δ 8.61 (ddd, *J*= 4.8, 1.7, 0.8 Hz, 1H), 8.10 - 8.06 (m, 1H), 7.92 (d, *J*= 16.4 Hz, 1H), 7.82 (td, *J* = 7.6, 1.8 Hz, 1H), 7.72 - 7.65 (m, 2H), 7.58 (d, *J* = 16.3 Hz, 1H), 7.36 - 7.24 (m, 6H), 7.22 - 7.13 (m, 1H), 5.85 (dd, *J* = 9.7, 2.5 Hz,

1H), 4.12 (s, 2H), 3.94 - 3.82 (m, 1H), 3.81 - 3.66 (m, 1H), 2.48 - 2.34 (m, 1H), 2.13 - 1.90 (m, 2H), 1.83 - 1.67 (m, 1H), 1.60 (dq, J= 8.4, 4.2 Hz, 2H). LCMS (ESI), m/z: 396.2 $[M+H]^+$

Step 7: Preparation of (E)-5-benzyl-3-(2-(pyridin-2-yl)vinyl)-1H-indazole (Compound 7)

Compound 6 (150 mg, 0.38 mmol) was dissolved in 5 mL of dichloromethane, and 5 mL of trifluoroacetic acid was added thereto; and the mixture was stirred for 4 hours at room temperature. After the reaction was completed, the solvent was removed by evaporation. Saturated sodium carbonate solution was added to adjust the pH to 6-7, followed by extraction with ethyl acetate. The organic layer was washed with saturated brine, dried over anhydrous sodium sulfate, filtered by suction, and evaporated to dryness, and column chromatography was performed to give 90 mg of Compound 7 (yield: 76.4%).

$^1$H NMR (400 MHz, DMSO-$d_6$) δ 13.22 (s, 1H), 8.63 - 8.57 (m, 1H), 8.08 (s, 1H), 7.95 (d, *J*= 16.4 Hz, 1H), 7.81 (td, *J*= 7.6, 1.9 Hz, 1H), 7.67 (dt, *J*= 7.8, 1.1 Hz, 1H), 7.55 (d, *J*= 16.4 Hz, 1H), 7.48 (d, *J*= 8.5 Hz, 1H), 7.33 - 7.23 (m, 6H), 7.17 (dtdd, *J* = 8.8, 6.2, 4.2, 2.1 Hz, 1H), 4.11 (s, 2H). LCMS (ESI), m/z: 312.1 $[M+H]^+$

Example 2: Preparation of (E)-5-(2-fluorobenzyl)-3-(2-(pyridin-2-yl)vinyl)-1H-indazole (designated as DYX-032)

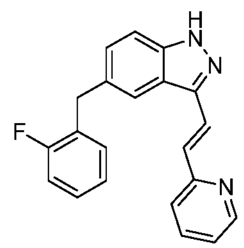

Synthesis method was as Example 1 (yield: 70.5%).

$^1$H NMR (400 MHz, DMSO-$d_6$) δ 13.27 (s, 1H), 8.61 (dd, J= 5.0, 1.7 Hz, 1H), 8.07 (s, 1H), 7.95 (d, J= 16.3 Hz, 1H), 7.82 (td, J= 7.6, 1.8 Hz, 1H), 7.68 (d, J= 7.8 Hz, 1H), 7.57 - 7.46 (m, 2H), 7.35 (td, J= 7.8, 1.9 Hz, 1H), 7.27 (dddd, J= 9.3, 5.0, 3.9, 1.5 Hz, 3H), 7.22 - 7.11 (m, 2H), 4.15 (s, 2H). LCMS (ESI), m/z: 330.1 $[M+H]^+$

Example 3: Preparation of (E)-5-(3-fluorobenzyl)-3-(2-(pyridin-2-yl)vinyl)-1H-indazole (designated as DYX-038)

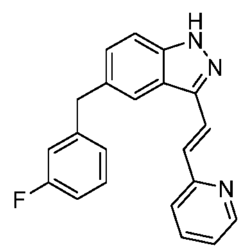

Synthesis method was as Example 1 (yield: 77.2 %).

$^1$H NMR (400 MHz, DMSO-$d_6$) δ 13.28 (s, 1H), 8.61 (dd, J= 5.0, 1.7 Hz, 1H), 8.13 (s, 1H), 7.98 (d, J= 16.4 Hz, 1H), 7.82 (td, J= 7.6, 1.8 Hz, 1H), 7.69 (d, J= 7.8 Hz, 1H), 7.59 (d, *J* = 16.4 Hz, 1H), 7.51 (d, *J* = 8.6 Hz, 1H), 7.38 - 7.22 (m, 3H), 7.19 - 7.10 (m, 2H), 7.05 - 6.94 (m, 1H), 4.13 (s, 2H).

LCMS (ESI), m/z: 330.1 $[M+H]^+$

Example 4: Preparation of (E)-5-(4-fluorobenzyl)-3-(2-(pyridin-2-yl)vinyl)-1H-indazole (designated as DYX-042)

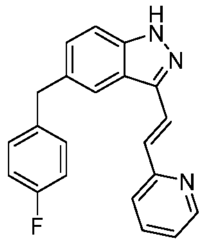

Synthesis method was as Example 1 (yield: 71.2%).

$^1$H NMR (400 MHz, DMSO-$d_6$) δ 13.24 (s, 1H), 8.60 (dd, J= 4.9, 1.7 Hz, 1H), 8.09 (s, 1H), 7.95 (d, J= 16.4 Hz, 1H), 7.82 (td, J= 7.6, 1.9 Hz, 1H), 7.68 (d, J= 7.8 Hz, 1H), 7.55 (d, *J* = 16.4 Hz, 1H), 7.48 (d, *J* = 8.6 Hz, 1H), 7.38 - 7.29 (m, 2H), 7.30 - 7.21 (m, 2H), 7.16 - 7.06 (m, 2H), 4.10 (s, 2H).

LCMS (ESI), m/z: 330.1 [M+H]$^+$

Example 5: Preparation of (E)-5-(2,3-difluorobenzyl)-3-(2-(pyridin-2-yl)vinyl)-1H-indazole (designated as DYX-082)

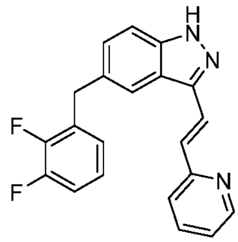

Synthesis method was as Example 1 (yield: 74.7%).

$^1$H NMR (400 MHz, DMSO-$d_6$) δ 13.28 (s, 1H), 8.60 (dd, J= 4.9, 1.7 Hz, 1H), 8.09 (s, 1H), 7.94 (d, J= 16.4 Hz, 1H), 7.82 (td, J= 7.7, 1.9 Hz, 1H), 7.68 (d, J= 7.8 Hz, 1H), 7.58 - 7.48 (m, 2H), 7.28 (qt, *J* = 6.7, 2.5 Hz, 3H), 7.19 - 7.10 (m, 2H), 4.19 (s, 2H).

LCMS (ESI), m/z: 348.1 [M+H]$^+$

Example 6: Preparation of (E)-5-(2,4-difluorobenzyl)-3-(2-(pyridin-2-yl)vinyl)-1H-indazole (designated as DYX-060)

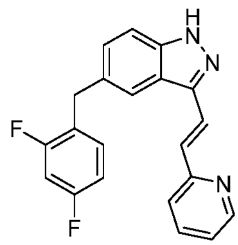

Synthesis method was as Example 1 (yield: 70.6%).

$^1$H NMR (400 MHz, DMSO-$d_6$) δ 13.60 (s, 1H), 8.78 (dd, J= 5.2, 1.4 Hz, 1H), 8.35 - 8.28 (m, 2H), 8.22 (d, *J* = 16.5 Hz, 1H), 8.08 (s, 1H), 7.72 - 7.62 (m, 2H), 7.57 (d, *J* = 8.6 Hz, 1H), 7.37 (td, *J* = 8.7, 6.6 Hz, 1H), 7.29 (dd, *J* = 8.6, 1.4 Hz, 1H), 7.22 (td, *J* = 9.9, 2.6 Hz, 1H), 7.04 (td, *J* = 8.6, 2.6 Hz, 1H), 4.12 (s, 2H).

LCMS (ESI), m/z: 348.1 [M+H]$^+$

Example 7: Preparation of (E)-5-(2,5-difluorobenzyl)-3-(2-(pyridin-2-yl)vinyl)-1H-indazole (designated as DYX-054)

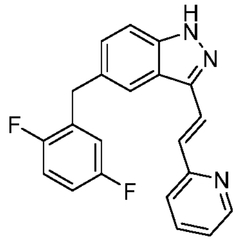

Synthesis method was as Example 1 (yield: 76.4%).

$^1H$ NMR (400 MHz, DMSO-$d_6$) δ 13.28 (s, 1H), 8.61 (dd, J= 5.0, 1.7 Hz, 1H), 8.09 (s, 1H), 7.95 (d, J= 16.4 Hz, 1H), 7.81 (td, J= 7.7, 1.8 Hz, 1H), 7.67 (d, J= 7.8 Hz, 1H), 7.59 - 7.47 (m, 2H), 7.32 - 7.17 (m, 4H), 7.15 - 7.02 (m, 1H), 4.13 (s, 2H).

LCMS (ESI), m/z: 348.1 $[M+H]^+$

Example 8: Preparation of (E)-5-(2,6-difluorobenzyl)-3-(2-(pyridin-2-yl)vinyl)-1H-indazole (designated as DYX-081)

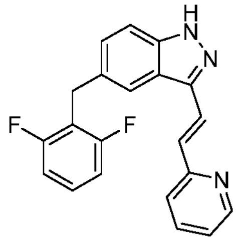

Synthesis method was as Example 1 (yield: 65.2%).

LCMS (ESI), m/z: 348.1 $[M+H]^+$

Example 9: Preparation of (E)-5-(3,4-difluorobenzyl)-3-(2-(pyridin-2-yl)vinyl)-1H-indazole (designated as DYX-059)

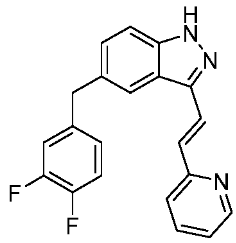

Synthesis method was as Example 1 (yield: 68.8%).

$^1H$ NMR (400 MHz, DMSO-$d_6$) δ 13.26 (s, 1H), 8.61 (dd, J= 4.8, 1.7 Hz, 1H), 8.12 (s, 1H), 7.96 (d, *J*= 16.3 Hz, 1H), 7.82 (td, J= 7.7, 1.8 Hz, 1H), 7.68 (d, *J*= 7.8 Hz, 1H), 7.62 - 7.45 (m, 2H), 7.43 - 7.22 (m, 4H), 7.14 (ddt, *J* = 8.2, 3.7, 1.6 Hz, 1H), 4.10 (s, 2H).

LCMS (ESI), m/z: 348.1 $[M+H]^+$

Example 10: Preparation of (E)-5-(3,5-difluorobenzyl)-3-(2-(pyridin-2-yl)vinyl)-1H-indazole (designated as CCB-489)

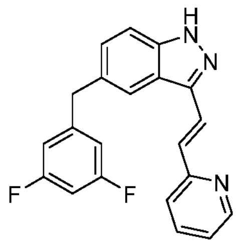

Synthesis method was as Example 1 (yield: 73.5%).
$^1H$ NMR (400 MHz, DMSO-$d_6$) δ 13.27 (s, 1H), 8.61 (d, *J* = 3.9 Hz, 1H), 8.14 (s, 1H), 7.97 (d, *J* = 16.4 Hz, 1H), 7.83 (td, *J* = 7.7, 1.7 Hz, 1H), 7.68 (d, *J* = 7.8 Hz, 1H), 7.57 (d, *J* = 16.4 Hz, 1H), 7.51 (d, *J* = 8.5 Hz, 1H), 7.34 - 7.24 (m, 2H), 7.11 - 6.98 (m, 3H), 4.13 (s, 2H).
LCMS (ESI), m/z: 348.1 $[M+H]^+$

Example 11: Preparation of (E)-3-(2-(pyridin-2-yl)vinyl)-5-(3,4,5-trifluorobenzyl)-1H-indazole (designated as DYX-083)

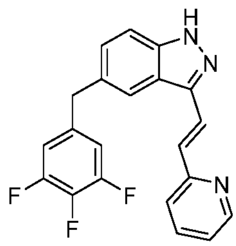

Synthesis method was as Example 1 (yield: 79.2%).
$^1H$ NMR (400 MHz, DMSO-$d_6$) δ 13.28 (s, 1H), 8.61 (dd, J= 4.6, 1.7 Hz, 1H), 8.13 (s, 1H), 7.96 (d, *J*= 16.4 Hz, 1H), 7.83 (td, J= 7.7, 1.9 Hz, 1H), 7.69 (d, *J*= 7.9 Hz, 1H), 7.62 - 7.46 (m, 2H), 7.35 - 7.23 (m, 4H), 4.09 (s, 2H).
LCMS (ESI), m/z: 366.1 $[M+H]^+$

Example 12: Preparation of (E)-5-(3-chlorobenzyl)-3-(2-(pyridin-2-yl)vinyl)-1H-indazole (designated as DYX-134X)

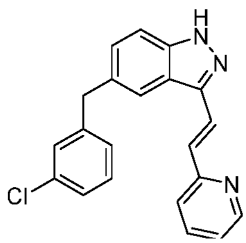

Synthesis method was as Example 1 (yield: 63.3%).
$^1H$ NMR (400 MHz, DMSO-$d_6$) δ 13.25 (s, 1H), 8.64 - 8.58 (m, 1H), 8.12 (s, 1H), 8.00 - 7.92 (m, 1H), 7.82 (td, *J*= 7.6, 1.9 Hz, 1H), 7.68 (d, *J*= 7.8 Hz, 1H), 7.57 (d, *J* = 16.4 Hz, 1H), 7.50 (d, *J* = 8.5 Hz, 1H), 7.37 (t, *J* = 1.8 Hz, 1H), 7.34 - 7.20 (m, 5H), 4.12 (s, 2H).
LCMS (ESI), m/z: 346.1 $[M+H]^+$

Example 13: Preparation of (E)-5-(3-methoxybenzyl)-3-(2-(pyridin-2-yl)vinyl)-1H-indazole (designated as DYX-135X)

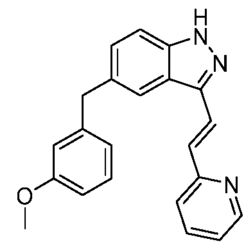

Synthesis method was as Example 1 (yield: 66.9%).

$^1$H NMR (400 MHz, DMSO-$d_6$) δ 13.25 (s, 1H), 8.60 (d, *J* = 4.8 Hz, 1H), 8.09 (s, 1H), 7.96 (d, *J* = 16.3 Hz, 1H), 7.81 (t, *J* = 7.8 Hz, 1H), 7.68 (d, *J* = 7.9 Hz, 1H), 7.52 (dd, *J* = 31.8, 12.4 Hz, 2H), 7.32 - 7.14 (m, 3H), 6.93 - 6.65 (m, 3H), 4.07 (2H) 31.8, 12.4 Hz, 2H), 7.32 - 7.14 (m, 3H), 6.93 - 6.65 (m, 3H), 4.07 (s, 2H), 3.71 (s, 3H).

LCMS (ESI), m/z: 342.1 $[M+H]^+$

Example 14: Preparation of (E)-3-((3-(2-(pyridin-2-yl)vinyl)-1H-indazol-5-yl)methyl)benzonitrile (designated as DYX-136X)

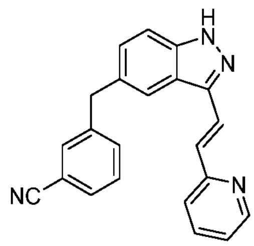

Synthesis method was as Example 1 (yield: 70.5%).

$^1$H NMR (400 MHz, DMSO-$d_6$) δ 13.54 (s, 1H), 8.73 (d, *J* = 5.3 Hz, 1H), 8.26 - 8.10 (m, 4H), 7.79 (s, 1H), 7.67 (q, *J* = 6.7, 4.5 Hz, 3H), 7.55 (ddt, *J* = 20.8, 15.5, 7.1 Hz, 3H), 7.33 (d, *J*= 8.6 Hz, 1H), 4.18 (s, 2H).

LCMS (ESI), m/z: 337.1 $[M+H]^+$

Example 15: Preparation of (E)-5-(3-methylbenzyl)-3-(2-(pyridin-2-yl)vinyl)-1H-indazole (designated as DYX-137X)

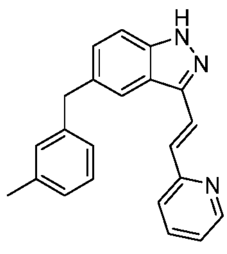

Synthesis method was as Example 1 (yield: 78.6%).

$^1$H NMR (400 MHz, DMSO-$d_6$) δ 13.25 (s, 1H), 8.60 (d, *J* = 4.8 Hz, 1H), 8.07 (s, 1H), 7.96 (d, *J* = 16.3 Hz, 1H), 7.81 (td, *J* = 7.6, 1.9 Hz, 1H), 7.68 (d, *J* = 7.8 Hz, 1H), 7.56 *(d, J*= 16.4 Hz, 1H), 7.48 (d, *J*= 8.5 Hz, 1H), 7.31 - 7.20 (m, 2H), 7.16 (t, *J* = 7.4 Hz, 1H), 7.08 (d, *J* = 8.0 Hz, 2H), 6.98 (d, *J* = 7.4 Hz, 1H), 4.06 (s, 2H), 2.24 (s, 3H).

LCMS (ESI), m/z: 326.1 $[M+H]^+$

Example 16: Preparation of (E)-3-(2-(pyridin-2-yl)vinyl)-5-(3-(trifluoromethyl)benzyl)-1H-indazole (designated as DYX-138X)

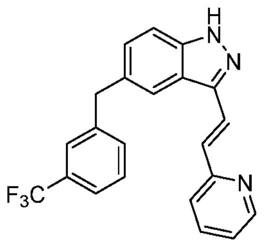

Synthesis method was as Example 1 (yield: 67.0%).

$^1H$ NMR (400 MHz, DMSO-$d_6$) δ 13.28 (s, 1H), 8.61 (dd, J= 5.2, 1.7 Hz, 1H), 8.16 (s, 1H), 7.97 (d, *J* = 16.4 Hz, 1H), 7.84 (td, *J* = 7.7, 1.9 Hz, 1H), 7.72 - 7.66 (m, 2H), 7.65 - 7.60 (m, 1H), 7.60 - 7.48 (m, 4H), 7.32 - 7.26 (m, 2H), 4.22 (s, 2H).

LCMS (ESI), m/z: 380.1 $[M+H]^+$

Example 17: Preparation of (E)-5-(3,5-difluorophenoxy)-3-(2-(pyridin-2-yl)vinyl)-1H-indazole (designated as DYX-101)

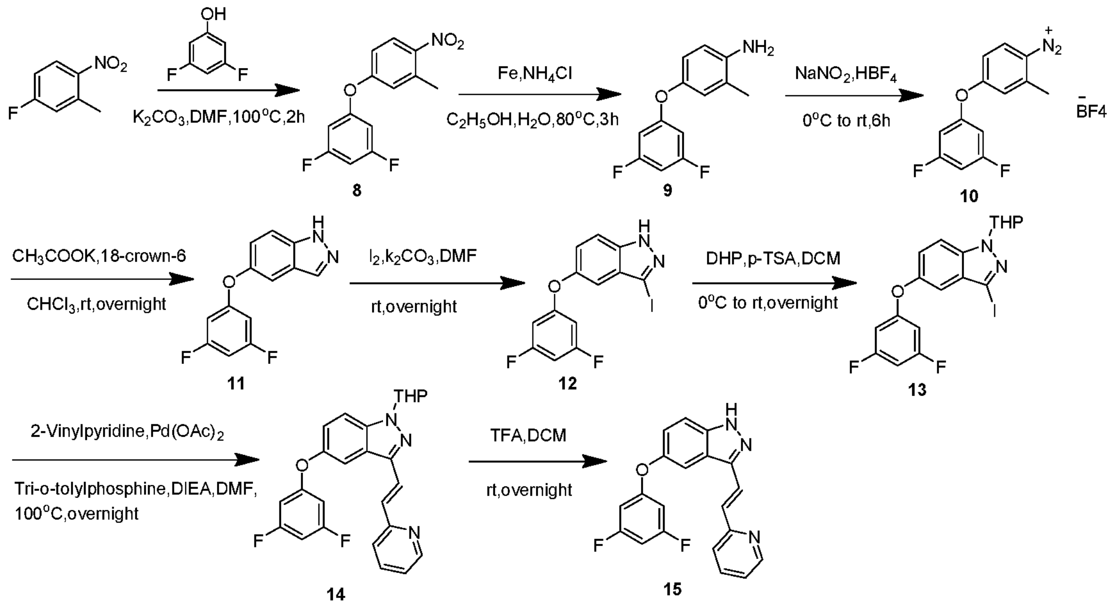


Step 1: Preparation of 4-(3,5-difluorophenoxy)-2-methyl-1-nitrobenzene (Compound 8)

4-fluoro-2-methyl-1-nitrobenzene (5 g, 32.2 mmol) was dissolved in 30 mL of DMF. 3,5-difluorophenol (4.62 g, 35.5 mmol) and potassium carbonate (8.91 g, 64.4 mmol) were added and react at 100°C for 2 hours. After the reaction was completed, reaction flask was moved to ice bath. The resulting reaction solution was stirred with a large amount of ice water to precipitate solid. After stirring for 30 minutes, suction filtration was performed, and the obtained filter residue was washed with water for five times. The filter residue was dried, slurried with diethyl ether, and filtered by suction to obtain 7.6 g of the compound 8 (yield: 88.9%). $^1H$ NMR (400 MHz, DMSO-$d_6$) δ 8.09 (d, *J* = 90 Hz, 1H), 7.21 (d, *J* = 2.8 Hz, 1H), 7.20 - 7.12 (m, 1H), 7.09 (dd, *J* = 9.0, 2.8 Hz, 1H), 7.03 - 6.94 (m, 2H), 2.53 (s, 3H). LCMS (ESI), m/z: 266.1 $[M+H]^+$

Step 2: Preparation of 4-(3,5-difluorophenoxy)-2-methylaniline (Compound 9)

Compound 8 (7 g, 26.4 mmol) and ammonium chloride (4.24 g, 79.2 mmol) were dissolved in a mixed solvent of 100 mL of ethanol and 10 mL of water. Iron powder (7.39 g, 132 mmol) was added slowly with stirring. After addition, the obtained mixture was heated to 80°C and reacted for 3 hours. After the reaction was completed, diatomite was used for suction filtration. The obtained filter residue was washed with ethyl acetate for three times, and the obtained filtrate was evaporated to dryness. Water and ethyl acetate were added for extraction. The organic layer was washed with saturated brine, dried over anhydrous sodium sulfate, filtered by suction, and evaporated to dryness. Column chromatography was performed to give 4.4 g of the compound 9 (yield: 70.9%). $^1$H NMR (400 MHz, DMSO-$d_6$) δ 6.91 - 6.60 ( m, 4H), 6.58 - 6.42 (m, 2H), 4.89 (s, 2H), 2.06 (s, 3H).

LCMS (ESI), m/z: 236.1 $[M+H]^+$

Step 3: Preparation of 4-(3,5-difluorophenoxy)-2-methylbenzenediazonium tetrafluoroborate (Compound 10)

Compound 9 (2 g, 8.5 mmol) was added to a flask contained 20 ml of 40% tetrafluoroboric acid solution. 5 mL of aqueous solution of sodium nitrite (616 mg, 8.9 mmol) was added slowly dropwise at 0 °C. After the addition, the obtained mixture was slowly heated to room temperature for a reaction for 6 hours. After the reaction was completed, suction filtration was carried out. Water washing and drying were performed to give 2.1 g of compound 10 (yield: 73.9%).

$^1$H NMR (400 MHz, DMSO-$d_6$) δ 8.65 (d, $J$ = 9.2 Hz, 1H), 7.47 - 7.30 (m, 3H), 7.28 - 7.16 (m, 2H), 2.69 (s, 3H). LCMS (ESI), m/z: 247.0 $[M+H]^+$

Step 4: Preparation of 5-(3,5-difluorophenoxy)-1H-indazole (Compound 11)

Compound 10 (2 g, 6 mmol) was dissolved in 10 mL of chloroform. Potassium acetate (1.76 g, 18 mmol) and 18-crown-6 (160 mg, 0.6 mmol) were added, and the obtained mixture was stirred at room temperature overnight. After the reaction was completed, water was added for extraction. The organic layer was washed with saturated brine, dried over anhydrous sodium sulfate, filtered by suction, and evaporated to dryness. Column chromatography was performed to give 880 mg of compound 11 (yield: 59.7%).

$^1$H NMR (400 MHz, DMSO-$d_6$) δ 13.23 (s, 1H), 8.07 (t, $J$ = 1.3 Hz, 1H), 7.62 (dt, $J$ = 8.9, 0.9 Hz, 1H), 7.53 (d, $J$ = 2.2 Hz, 1H), 7.17 (dd, $J$ = 8.9, 2.3 Hz, 1H), 6.94 (tt, $J$ = 9.4, 2.3 Hz, 1H), 6.69 - 6.58 (m, 2H). LCMS (ESI), m/z: 247 $[M+H]^+$.

The synthesis methods in remaining steps were the same as those in the steps 4-7 in Example 1 (yield: 67.2%).

$^1$H NMR (400 MHz, DMSO-$d_6$) δ 13.44 (s, 1H), 8.59 (ddd, J= 4.8, 1.9, 0.9 Hz, 1H), 8.04 (d, $J$ = 2.2 Hz, 1H), 7.96 (d, $J$ = 16.4 Hz, 1H), 7.79 (td, $J$ = 7.6, 1.8 Hz, 1H), 7.71 - 7.64 (m, 2H), 7.55 (d, $J$ = 16.4 Hz, 1H), 7.30 - 7.21 (m, 2H), 6.95 (tt, $J$ = 9.4, 2.3 Hz, 1H), 6.69 (dt, $J$= 8.8, 2.1 Hz, 2H).

LCMS (ESI), m/z: 350.1 $[M+H]^+$

Example 18: Preparation of (E)-5-((3,5-difluorophenyl)thio)-3-(2-(pyridin-2-yl)vinyl)-1H-indazole (designated as DYX-090)

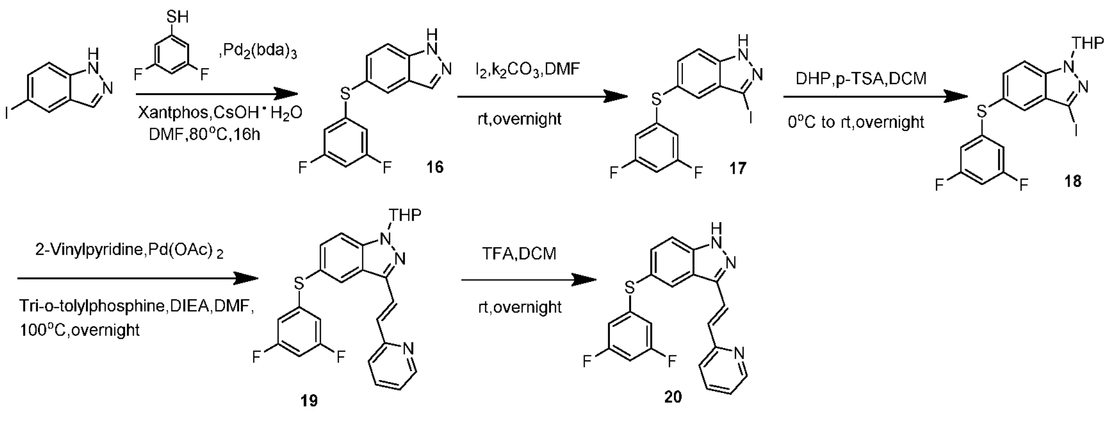


Step 1: Synthesis of 5-((3,5-difluorophenyl)thio)-1H-indazole (Compound 16)

5-Iodo-1H-indazole (2g, 8.2mmol), $Pd_2(dba)_3$ (375mg, 0.41mmol), Xantphos (475mg, 0.82mmol), and cesium hydroxide monohydrate (2.75g, 16.4mmol) were dissolved in 20 mL of DMF with argon replacement for three times. The mixture was heated to 60°C, and slowly added with 3,5-difluorothiophenol (1.32 g, 9.02 mmol) by a syringe. After the addition was completed, the mixture was heated to 80°C to react for 16 hours. After the reaction was completed, an appropriate amount of water was added, followed by extraction with ethyl acetate for three times. The organic layer was washed with water for three times, and then washed once with saturated brine, dried over anhydrous sodium sulfate, filtered by suction, and evaporated to dryness. Column chromatography was performed to give 1.28 g of compound 16 (yield: 59.7%).

$^1$H NMR (400 MHz, Chloroform-$d$) δ 8.15 (s, 1H), 8.05 - 8.00 (m, 1H), 7.59 (d, $J$ = 8.6 Hz, 1H), 7.52 (dd, $J$ =

8.7, 1.6 Hz, 1H), 6.63 - 6.51 (m, 3H). LCMS (ESI), m/z: 263.0 [M+H]+

The synthesis methods in remaining steps were the same as those in the steps 4-7 in Example 1 (yield: 71.1%).

$^1$H NMR (400 MHz, DMSO-$d_6$) δ 13.61 (s, 1H), 8.60 (dd, J= 4.8, 1.7 Hz, 1H), 8.55 (d, *J* = 1.5 Hz, 1H), 8.00 (d, *J* = 16.4 Hz, 1H), 7.82 (td, *J* = 7.6, 1.8 Hz, 1H), 7.73 (dd, *J* = 8.3, 6.0 Hz, 2H), 7.63 (d, *J* = 16.4 Hz, 1H), 7.53 (dd, *J* = 8.7, 1.6 Hz, 1H), 7.33 - 7.24 (m, 1H), 7.05 (tt, *J* = 9.3, 2.3 Hz, 1H), 6.78 (h, *J* = 4.9 Hz, 2H).

LCMS (ESI), m/z: 366.1 [M+H]+

Example 19: Preparation of (E)-5-((3,5-difluorophenyl)sulfonyl)-3-(2-(pyridin-2-yl)vinyl)-1H-indazole (designated as DYX-114)

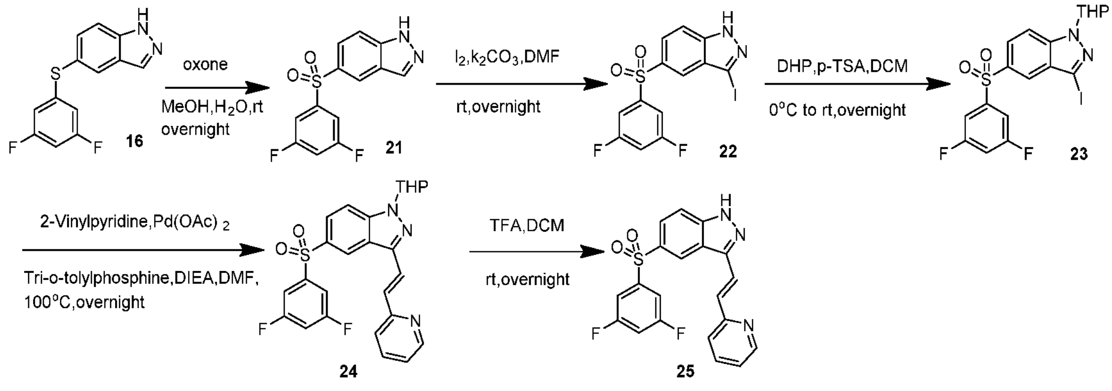


Step 1: Synthesis of 5-((3,5-difluorophenyl)sulfonyl)-1H-indazole (Compound 21)

Compound 16 (1 g, 3.8 mmol) was dissolved in a mixed solution of 20 mL of methanol and 10 mL of water. Oxone (3.2 g, 19 mmol) was added and the mixture was stirred overnight at room temperature. After the reaction was completed, methanol was removed by evaporation, and an appropriate amount of ethyl acetate was added for extraction. The organic layer was washed with saturated brine, dried over anhydrous sodium sulfate, filtered by suction, and evaporated to dryness. Column chromatography was performed to give 841 mg of compound 21 (yield: 75.0%).

$^1$H NMR (400 MHz, Chloroform-*d*) δ 8.56 - 8.47 (m, 1H), 8.27 (s, 1H), 7.88 (dd, *J* = 8.9, 1.7 Hz, 1H), 7.64 (d, *J* = 8.8 Hz, 1H), 7.55 - 7.42 (m, 2H), 6.98 (tt, *J* = 8.4, 2.3 Hz, 1H). LCMS (ESI), m/z: 295.0 [M+H]+

The synthesis methods in remaining steps were the same as those in the steps 4-7 in Example 1 (yield: 70.0%).

$^1$H NMR (400 MHz, DMSO-$d_6$) δ 13.84 (s, 1H), 8.93 (d, *J* = 1.8 Hz, 1H), 8.64 (dd, *J* = 4.7, 1.7 Hz, 1H), 8.07 (d, *J* = 16.3 Hz, 1H), 7.95 (dd, *J* = 8.8, 1.7 Hz, 1H), 7.86 (td, *J* = 7.4, 6.9, 2.1 Hz, 3H), 7.78 (dd, *J* = 8.4, 6.2 Hz, 2H), 7.33 (ddd, *J* = 7.4, 4.7, 1.2 Hz, 1H).

LCMS (ESI), m/z: 398.0 [M+H]+

Example 20: Preparation of (E)-N-(3,5-difluorophenyl)-3-(2-(pyridin-2-yl)vinyl)-1H-indazol-5-amine (designated as DYX-105)

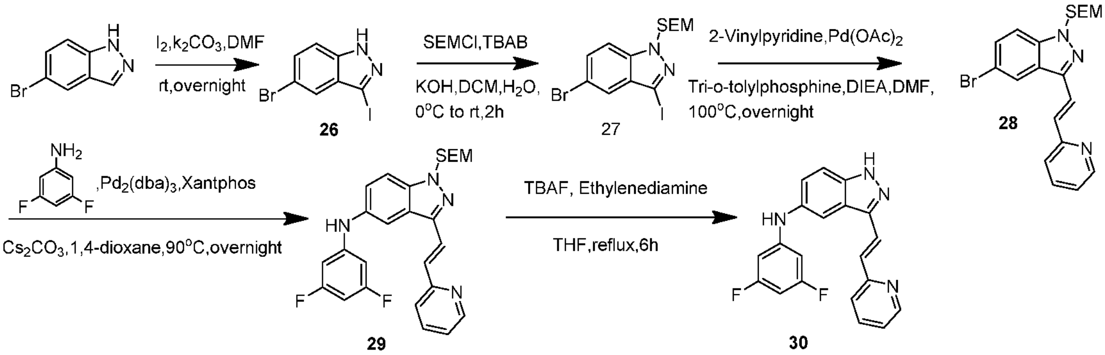

Step 1: Synthesis of 5-bromo-3-iodo-1H-indazole (Compound 26)

Compound 26 was synthesized the same as step 4 in Example 1 (yield: 90.8%).

Step 2: Synthesis of 5-bromo-3-iodo-1-((2-(trimethylsilyl)ethoxy)methyl)-1H-indazole (Compound 27)

Compound 26 (3 g, 9.3 mmol) and TBAB (150 mg, 0.46 mmol) were dissolved in 20 mL of DCM. An equal volume of 50% KOH solution was prepared and added, and SEMCl (2.32 g, 13.9 mmol) was added slowly dropwise under stirring in an ice bath. After the addition, the reaction was continued to be carried out for 2 hours. After the reaction was completed, DCM was added for diluting, and the aqueous layer was extracted with DCM for three times. The organic layer was washed twice with water and then washed once with saturated brine, dried over anhydrous sodium sulfate, filtered by suction, and evaporated to dryness. Column chromatography was performed to give 2.65 g of compound 27 (yield: 62.9%).

$^1$H NMR (400 MHz, DMSO-$d_6$) δ 7.82 - 7.74 (m, 1H), 7.70 - 7.61 (m, 2H), 5.75 (s, 2H), 3.53 - 3.47 (m, 2H), 0.80 - 0.72 (m, 2H), -0.13 (s, 9H). LCMS (ESI), m/z: 453.0 $[M+H]^+$

Step 3: Synthesis of (E)-5-bromo-3-(2-(pyridin-2-yl)vinyl)-1-((2-(trimethylsilyl)ethoxy)methyl)-1H-indazole (Compound 28)

The synthesis of compound 28 from compound 27 was performed the same as step 6 in Example 1 (yield: 59.6%). $^1$H NMR (400 MHz, DMSO-$d_6$) δ 8.61 (ddd, *J* = 4.8, 1.8, 0.9 Hz, 1H), 8.52 - 8.45 (m, 1H), 7.93 (d, *J* = 16.4 Hz, 1H), 7.87 - 7.70 (m, 3H), 7.68 - 7.57 (m, 2H), 7.29 (ddd, *J*= 7.5, 4.7, 1.2 Hz, 1H), 5.79 (s, 2H), 3.54 (dd, *J*= 8.5, 7.4 Hz, 2H), 0.87 - 0.73 (m, 2H), -0.12 (s, 9H). LCMS (ESI), m/z: 430.1 $[M+H]^+$

Step 4: Synthesis of (E)-N-(3,5-difluorophenyl)-3-(2-(pyridin-2-yl)vinyl)-1-((2-(trimethylsilyl)ethoxy)methy 1)-1H-indazol-5-amine (Compound 29)

Compound 28 (500 mg, 1.16 mmol), 3,5-difluoroaniline (225 mg, 1.74 mmol), cesium carbonate (756 mg, 2.32 mmol), $Pd_2(dba)_3$ (55 mg, 0.06 mmol), and Xantphos (69 mg, 0.12 mmol) were added sequentially to a two-necked flask with argon replacement for three times. 20 mL of anhydrous dioxane was added, argon replacement was performed for three times, and a reaction was carried out at 90°C overnight. After the reaction was completed, dioxane was removed by evaporation, and water was added, followed by extraction with ethyl acetate. The organic layer was washed with saturated brine, dried over anhydrous sodium sulfate, filtered by suction, and evaporated to dryness. Column chromatography was performed to give 232 mg of compound 29 (yield: 41.7%). $^1$H NMR (400 MHz, DMSO-$d_6$) δ 8.71 (s, 1H), 8.60 (ddd, *J* = 4.8, 1.8, 0.9 Hz, 1H), 7.97 - 7.85 (m, 2H), 7.85 - 7.74 (m, 2H), 7.69 (dt, *J* = 7.9, 1.1 Hz, 1H), 7.52 (d, *J* = 16.4 Hz, 1H), 7.35 (dd, *J* = 8.9, 2.0 Hz, 1H), 7.28 (ddd, *J* = 7.5, 4.8, 1.2 Hz, 1H), 6.61 - 6.40 (m, 3H), 5.77 (s, 2H), 3.56 (dd, *J*= 8.5, 7.4 Hz, 2H), 0.89 - 0.74 (m, 2H), -0.10 (s, 9H). LCMS (ESI), m/z: 479.2 $[M+H]^+$

Step 5: Synthesis of (E)-N-(3,5-difluorophenyl)-3-(2-(pyridin-2-yl)vinyl)-1H-indazol-5-amine (Compound 30)

Compound 29 (200 mg, 0.42 mml) was dissolved in 5 mL of THF, and TBAF (1.1 g, 4.2 mmol) and ethylenediamine (252 mg, 4.2 mmol) were added. A reflux reaction was carried out for 6 hours. After the reaction was completed, THF was removed by evaporation, and water was added, followed by extraction with ethyl acetate. The organic layer was washed with saturated brine, dried over anhydrous sodium sulfate, filtered by suction, and evaporated to dryness. Column chromatography was performed to give 90 mg of compound 30 (yield: 61.6%).

$^1$H NMR (400 MHz, DMSO-$d_6$) δ 13.33 (s, 1H), 8.74 - 8.52 (m, 2H), 7.97 (d, *J* = 16.4 Hz, 1H), 7.90 - 7.78 (m, 2H), 7.71 (d, *J* = 7.9 Hz, 1H), 7.59 (d, *J* = 8.8 Hz, 1H), 7.48 (d, *J* = 16.3 Hz, 1H), 7.28 (t, *J* = 6.6 Hz, 2H), 6.49 (dd, *J* = 34.8, 9.5 Hz, 3H).

LCMS (ESI), m/z: 349.1 $[M+H]^+$

Example 21: Preparation of (E)-N-(3,5-difluorophenyl)-N-methyl-3-(2-(pyridin-2-yl)vinyl)-1H-indazol-5-amine (designated as DYX-139)

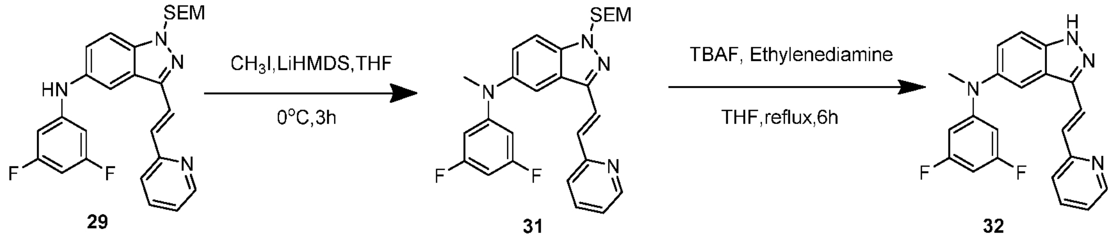


29 31 32

Step 1: Synthesis of (E)-N-(3,5-difluorophenyl)-N-methyl-3-(2-(pyridin-2-yl)vinyl)-1-((2-(trimethylsilyl)eth oxy)methyl)-1H-indazol-5-amine (Compound 31)

Raw material 29 (200mg, 0.42mmol) was dissolved in 10mL of anhydrous THF, and argon replacement was carried out for three times. Iodomethane (120mg, 0.84mmol) was added, and LiHMDS (210mg, 1.25mmol) was added slowly dropwise under stirring in an ice bath. After the addition, the reaction was performed at 0°C for 3 hours. After the reaction was completed, saturated ammonium chloride solution was added for quenching. After THF was removed by evaporation. water was added. Extraction was performed with ethyl acetate, and the organic layer was washed with saturated brine, dried over anhydrous sodium sulfate, filtered by suction and evaporated to dryness. Column chromatography was performed to give 144 mg of compound 31 (yield: 70.0%).

$^1$H NMR (400 MHz, DMSO-$d_6$) $\delta$ 8.60 (ddd, *J* = 4.8, 1.8, 0.8 Hz, 1H), 8.18 - 8.10 (m, 1H), 7.95 (d, *J*= 16.4 Hz, 1H), 7.90 - 7.84 (m, 1H), 7.81 (td, *J*= 7.6, 1.8 Hz, 1H), 7.71 (dt, *J* = 8.0, 1.1 Hz, 1H), 7.61 (d, *J* = 16.3 Hz, 1H), 7.38 (dd, *J* = 8.9, 1.9 Hz, 1H), 7.28 (ddd, *J* = 7.5, 4.8, 1.2 Hz, 1H), 6.45 (tt, *J* = 9.2, 2.3 Hz, 1H), 6.33 - 6.18 (m, 2H), 5.82 (s, 2H), 3.58 (dd, *J* = 8.4, 7.4 Hz, 2H), 3.37 (s, 3H), 0.88 - 0.79 (m, 2H), -0.11 (s, 9H). LCMS (ESI), m/z: 493.2 $[M+H]^+$

Step 2: Synthesis of (E)-N-(3,5-difluorophenyl)-N-methyl-3-(2-(pyridin-2-yl)vinyl)-1H-indazol-5-amine (Compound 32)

Compound 32 was synthesized as same as step 5 in Example 20 (yield: 82.4%).

$^1$H NMR (400 MHz, DMSO-$d_6$) $\delta$ 13.43 (s, 1H), 8.63 - 8.54 (m, 1H), 8.10 (d, *J* = 1.9 Hz, 1H), 7.97 (d, *J* = 16.4 Hz, 1H), 7.79 (td, *J* = 7.7, 1.9 Hz, 1H), 7.74 - 7.61 (m, 2H), 7.55 (d, *J* = 16.4 Hz, 1H), 7.31 - 7.19 (m, 2H), 6.43 (tt, *J* = 9.3, 2.3 Hz, 1H), 6.33 - 6.14 (m, 2H), 3.34 (s, 3H).

LCMS (ESI), m/z: 363.1 $[M+H]^+$

Example 22: Preparation of (E)-5-((3,5-difluorobenzyl)oxo)-3-(2-(pyridin-2-yl)vinyl)-1H-indazole (designated as DYX-132)

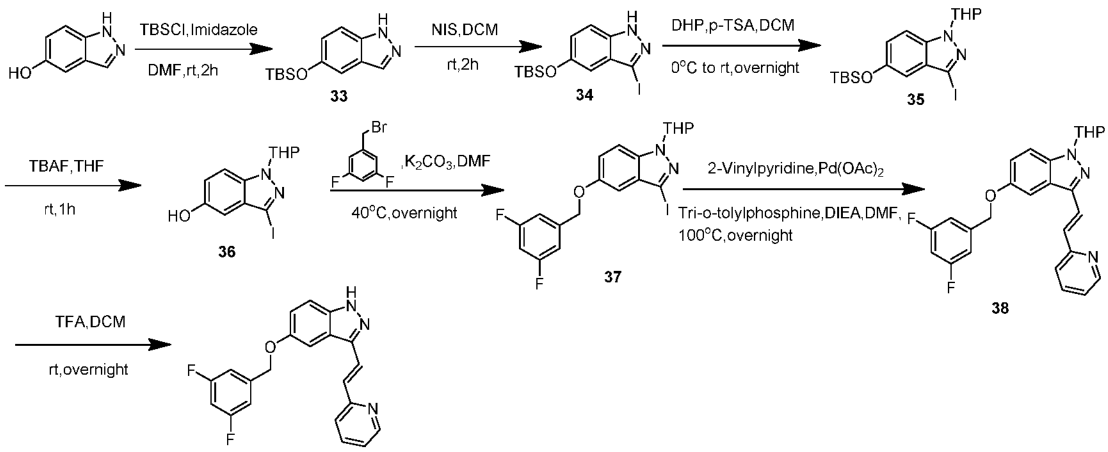


33 34 35 36 37 38 39

Step 1: Synthesis of 5-((tert-butyldimethylsilyl)oxo)-1H-indazole (Compound 33)

1H-indazol-5-ol (3 g, 22.4 mmol) and imidazole (3.05 g, 44.8 mmol) were dissolved in 30 mL of DMF. TBSCl (5.06 g, 33.6 mmol) was slowly added, and the mixture was stirred at room temperature for 2 hours. After the reaction was completed, water was added. Extraction was performed with ethyl acetate for three times, and the organic layer

was washed with water for four times, washed once with saturated brine, dried over anhydrous sodium sulfate, filtered by suction, and evaporated to remove the solvent to obtain 4.96 g of compound 33 (yield: 89.2%). $^1$H NMR (400 MHz, DMSO-$d_6$) δ 12.92 (s, 1H), 7.92 (t, *J* = 1.3 Hz, 1H), 7.41 (dt, *J* = 8.9, 0.9 Hz, 1H), 7.12 (d, *J* = 2.2 Hz, 1H), 6.91 (dd, *J* = 8.8, 2.3 Hz, 1H), 0.96 (s, 9H), 0.17 (s, 6H). LCMS (ESI), m/z: 249.1 $[M+H]^+$

Step 2: Synthesis of 5-((tert-butyldimethylsilyl)oxo)-3-iodo-1H-indazole (Compound 34)

Compound 33 (4 g, 16.1 mmol) was dissolved in 40 mL of DCM. NIS (5.4 g, 24.2 mmol) was added, and the mixture was stirred at room temperature for 2 hours. After completion of the reaction, saturated sodium thiosulfate solution was added for quenching, followed by extraction with DCM. The organic layer was washed with saturated brine, dried over anhydrous sodium sulfate, filtered by suction, and evaporated to dryness to give 5.61 g of compound 34 (yield: 93.1%). $^1$H NMR (400 MHz, DMSO-$d_6$) δ 13.40 (s, 1H), 7.45 (dd, *J* = 8.9, 2.4 Hz, 1H), 7.00 (dt, *J* = 9.1, 2.4 Hz, 1H), 6.71 (d, *J* = 2.2 Hz, 1H), 0.96 (d, *J* = 2.7 Hz, 9H), 0.18 (d, *J* = 2.7 Hz, 6H). LCMS (ESI), m/z: 375.0 $[M+H]^+$

Step 3: Synthesis of 5-((tert-butyldimethylsilyl)oxo)-3-iodo-1-(tetrahydro-2H-pyran-2-yl)-1H-indazole (Compound 35)

The synthesis of compound 35 from compound 34 was performed the same as step 1 in Example 1 (yield: 76.7%). $^1$H NMR (400 MHz, DMSO-$d_6$) δ 7.66 (d, *J* = 9.0 Hz, 1H), 7.09 (dd, *J* = 9.0, 2.2 Hz, 1H), 6.73 (d, *J* = 2.3 Hz, 1H), 5.80 (dd, *J* = 9.9, 2.3 Hz, 1H), 3.87 (dt, *J* = 13.2, 3.0 Hz, 1H), 3.71 (ddd, *J* = 11.4, 8.0, 5.8 Hz, 1H), 2.43 - 2.26 (m, 1H), 1.98 (tq, *J* = 16.3, 4.1, 3.4 Hz, 2H), 1.72 (tdd, *J* = 15.7, 8.7, 5.0 Hz, 1H), 1.57 (dq, *J* = 8.9, 5.0, 4.3 Hz, 2H), 0.97 (s, 9H), 0.20 (s, 6H). LCMS (ESI), m/z: 459.1 $[M+H]^+$

Step 4: Synthesis of 3-iodo-1-(tetrahydro-2H-pyran-2-yl)-1H-indazol-5-ol (Compound 36)

Compound 35 (2 g, 4.4 mmol) was dissolved in 20 mL of THF, and TBAF (5.7 g, 22 mmol) was added under stirring at room temperature. After the addition was completed, the stirring was continued for 2 hours. After the reaction was completed, THF was removed by evaporation, and water was added, followed by extraction with ethyl acetate. The organic layer was washed with saturated brine, dried over anhydrous sodium sulfate, filtered by suction, and evaporated to dryness. Column chromatography was performed to give 1.23 g of compound 36 (yield. 82.0%). $^1$H NMR (400 MHz, DMSO-$d_6$) δ 9.52 (s, 1H), 7.57 (d, *J* = 9.0 Hz, 1H), 7.02 (dd, *J* = 8.9, 2.3 Hz, 1H), 6.65 (d, *J* = 2.2 Hz, 1H), 5.74 (dd, *J* = 9.8, 2.5 Hz, 1H), 3.91 - 3.80 (m, 1H), 3.69 (ddd, *J* = 11.4, 8.0, 5.8 Hz, 1H), 2.40 - 2.24 (m, 1H), 2.07 - 1.87 (m, 2H), 1.81 - 1.61 (m, 1H), 1.55 (hept, *J* = 3.9 Hz, 2H).

LCMS (ESI), m/z: 345.0 $[M+H]^+$

Step 5: Synthesis of 5-((3,5-difluorobenzyl)oxo)-3-iodo-1-(tetrahydro-2H-pyran-2-yl)-1H-indazole (Compound 37)

Compound 36 (1.2 g, 3.5 mmol) was dissolved in DMF. 3,5-difluorobenzyl bromide (1.45 g, 7 mmol) and potassium carbonate (960 mg, 7 mmol) were added, and a reaction was carried out at 40°C overnight. After the reaction was completed, ice water was added, and stirred for 5 minutes to precipitate solid, followed by suction filtration. The obtained filter residue was washed with water for three times, dried and slurried with diethyl ether to give 1.45 g of compound 37 (yield: 88.6%). $^1$H NMR (400 MHz, DMSO-$d_6$) δ 7.70 (d, *J* = 9.1 Hz, 1H), 7.23 (dddd, *J* = 20.1, 11.7, 6.9, 2.4 Hz, 4H), 6.89 (d, *J* = 2.4 Hz, 1H), 5.80 (dd, *J* = 9.8, 2.3 Hz, 1H), 5.22 (s, 2H), 3.86 (dq, *J* = 11.7, 3.0 Hz, 1H), 3.71 (ddd, *J* = 11.4, 8.3, 5.9 Hz, 1H), 2.41 - 2.23 (m, 1H), 1.97 (ddq, *J*= 19.2, 12.4, 3.1 Hz, 2H), 1.71 (dtdt, *J* = 11.2, 8.7, 6.3, 2.9 Hz, 1H), 1.55 (dq, *J* = 8.6, 4.4, 3.8 Hz, 2H). LCMS (ESI), m/z: 471.0 $[M+H]^+$

Compounds 38 to 39 were synthesized the same as step 6-7 in Example 1 (yield: 70.2%).

$^1$H NMR (400 MHz, DMSO-$d_6$) δ 13.23 (s, 1H), 8.64 - 8.56 (m, 1H), 7.96 (d, *J* = 16.3 Hz, 1H), 7.81 (td, *J* = 7.6, 1.8 Hz, 1H), 7.70 - 7.60 (m, 2H), 7.55 - 7.43 (m, 2H), 7.32 - 7.14 (m, 5H), 5.28 (s, 2H).

LCMS (ESI), m/z: 364.1 $[M+H]^+$

Example 23: Preparation of (E)-5-((3,5-difluorophenylmethoxy)methyl)-3-(2-(pyridin-2-yl)vinyl)-1H-indazole (designated as DYX-176)

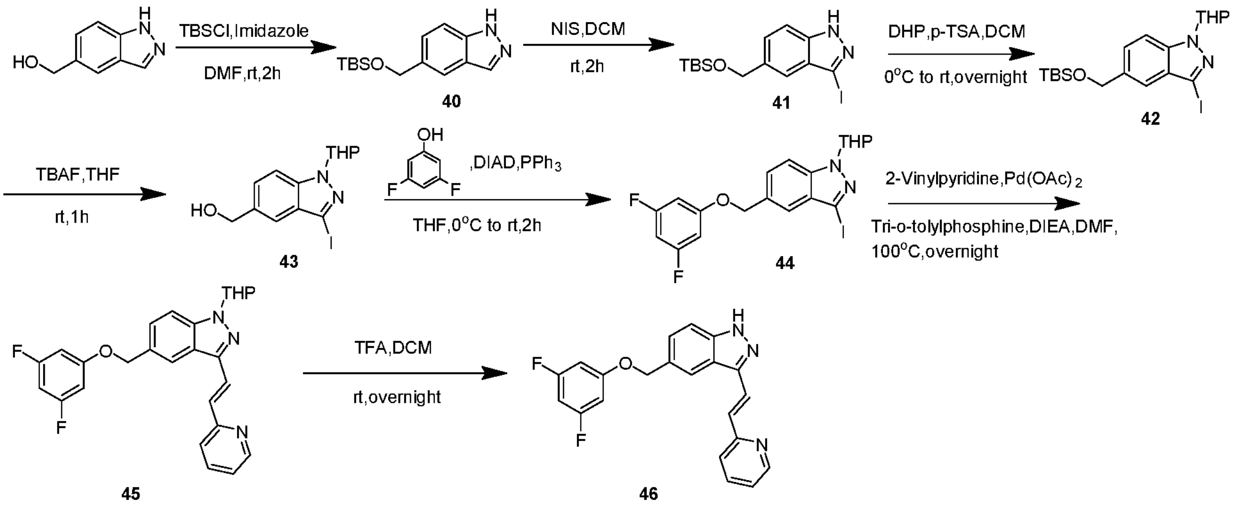


The synthesis in step 1-4 was performed as same as step 1-4 in Example 22.

Step 5: Synthesis of 5-((3,5-difluorophenoxy)methyl)-3-iodo-1-(tetrahydro-2H-pyran-2-yl)-1H-indazole (Compound 44)

Compound 43 (1.5 g, 4.2 mmol), triphenylphosphine (1.3 g, 5 mmol), and 3,5-difluorophenol (650 mg, 5 mmol) were dissolved in 10 mL of anhydrous THF with argon replacement for three times. DIAD (1.01 g, 5 mmol) was added slowly dropwise by a syringe under stirring in an ice bath. After the addition was completed, a reaction was carried out for 2 hours. After the reaction was completed, THF was removed by evaporation, and water was added, followed by extraction with ethyl acetate. The organic layer was washed with saturated brine, dried over anhydrous sodium sulfate, filtered by suction, and evaporated to dryness. Column chromatography was performed to give 1.17 g of compound 44 (yield: 59.4%). $^1$H NMR (400 MHz, DMSO-$d_6$) $\delta$ 7.79 (dd, $J$ = 8.6, 0.8 Hz, 1H), 7.61 - 7.53 (m, 2H), 6.89 - 6.73 (m, 3H), 5.86 (dd, $J$ = 9.8, 2.3 Hz, 1H), 5.26 (s, 2H), 3.87 (dq, $J$ = 11.3, 3.2 Hz, 1H), 3.72 (ddd, $J$= 11.4, 8.3, 6.0 Hz, 1H), 2.44 - 2.28 (m, 1H), 1.99 (tdt, $J$ = 16.6, 6.6, 3.1 Hz, 2H), 1.83 - 1.63 (m, 1H), 1.58 (dhept, $J$ = 8.2, 3.6 Hz, 2H). LCMS (ESI), m/z: 470 $[M+H]^+$

Compounds 45-46 were synthesized as same as step 6-7 in Example 1 (yield: 63.3%).

$^1$H NMR (300 MHz, DMSO-$d_6$) $\delta$ 13.52 (s, 1H), 8.65 - 8.55 (m, 1H), 8.33 (s, 1H), 7.97 (d, $J$ = 16.4 Hz, 1H), 7.82 (td, $J$ = 7.6, 1.8 Hz, 1H), 7.74 - 7.44 (m, 4H), 7.33 - 7.21 (m, 1H), 6.94 - 6.73 (m, 3H), 5.27 (s, 2H). LCMS (ESI), m/z: 364.1 $[M+H]^+$

Example 24: Preparation of (E)-3,5-difluoro-N-((3-(2-(pyridin-2-yl)vinyl)-1H-indazol-5-yl)methyl)aniline (designated as DYX-191)

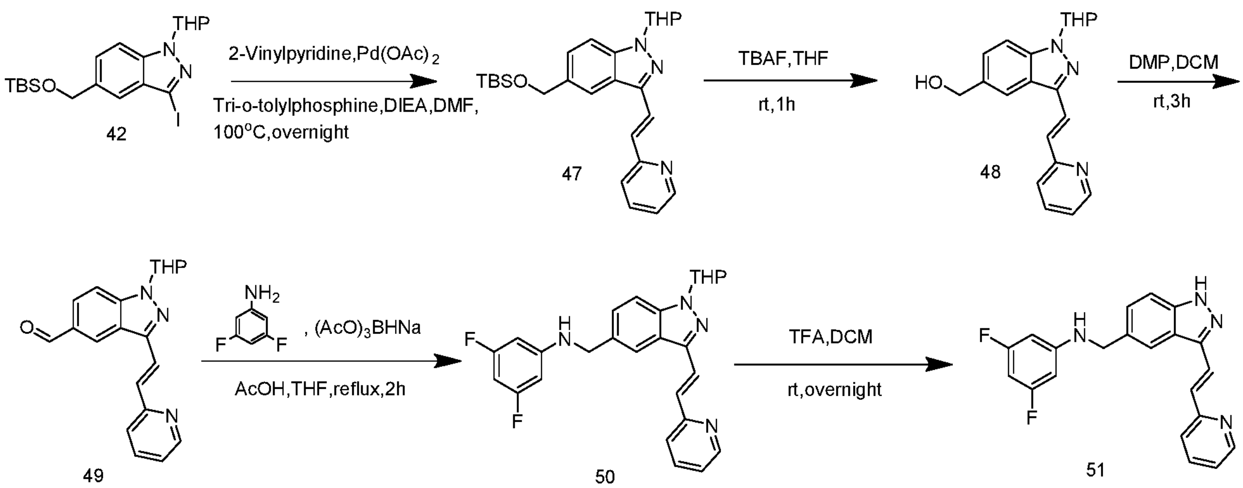

Step 1: Synthesis of (E)-5-(((tert-butyldimethylsilyl)oxo)methyl)-3-(2-(pyridin-2-yl)vinyl)-1-(tetrahydro-2H-pyran-2-yl)-1H-indazole (Compound 47)

Compound 47 was synthesized as same as step 6 in Example 1 (yield: 61.7%). $^1$H NMR (400 MHz, DMSO-*d*$_6$) δ 8.61 (ddd, *J* = 4.8, 1.8, 0.9 Hz, 1H), 8.12 - 8.04 (m, 1H), 7.93 (d, *J* = 16.4 Hz, 1H), 7.82 (td, *J* = 7.7, 1.8 Hz, 1H), 7.74 (dd, *J* = 8.7, 0.7 Hz, 1H), 7.65 (dt, *J* = 7.9, 1.1 Hz, 1H), 7.55 (d, *J* = 16.4 Hz, 1H), 7.43 (dd, *J* = 8.7, 1.4 Hz, 1H), 7.28 (ddd, *J* = 7.5, 4.8, 1.1 Hz, 1H), 5.86 (dd, *J* = 9.7, 2.5 Hz, 1H), 4.87 (s, 2H), 3.95 - 3.84 (m, 1H), 3.75 (dq, *J* = 9.5, 6.0 Hz, 1H), 2.49 - 2.34 (m, 1H), 2.11 - 1.93 (m, 2H), 1.75 (qd, *J* = 11.2, 3.5 Hz, 1H), 1.59 (h, *J* = 4.7, 3.9 Hz, 2H). LCMS (ESI), m/z: 450.2 $[M+H]^+$

Step 2: Synthesis of (E)-(3-(2-(pyridin-2-yl)vinyl)-1-(tetrahydro-2H-pyran-2-yl)-1H-indazol-5-yl)methanol (Compound 48)

Compound 48 was synthesized as same as step 4 in Example 22 (yield: 83.6%). $^1$H NMR (400 MHz, DMSO-*d*$_6$) δ 8.65 - 8.57 (m, 1H), 8.08 (s, 1H), 7.93 (d, *J* = 16.4 Hz, 1H), 7.82 (td, *J* = 7.6, 1.9 Hz, 1H), 7.73 (dd, *J* = 8.3, 3.0 Hz, 2H), 7.57 (d, *J* = 16.4 Hz, 1H), 7.45 (dd, *J* = 8.6, 1.4 Hz, 1H), 7.29 (ddd, *J* = 7.5, 4.8, 1.2 Hz, 1H), 5.88 (dd, *J* = 9.7, 2.5 Hz, 1H), 5.27 (t, *J* = 5.7 Hz, 1H), 4.66 (d, *J* = 5.0 Hz, 2H), 3.97 - 3.84 (m, 1H), 3.84 - 3.65 (m, 1H), 2.47 - 2.36 (m, 1H), 2.03 (dddd, *J* = 28.0, 12.7, 6.0, 3.5 Hz, 2H), 1.77 (dq, *J* = 12.3, 9.0, 8.3 Hz, 1H), 1.60 (dq, *J* = 8.0, 4.7, 3.8 Hz, 2H). LCMS (ESI), m/z: 336.1 $[M+H]^+$

Step 3: Synthesis of (E)-3-(2-(pyridin-2-yl)vinyl)-1-(tetrahydro-2H-pyran-2-yl)-1H-indazole-5-aldehyde (Compound 49)

Compound 48 (1 g, 3 mmol) was dissolved in 10 mL of DCM. Dess-martin periodinane (DMP) (1.9 g, 4.5 mmol) was added, and the mixture was stirred at room temperature for 3 hours. After the reaction was completed, water was added for extraction, and the organic layer was washed with saturated brine, dried over anhydrous sodium sulfate, filtered by suction, and evaporated to dryness. Column chromatography was performed to give 740 mg of compound 49 (yield: 74%). $^1$H NMR (400 MHz, DMSO-*d*$_6$) δ 10.13 (s, 1H), 8.89 (t, *J* = 1.1 Hz, 1H), 8.64 (ddd, *J* = 4.8, 1.7, 0.8 Hz, 1H), 8.03 - 7.93 (m, 3H), 7.85 (td, *J* = 7.6, 1.8 Hz, 1H), 7.77 - 7.69 (m, 2H), 7.32 (ddd, *J* = 7.5, 4.8, 1.2 Hz, 1H), 5.98 (dd, *J* = 9.7, 2.4 Hz, 1H), 3.98 - 3.86 (m, 1H), 3.87 - 3.72 (m, 1H), 2.49 - 2.34 (m, 1H), 2.13 - 1.96 (m, 2H), 1.77 (tq, *J* = 13.5, 7.0, 5.1 Hz, 1H), 1.61 (dq, *J* = 7.6, 5.0, 3.6 Hz, 2H). LCMS (ESI), m/z: 334.1 $[M+H]^+$

Step 4: Synthesis of (E)-3,5-difluoro-N-((3-(2-(pyridin-2-yl)vinyl)-1-(tetrahydro-2H-pyran-2-yl)-1H-indazol -5-yl)methyl)aniline (Compound 50)

Compound 49 (700 mg, 2.1 mmol), 3,5-difluoroaniline (325 mg, 2.52 mmol), and sodium triacetoxyborohydride (890 mg, 4.2 mmol) were dissolved in 10 mL of anhydrous THF. 2 drops of acetic acid were added dropwise , and a reflux reaction was carried out for 2 hours. After the reaction was completed, THF was removed by evaporation, and water was added, followed by extraction with ethyl acetate. The organic layer was washed with saturated brine, dried over anhydrous sodium sulfate, filtered by suction, and evaporated to dryness. Column chromatography was performed to give 412 mg of compound 50 (yield: 43.9%). $^1$H NMR (400 MHz, DMSO-*d*$_6$) δ 8.61 (dd, *J* = 4.9, 1.7 Hz, 1H), 8.18 (s, 1H), 7.92 (d, *J* = 16.4 Hz, 1H), 7.83 (td, *J* = 7.7, 1.9 Hz, 1H), 7.71 (dd, *J* = 28.4, 8.2 Hz, 2H), 7.57 (d, *J* = 16.4 Hz, 1H), 7.48 (dd, *J* = 8.7, 1.5 Hz, 1H), 7.29 (ddd, *J* = 7.6, 4.8, 1.2 Hz, 1H), 6.95 (t, *J* = 5.9 Hz, 1H), 6.36 - 6.13 (m, 3H), 5.87 (dd, *J* = 9.7, 2.5 Hz, 1H), 4.43 (d, *J* = 5.9 Hz, 2H), 3.90 (d, *J* = 11.5 Hz, 1H), 3.79 - 3.69 (m, 1H), 2.48 - 2.31 (m, 1H), 2.12 - 1.92 (m, 2H), 1.75 (d, *J* = 12.9 Hz, 1H), 1.60 (dp, *J* = 8.5, 4.2 Hz, 2H). LCMS (ESI), m/z: 447.2 $[M+H]^+$

Step 5: Synthesis of (E)-3,5-difluoro-N-((3-(2-(pyridin-2-yl)vinyl)-1H-indazol-5-yl)methyl)aniline (Compound 51)

Compound 51 was synthesized as same as step 7 in Example 1 (yield: 75.5%).

$^1$H NMR (400 MHz, DMSO-*d*$_6$) δ 13.27 (s, 1H), 8.61 (dd, *J* = 4.9, 1.7 Hz, 1H), 8.17 (s, 1H), 7.95 (d, *J* = 16.4 Hz, 1H), 7.82 (td, *J* = 7.7, 1.9 Hz, 1H), 7.65 (d, *J* = 7.8 Hz, 1H), 7.60 - 7.48 (m, 2H), 7.42 (dd, *J* = 8.6, 1.5 Hz, 1H), 7.27 (dd, *J* = 7.4, 4.8 Hz, 1H), 6.93 (t, *J* = 5.9 Hz, 1H), 6.36 - 6.26 (m, 2H), 6.20 (tt, *J* = 9.6, 2.3 Hz, 1H), 4.42 (d, *J* = 5.8 Hz, 2H).

LCMS (ESI), m/z: 363.1 $[M+H]^+$

Example 25: Preparation of (E)-3,5-difluoro-N-methyl-N-((3-(2-(pyridin-2-yl)vinyl)-1H-indazol-5-yl)methyl)aniline (designated as DYX-193)

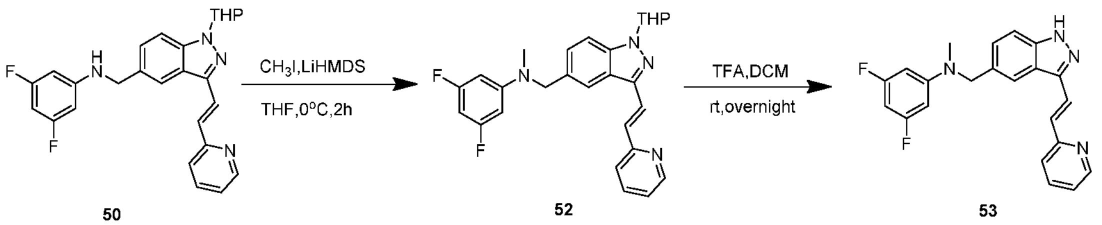


50 52 53

The synthesis of compound 52 from compound 50 was performed as same as step 1-2 in Example 21 (yield: 73.1%).

Compound 52: $^1$H NMR (400 MHz, DMSO-$d_6$) $\delta$ 8.61 (ddd, $J$ = 4.8, 1.8, 0.8 Hz, 1H), 8.06 - 7.98 (m, 1H), 7.90 (d, $J$ = 16.3 Hz, 1H), 7.82 (td, $J$ = 7.7, 1.8 Hz, 1H), 7.73 (d, $J$ = 8.7 Hz, 1H), 7.65 (dt, $J$= 8.0, 1.1 Hz, 1H), 7.52 (d, $J$= 16.4 Hz, 1H), 7.34 - 7.25 (m, 2H), 6.51 - 6.42 (m, 2H), 6.32 (tt, $J$ = 9.3, 2.2 Hz, 1H), 5.86 (dd, $J$ = 9.7, 2.5 Hz, 1H), 4.75 (s, 2H), 3.89 (d, $J$= 11.6 Hz, 1H), 3.82 - 3.68 (m, 1H), 3.07 (s, 3H), 2.48 - 2.36 (m, 1H), 2.10 - 1.94 (m, 2H), 1.75 (d, $J$ = 12.8 Hz, 1H), 1.59 (p, $J$ = 4.9, 4.3 Hz, 2H). LCMS (ESI), m/z: 461.2 $[M+H]^+$

Compound 53: $^1$H NMR (400 MHz, DMSO-$d_6$) $\delta$ 13.28 (s, 1H), 8.61 (dd, $J$= 4.9, 1.7 Hz, 1H), 8.06 - 7.89 (m, 2H), 7.82 (td, $J$ = 7.7, 1.9 Hz, 1H), 7.63 (d, $J$ = 7.8 Hz, 1H), 7.57 - 7.45 (m, 2H), 7.32 - 7.19 (m, 2H), 6.54 - 6.41 (m, 2H), 6.33 (tt, $J$ = 9.3, 2.2 Hz, 1H), 4.75 (s, 2H), 3.07 (s, 3H).

LCMS (ESI), m/z: 377.1 $[M+H]^+$

Example 26: Preparation of (E)-3,5-difluoro-N-(3-(2-(pyridin-2-yl)vinyl)-1H-indazol-5-yl)benzamide (designated as DYX-164)

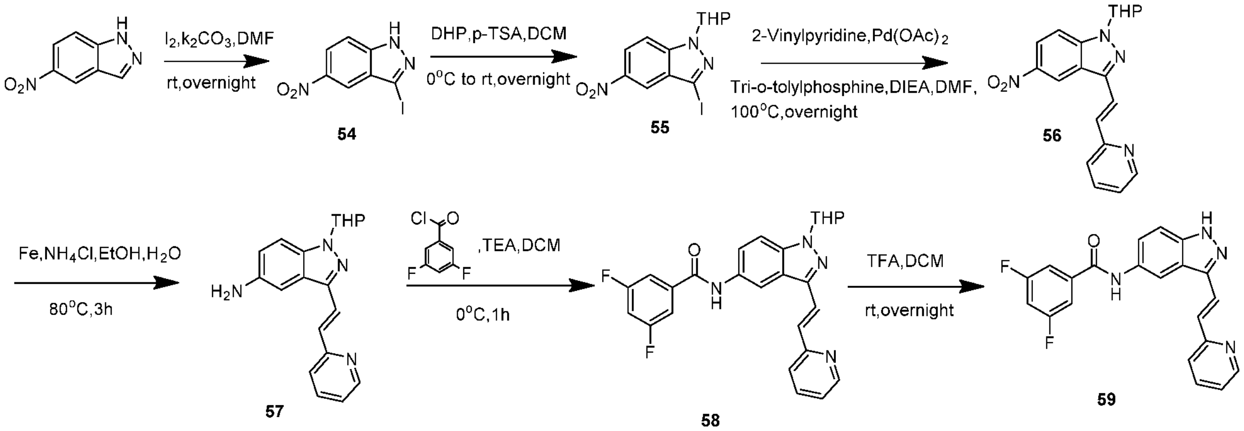


54 55 56 57 58 59

Steps 1-3: Compound 54-56 were synthesized the same as step 4-6 in Example 1.

Step 4: Synthesis of (E)-3-(2-(pyridin-2-yl)vinyl)-1-(tetrahydro-2H-pyran-2-yl)-1H-indazol-5-amine (Compound 57)

The synthesis of compound 57 from compound 56 was performed the same as step 2 in Example 17 (yield: 82.1%).

$^1$H NMR (400 MHz, DMSO-$d_6$) $\delta$ 8.59 (d, $J$ = 4.7 Hz, 1H), 7.89 - 7.70 (m, 2H), 7.64 (d, $J$ = 7.9 Hz, 1H), 7.53 - 7.31 (m, 2H), 7.31 - 7.07 (m, 2H), 6.88 (d, $J$ = 8.9 Hz, 1H), 5.73 (d, $J$ = 9.5 Hz, 1H), 5.06 (s, 2H), 3.89 (d, $J$= 11.4 Hz, 1H), 3.72 (q, $J$ = 6.3 Hz, 1H), 2.38 (t, $J$ = 12.8 Hz, 1H), 1.99 (dd, $J$ = 35.4, 13.1 Hz, 2H), 1.72 (q, $J$ = 10.3, 9.7 Hz, 1H), 1.66 - 1.44 (m, 2H). LCMS (ESI), m/z: 321.2 $[M+H]^+$

Step 5: Synthesis of (E)-3,5-difluoro-N-(3-(2-(pyridin-2-yl)vinyl)-1-(tetrahydro-2H-pyran-2-yl)-1H-indazol-5-yl)benzamide (Compound 58)

Compound 57 (500 mg, 1.56 mmol) was dissolved in 10 mL of anhydrous DCM. Triethylamine (473 mg, 4.68 mmol) was added, and 3,5-difluorobenzoyl chloride (303 mg, 1.72 mmol) was added dropwise under stirring in an ice bath. After the addition was completed, a reaction was continued for 1 hour. After the reaction was completed, water

was added, followed by extraction with DCM. The organic layer was washed with saturated brine, dried over anhydrous sodium sulfate, filtered by suction, and evaporated to dryness. Column chromatography was performed to give 620 mg of compound 58 (yield: 86.2%).

$^{1}H$ NMR (400 MHz, DMSO-$d_6$) δ 10.54 (s, 1H), 8.61 (ddt, J= 5.8, 2.5, 1.0 Hz, 2H), 7.92 (d, *J* = 16.4 Hz, 1H), 7.86 - 7.67 (m, 6H), 7.61 - 7.46 (m, 2H), 7.30 (ddd, *J* = 7.5, 4.8, 1.2 Hz, 1H), 5.89 (dd, *J*= 9.8, 2.3 Hz, 1H), 3.98 - 3.86 (m, 1H), 3.77 (ddd, *J* = 12.6, 9.0, 4.6 Hz, 1H), 2.43 (ddd, *J* = 13.2, 9.6, 3.6 Hz, 1H), 2.11 - 1.96 (m, 2H), 1.76 (d, *J* = 12.6 Hz, 1H), 1.61 (dq, *J* = 8.0, 4.9, 3.8 Hz, 2H).

LCMS (ESI), m/z: 461.2 $[M+H]^+$

Step 6: Synthesis of (E)-3,5-difluoro-N-(3-(2-(pyridin-2-yl)vinyl)-1H-indazol-5-yl)benzamide (Compound 59)

The synthesis of compound 59 from compound 58 was performed the same as step 7 in Example 1 (yield: 70.9%).

$^{1}H$ NMR (400 MHz, DMSO-$d_6$) δ 13.33 (s, 1H), 10.54 (s, 1H), 8.64 - 8.56 (m, 2H), 7.95 (d, *J*= 16.4 Hz, 1H), 7.85 - 7.71 (m, 4H), 7.71 - 7.43 (m, 4H), 7.32 - 7.22 (m, 1H). LCMS (ESI), m/z: 377.1 $[M+H]^+$

Example 27: Preparation of (E)-3,5-difluoro-N-(3-(2-(pyridin-2-yl)vinyl)-1H-indazol-5-yl)benzenesulfonamide (designated as DYX-165)

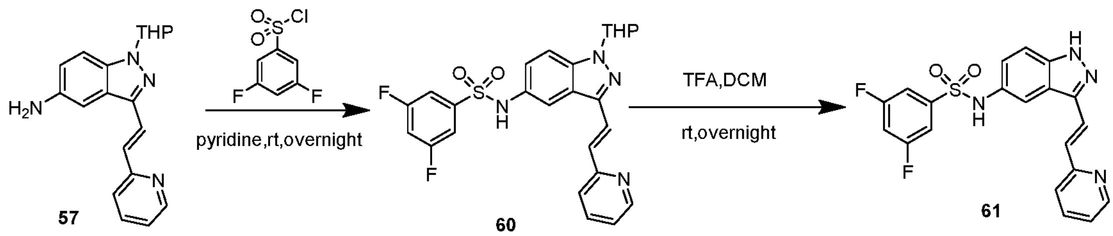

57
60
61

Step 1: Synthesis of (E)-3,5-difluoro-N-(3-(2-(pyridin-2-yl)vinyl)-1-(tetrahydro-2H-pyran-2-yl)-1H-indazol-5-yl)benzenesulfonamide (Compound 60)

Compound 57 (500 mg, 1.56 mmol) was dissolved in 10 mL of pyridine. 3,5-difluorobenzenesulfonyl chloride (365 mg, 1.72 mmol) was slowly added, and the reaction was carried out overnight. After the reaction was completed, 20 mL of ethyl acetate was added. Extraction was performed twice with 1 M of hydrochloric acid, and the pyridine was washed off. The organic layer was washed with saturated brine, dried over anhydrous sodium sulfate, filtered by suction, and evaporated to dryness. Column chromatography was performed to give 653 mg of compound 60 (yield: 84.3%). $^{1}H$ NMR (400 MHz, DMSO-$d_6$) δ 10.48 (s, 1H), 8.67 - 8.58 (m, 1H), 7.89 - 7.80 (m, 2H), 7.79 (d, *J* = 1.9 Hz, 1H), 7.71 (dd, *J* = 8.5, 2.9 Hz, 2H), 7.61 (tt, *J* = 9.1, 2.3 Hz, 1H), 7.47 - 7.36 (m, 3H), 7.31 (ddd, *J*= 7.5, 4.8, 1.2 Hz, 1H), 7.18 (dd, *J* = 9.0, 1.9 Hz, 1H), 5.83 (dd, *J* = 9.8, 2.4 Hz, 1H), 3.93 - 3.83 (m, 1H), 3.72 (ddd, *J* = 11.4, 8.1, 6.0 Hz, 1H), 2.47 - 2.31 (m, 1H), 2.08 - 1.92 (m, 2H), 1.72 (m, 1H), 1.58 (m, 2H). LCMS (ESI), m/z: 497.1 $[M+H]^+$

Step 2: Synthesis of (E)-3,5-difluoro-N-(3-(2-(pyridin-2-yl)vinyl)-1H-indazol-5-yl)benzenesulfonamide (Compound 61)

The synthesis of compound 61 from compound 60 was performed as same as step 7 in Example 1 (yield: 67.9%).

$^{1}H$ NMR (400 MHz, DMSO-$d_6$) δ 13.36 (s, 1H), 10.38 (s, 1H), 8.61 (dd, *J* = 4.8, 1.7 Hz, 1H), 7.92 - 7.75 (m, 3H), 7.66 (d, *J* = 7.9 Hz, 1H), 7.60 (tt, *J* = 9.1, 2.3 Hz, 1H), 7.50 (d, *J* = 8.8 Hz, 1H), 7.45 - 7.32 (m, 3H), 7.28 (ddd, *J* = 7.6, 4.7, 1.1 Hz, 1H), 7.12 (dd, *J* = 8.9, 1.9 Hz, 1H).

LCMS (ESI), m/z: 413 $[M+H]^+$

Example 28: Preparation of (E)-1-(3,5-difluorophenyl)-3-(3-(2-(pyridin-2-yl)vinyl)-1H-indazol-5-yl)urea (designated as DYX-174)

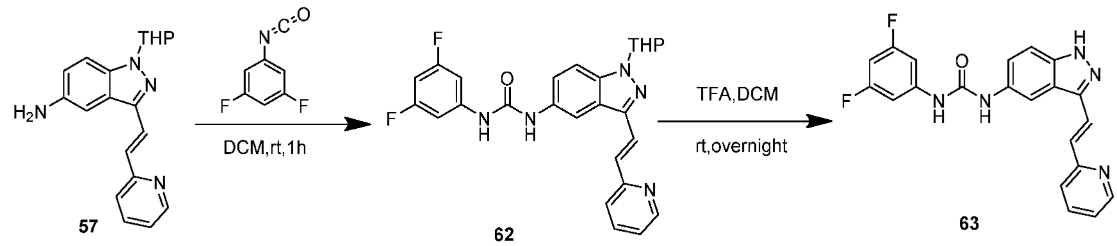


57 62 63

Step 1: Synthesis of (E)-1-(3,5-difluoro)-3-(3-(2-(pyridin-2-yl)vinyl)-1-(tetrahydro-2H-pyran-2-yl)-1H-indaz ol-5-yl)urea (Compound 62)

Compound 57 (500 mg, 1.56 mmol) was dissolved in 10 mL of DCM. 3,5-difluorophenyl isocyanate (266 mg, 1.72 mmol) was added slowly dropwise, and the mixture was stirred for 1 hour to precipitate solid. Suction filtration was performed. The obtained filter residue was washed with DCM for three times, and dried to give 660 mg of compound 62 (yield 89.0%).

$^1$H NMR (400 MHz, DMSO-$d_6$) $\delta$ 9.18 (s, 1H), 9.01 (s, 1H), 8.62 (ddd, *J* = 4.8, 1.8, 0.9 Hz, 1H), 8.30 (d, *J* = 2.1 Hz, 1H), 7.90 (d, *J* = 16.4 Hz, 1H), 7.82 (td, *J* = 7.7, 1.9 Hz, 1H), 7.76 - 7.68 (m, 2H), 7.51 - 7.40 (m, 2H), 7.32 - 7.19 (m, 3H), 6.81 (tt, *J* = 9.4, 2.4 Hz, 1H), 5.86 (dd, *J* = 9.8, 2.4 Hz, 1H), 3.96 - 3.84 (m, 1H), 3.84 - 3.69 (m, 1H), 2.42 (ddd, *J* = 13.0, 9.3, 3.5 Hz, 1H), 2.11 - 1.95 (m, 2H), 1.75 (d, *J* = 12.9 Hz, 1H), 1.60 (hept, *J* = 4.0 Hz, 2H). LCMS (ESI), m/z: 476.2 $[M+H]^+$

Step 2: Synthesis of (E)-1-(3,5-difluoro)-3-(3-(2-(pyridin-2-yl)vinyl)-1H-indazol-5-yl)urea (Compound 63)

The synthesis of compound 63 from compound 62 was performed the same as step 7 in Example 1 (yield: 78.5%).

$^1$H NMR (400 MHz, DMSO-$d_6$) $\delta$ 13.24 (s, 1H), 9.13 (s, 1H), 8.91 (s, 1H), 8.65 - 8.57 (m, 1H), 8.32 - 8.24 (m, 1H), 7.93 (d, *J* = 16.3 Hz, 1H), 7.81 (td, *J* = 7.7, 1.9 Hz, 1H), 7.67 (d, *J* = 7.8 Hz, 1H), 7.53 (d, *J* = 8.9 Hz, 1H), 7.49 - 7.33 (m, 2H), 7.25 (ddd, *J* = 14.9, 8.8, 3.6 Hz, 3H), 6.79 (tt, *J* = 9.5, 2.4 Hz, 1H).

LCMS (ESI), m/z: 392.1 $[M+H]^+$

Example 29: Preparation of (E)-N-(3,5-difluorobenzyl)-3-(2-(pyridin-2-yl)vinyl)-1H-indazol-5-amine (designated as DYX-185)

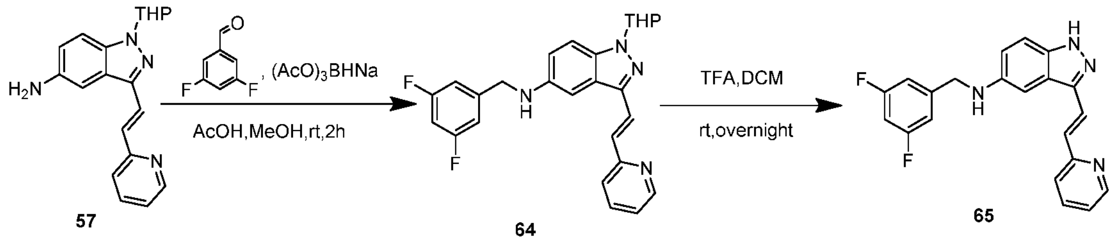


57 64 65

Step 1: Preparation of (E)-N-(3,5-difluorobenzyl)-3-(2-(pyridin-2-yl)vinyl)-1-(tetrahydro-2H-pyran-2-yl)-1H-i ndazol-5-amine (Compound 64)

Compound 57 (500 mg, 1.56 mmol) and 3,5-difluorobenzaldehyde (244 mg, 1.72 mmol) were dissolved in 10 mL of methanol, and 2 drops of acetic acid were added dropwise. The reaction was carried out at room temperature for 15 minutes to precipitate solid, and suction filtration was performed. The solid was dissolved in 10 mL of THF, and sodium triacetoxyborohydride (661 mg, 3.12 mmol) was added. The mixture was stirred at room temperature for 2 hours. After the reaction was completed, THF was removed by rotary evaporation, and water was added, followed by extraction with ethyl acetate. The organic layer was washed with saturated brine, dried over anhydrous sodium sulfate, filtered by suction, and evaporated to dryness. Column chromatography was performed to give 435 mg compound 64 (yield: 62.5%).

$^1$H NMR (400 MHz, DMSO-$d_6$) $\delta$ 8.59 (ddd, *J* = 4.8, 1.9, 0.9 Hz, 1H), 7.86 - 7.76 (m, 2H), 7.58 (dt, *J* = 7.9, 1.1 Hz, 1H), 7.53 (d, *J* = 8.9 Hz, 1H), 7.33 - 7.14 (m, 4H), 7.07 (tt, *J* = 9.4, 2.4 Hz, 1H), 7.00 - 6.92 (m, 2H), 6.43 (t, *J* = 6.3 Hz, 1H), 5.74 (dd, *J* = 9.7, 2.5 Hz, 1H), 4.44 (d, *J* = 6.2 Hz, 2H), 3.88 (d, *J* = 11.8 Hz, 1H), 3.78 - 3.64 (m, 1H), 2.45 - 2.27 (m, 1H), 2.07 - 1.87 (m, 2H), 1.72 (dd, *J* = 13.2, 7.1 Hz, 1H), 1.58 (h, *J* = 4.1 Hz, 2H). LCMS (ESI), m/z: 447.2 $[M+H]^+$

Step 2: Preparation of (E)-N-(3,5-difluorobenzyl)-3-(2-(pyridin-2-yl)vinyl)-1H-indazol-5-amine (Compound 65)

The synthesis of compound 65 from compound 64 was performed the same as step 7 in Example 1 (yield: 67.3%).
$^1$H NMR (400 MHz, DMSO-$d_6$) δ 12.95 (s, 1H), 8.60 - 8.54 (m, 1H), 7.88 - 7.75 (m, 2H), 7.54 (d, *J* = 8.0 Hz, 1H), 7.36 - 7.30 (m, 1H), 7.29 - 7.16 (m, 4H), 7.08 (tt, *J* = 9.3, 2.4 Hz, 1H), 6.97 - 6.90 (m, 2H), 6.36 (t, *J* = 6.2 Hz, 1H), 4.43 (d, *J* = 6.1 Hz, 2H).
LCMS (ESI), m/z: 363.1 [M+H]$^+$

Example 30: Preparation of (E)-N-(3,5-difluorobenzyl)-N-methyl-3-(2-(pyridin-2-yl)vinyl)-1H-indazol-5-amine (designated as DYX-187)

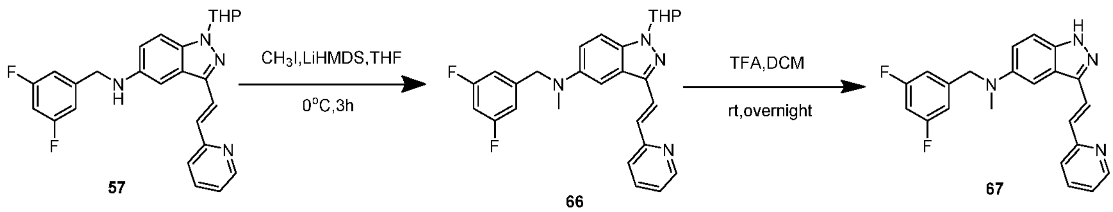


57 66 67

Step 1: Synthesis of (E)-N-(3,5-difluorobenzyl)-N-methyl-3-(2-(pyridin-2-yl)vinyl)-1-(tetrahydro-2H-pyran-2-yl)-1H-indazol-5-amine (Compound 66)

The synthesis of compound 66 from compound 57 was performed the same as step 1 in Example 21 (yield: 70.5%).
Compound 66: $^1$H NMR (400 MHz, DMSO-$d_6$) δ 8.59 (ddd, J= 4.8, 1.9, 0.9 Hz, 1H), 7.90 (d, *J* = 16.2 Hz, 1H), 7.80 (td, *J* = 7.6, 1.9 Hz, 1H), 7.65 (dt, *J* = 8.0, 1.1 Hz, 1H), 7.60 (dd, *J* = 9.0, 0.7 Hz, 1H), 7.40 (d, *J* = 16.3 Hz, 1H), 7.26 (ddd, *J* = 7.4, 4.8, 1.1 Hz, 1H), 7.18 - 7.05 (m, 3H), 6.98 (qd, *J* = 6.3, 3.2 Hz, 2H), 5.79 (dd, *J* = 9.7, 2.5 Hz, 1H), 4.68 (s, 2H), 3.88 (d, *J* = 11.3 Hz, 1H), 3.79 - 3.65 (m, 1H), 3.10 (s, 3H), 2.40 (tdd, J= 12.9, 9.8, 3.9 Hz, 1H), 2.09 - 1.88 (m, 2H), 1.81 - 1.64 (m, 1H), 1.58 (tq, J= 7.4, 3.8 Hz, 2H). LCMS (ESI), m/z: 461.2 [M+H]$^+$

Step 2: Synthesis of (E)-N-(3,5-difluorobenzyl)-N-methyl-3-(2-(pyridin-2-yl)vinyl)-1H-indazol-5-amine (Compound 67)

The synthesis of compound 67 from compound 66 was performed the same as step 7 in Example 1 (yield: 76.2%).
$^1$H NMR (400 MHz, DMSO-$d_6$) δ 13.04 (s, 1H), 8.62 - 8.48 (m, 1H), 7.93 (d, *J*= 16.3 Hz, 1H), 7.80 (td, *J*= 7.7, 1.9 Hz, 1H), 7.63 (d, *J*= 7.9 Hz, 1H), 7.48 - 7.32 (m, 2H), 7.25 (ddd, *J* = 7.5, 4.8, 1.1 Hz, 1H), 7.18 (d, *J* = 2.2 Hz, 1H), 7.10 (ddd, *J* = 12.0, 8.5, 2.3 Hz, 2H), 7.00 (qd, *J*= 6.4, 3.1 Hz, 2H), 4.66 (s, 2H), 3.09 (s, 3H).
LCMS (ESI), m/z: 377.2 [M+H]$^+$

Example 31: Preparation of (E)-N-(3,5-difluorophenyl)-3-(2-(pyridin-2-yl)vinyl)-1H-indazole-5-carboxamide (designated as DYX-171)

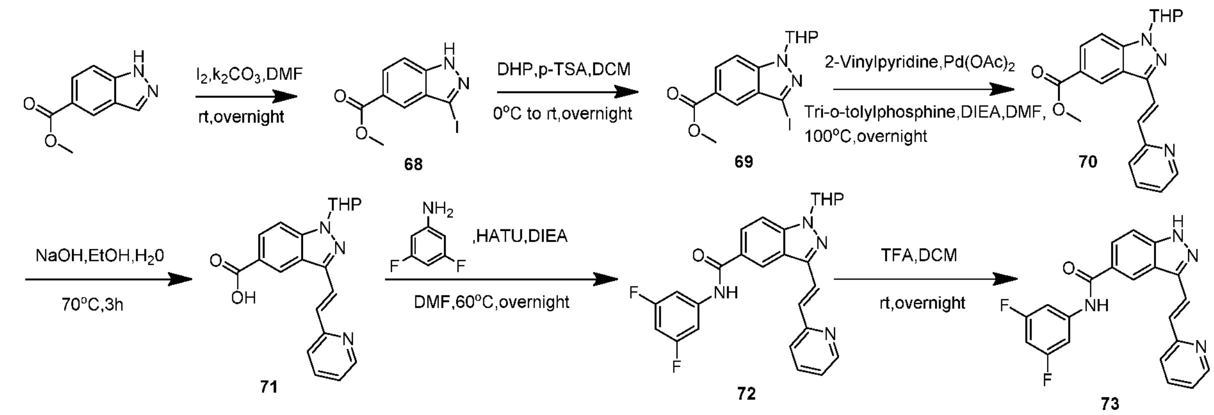


The synthesis methods in steps 1-3 were the same as those in the step 4-6 in Example 1.

Step 4: Synthesis of (E)-3-(2-(pyridin-2-yl)vinyl)-1-(tetrahydro-2H-pyran-2-yl)-1H-indazole-5-carboxylic acid (Compound 71)

Compound 70 (2g, 5.5 mmol) and NaOH (440 mg, 11 mol) were dissolved in a mixed solvent of 15 mL of ethanol and 5mL of water, and the reaction was carried out at 70 °C for 3 hours. After the reaction was completed, the ethanol in the solvent was removed by evaporation. The pH was adjusted to be acidic with 1M of hydrochloric acid while solid was precipitated. Suction filtration, water washing and drying were performed to give 1.57 g of Compound 71 (yield: 81.8%).

$^1$H NMR (400 MHz, DMSO-$d_6$) $\delta$ 13.02 (s, 1H), 8.74 (s, 1H), 8.62 (d, *J* = 4.7 Hz, 1H), 8.06 - 7.95 (m, 2H), 7.89 - 7.78 (m, 3H), 7.60 (d, *J*= 16.4 Hz, 1H), 7.31 (dt, *J* = 8.2, 3.7 Hz, 1H), 5.99 - 5.90 (m, 1H), 3.92 (d, *J* = 11.6 Hz, 1H), 3.78 (q, *J* = 11.7, 8.9 Hz, 1H), 2.48 - 2.33 (m, 1H), 2.14 - 1.95 (m, 2H), 1.78 (tt, *J* = 16.0, 6.2 Hz, 1H), 1.61 (tt, *J*= 8.1, 4.0 Hz, 2H). LCMS (ESI), m/z: 350.1 $[M+H]^+$

Step 5: (E)-N-(3,5-difluorophenyl)-3-(2-(pyridin-2-yl)vinyl)-1-(tetrahydro-2H-pyran-2-yl)-1H-i ndazole-5-amide

Compound 71 (1 g, 2.9 mmol), HATU (1.64 g, 4.3 mmol), and DIEA (1.12 g, 8.7 mmol) were dissolved in 10 mL of DMF, the mixture was stirred at room temperature for 10 min; and then 3,5-difluoroaniline (561 mg, 4.3 mmol) was added, and the obtained mixture was heated to 60 °C for reaction overnight. After the reaction was completed, an appropriate amount of water was added, followed by extraction with ethyl acetate three times. The organic layer was washed four times with water and once with saturated brine, dried over anhydrous sodium sulfate, filtered by suction and evaporated to dryness, and column chromatography was performed to give 776 mg of Compound 72 (yield: 58.9%). $^1$H NMR (400 MHz, DMSO-$d_6$) $\delta$ 10.71 (s, 1H), 8.82 (s, 1H), 8.64 (dd, *J* = 4.9, 1.7 Hz, 1H), 8.12 - 7.96 (m, 2H), 7.91 (d, *J* = 8.9 Hz, 1H), 7.85 (td, *J* = 7.6, 1.9 Hz, 1H), 7.77 - 7.66 (m, 2H), 7.66 - 7.56 (m, 2H), 7.32 (dd, *J* = 7.4, 4.8 Hz, 1H), 6.98 (tt, *J* = 9.3, 2.5 Hz, 1H), 5.97 (dd, *J* = 9.5, 2.3 Hz, 1H), 3.99 - 3.87 (m, 1H), 3.88 - 3.71 (m, 1H), 2.48 - 2.40 (m, 1H), 2.15 - 2.00 (m, 2H), 1.78 (td, *J* = 14.8, 12.6, 7.2 Hz, 1H), 1.62 (qd, *J* = 8.2, 5.1, 4.0 Hz, 2H). LCMS (ESI), m/z: 460.2 $[M+H]^+$

Step 6: Preparation of (E)-N-(3,5-difluorophenyl)-3-(2-(pyridin-2-yl)vinyl)-1H-indazole-5-amide (Compound 73)

The synthetic method of Compound 73 was the same as step 7 in Example 1 (yield: 66.4%).

$^1$H NMR (400 MHz, DMSO-$d_6$) $\delta$ 13.62 (s, 1H), 10.71 (s, 1H), 8.83 (s, 1H), 8.63 (dd, *J* = 4.8, 1.7 Hz, 1H), 8.10 - 7.94 (m, 2H), 7.85 (td, *J* = 7.7, 1.9 Hz, 1H), 7.74 - 7.66 (m, 3H), 7.65 - 7.56 (m, 2H), 7.31 (dd, *J* = 7.5, 4.8 Hz, 1H), 6.99 (tt, *J* = 9.3, 2.4 Hz, 1H).

LCMS (ESI), m/z: 377 $[M+H]^+$

Example 32: Preparation of (E)-5-((3,5-difluorophenyl)ethynyl)-3-(2-(pyridin-2-yl)vinyl)-1H-indazole (designated as DYX-183)

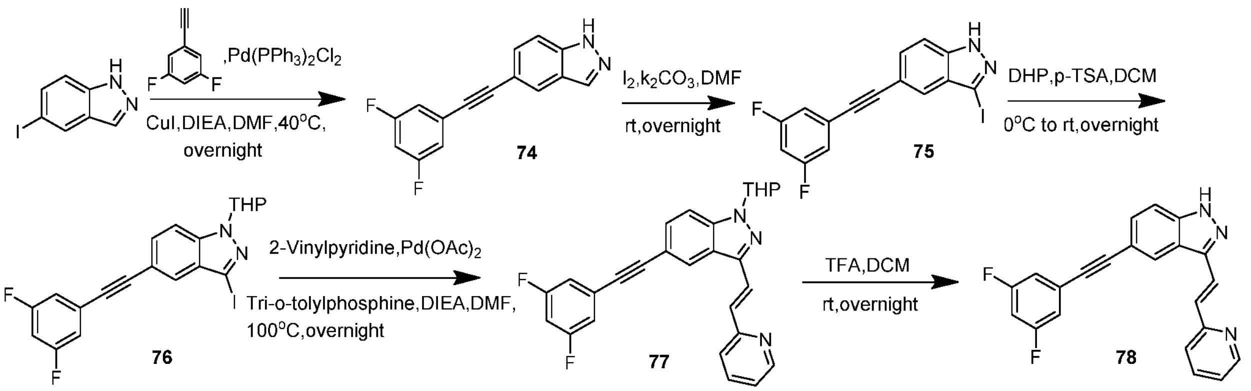


Step 1: Synthesis of 5-((3,5-difluorophenyl)ethynyl)-1H-indazole (Compound 74)

5-Iodo-1H-indazole (1 g, 4.1 mmol), bis(triphenylphosphine)palladium dichloride (288 mg, 0.41 mmol), and cuprous iodide (78 mg, 0.41 mmol) were added to a two-necked flask with argon replacement for three times. Then 1-ethynyl-3,5-difluorobenzene (1.14g, 8.2mmol), DIEA (1.59g, 12.3mmol) and 15mL DMF were sequentially added with a syringe, and the reaction was carried out overnight at 40 °C. After the reaction was completed, water was added thereto, followed by three times extraction with ethyl acetate . The organic layer was washed four times with water and once with saturated brine, dried over anhydrous sodium sulfate, filtered by suction and evaporated to dryness, and column chromatography was performed to give 822 mg of Compound 74 (yield: 78.9%). $^1$H NMR (400 MHz, DMSO-$d_6$) δ 13.35 (s, 1H), 8.15 (d, *J* = 1.3 Hz, 1H), 8.09 - 8.03 (m, 1H), 7.61 (dt, *J* = 8.6, 1.0 Hz, 1H), 7.50 (dd, *J* = 8.6, 1.5 Hz, 1H), 7.35 (td, J= 7.4, 2.9 Hz, 3H). LCMS (ESI), m/z: 255 [M+H]$^+$

The remaining synthesis procedures were the same as those of Compounds 4 to 7 in Example 1 (yield: 79.2%).

$^1$H NMR (400 MHz, DMSO-$d_6$) δ 13.54 (s, 1H), 8.61 (dd, *J*= 5.0, 1.8 Hz, 1H), 8.50 (s, 1H), 7.98 (d, *J* = 16.4 Hz, 1H), 7.82 (td, *J* = 7.6, 1.8 Hz, 1H), 7.76 - 7.70 (m, 1H), 7.67 - 7.54 (m, 3H), 7.40 - 7.31 (m, 3H), 7.29 (ddd, J= 7.4, 4.7, 1.2 Hz, 1H).

LCMS (ESI), m/z: 358.1 [M+H]$^+$

Example 33: Preparation of (E)-5-(3,5-difluorophenethyl)-3-(2-(pyridin-2-yl)vinyl)-1H-indazole (designated as DYX-180)

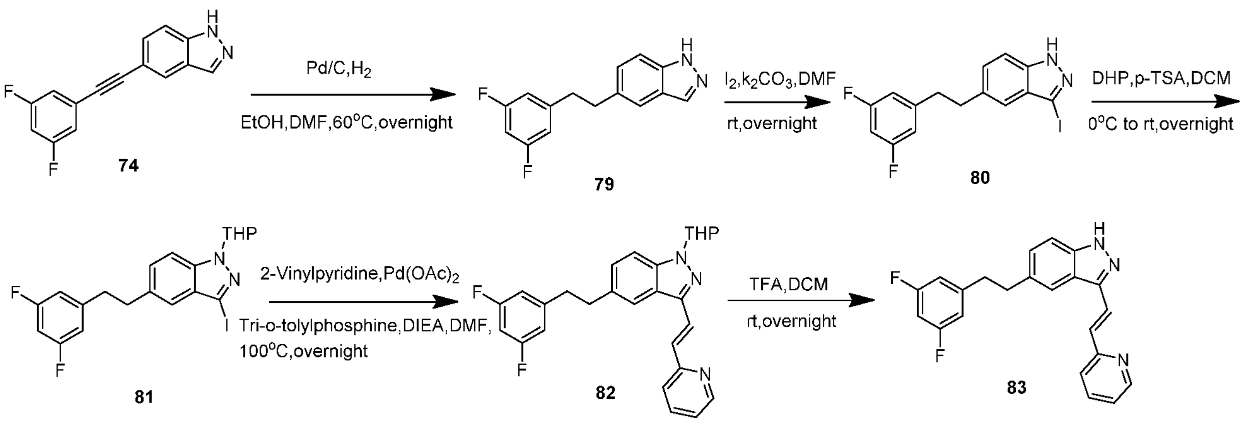


Step 1: Synthesis of 5-(3,5-difluorophenethyl)-1H-indazole (Compound 79)

Compound 74 (500 mg, 1.97 mmol) was dissolved in a mixed solvent of 5 mL of ethanol and 5 mL of DMF, then Pd/C was added. $H_2$ replacement was performed for five times, and a reaction was carried out overnight at 60°C. After the reaction was completed, suction filtration was carried out, and the obtained filter residue was washed with ethanol for three times. The obtained filtrate was removed by evaporation to give 468 mg of compound 79 (yield: 92.1%). $^1$H NMR (400 MHz, DMSO-$d_6$) δ 12.98 (s, 1H), 7.98 (s, 1H), 7.54 (s, 1H), 7.45 (d, *J* = 8.5 Hz, 1H), 7.24 (dd, *J* = 8.5, 1.6 Hz, 1H), 7.01 (dp, *J* = 12.1, 5.5, 4.0 Hz, 3H), 3.03 - 2.88 (m, 4H). LCMS (ESI), m/z: 334.1 [M+H]$^+$

The remaining synthesis procedures were the same as those of compound 4-7 in Example 1 (yield: 81.5%).
$^1H$ NMR (400 MHz, DMSO-$d_6$) δ 13.22 (s, 1H), 8.60 (dt, *J* = 4.8, 1.3 Hz, 1H), 8.03 - 7.91 (m, 2H), 7.82 (td, J= 7.7, 1.9 Hz, 1H), 7.67 (dt, *J*= 7.9, 1.1 Hz, 1H), 7.57 - 7.45 (m, 2H), 7.35 - 7.24 (m, 2H), 7.10 - 6.98 (m, 3H), 3.14 - 2.88 (m, 4H). LCMS (ESI), m/z: 362.1 $[M+H]^+$

Example 34: Preparation of (E)-5-(3,5-difluorophenyl)-3-(2-(pyridin-2-yl)vinyl)-1H-indazole (designated as DYX-119)

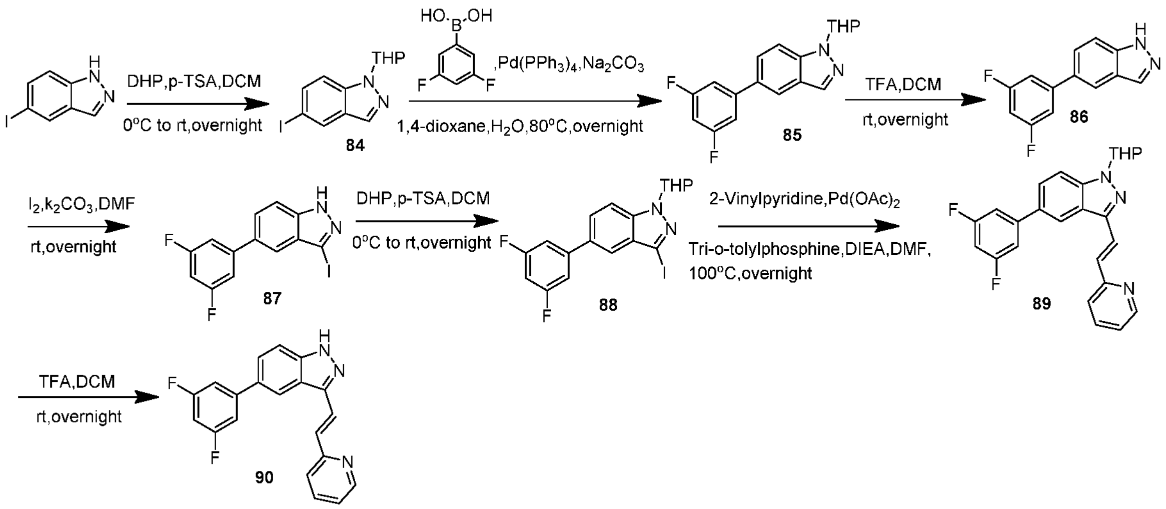


Step 1: Synthesis of 5-iodo-1-(tetrahydro-2H-pyran-2-yl)-1H-indazole (Compound 84)

Compound 84 was synthesized the same as step 1 in Example 1 (yield: 82.2%).

Step 2: Synthesis of 5-(3,5-difluorophenyl)-1-(tetrahydro-2H-pyran-2-yl)-1H-indazole (Compound 85)

Compound 84 (2 g, 6.1 mmol), 3,5-difluorophenylboronic acid (1.44 g, 9.1 mmol), and sodium carbonate (1.29 g, 12.2 mmol) were added to a mixed solvent of 30 mL of 1,4-dioxane and 6 mL of water with argon replacement for three times. Tetrakis(triphenylphosphine)palladium (693 mg, 0.6 mmol) was added. Argon replacement was performed for three times, and a reaction was carried out at 80°C overnight. After the reaction was completed, 1,4-dioxane was removed by evaporation, and ethyl acetate and water were added for extraction. The organic layer was washed with saturated brine, dried over anhydrous sodium sulfate, filtered by suction, and evaporated to dryness. Column chromatography was performed to give 1.17 g of compound 85 (yield: 61.1%). $^1H$ NMR (400 MHz, DMSO-$d_6$) δ 8.20 - 8.14 (m, 2H), 7.85 - 7.78 (m, 2H), 7.53 - 7.45 (m, 2H), 7.20 (tt, *J* = 9.3, 2.3 Hz, 1H), 5.90 (dd, *J*= 9.7, 2.5 Hz, 1H), 3.89 (dtd, *J*= 11.2, 3.8, 1.6 Hz, 1H), 3.76 (ddd, *J*= 11.4, 8.0, 5.8 Hz, 1H), 2.47 - 2.35 (m, 1H), 2.07 - 1.95 (m, 2H), 1.83 - 1.69 (m, 1H), 1.59 (tq, J= 8.2, 3.8 Hz, 2H).
LCMS (ESI), m/z: 315.1 $[M+H]^+$
The remaining steps were the same as steps 3-7 in Example 1 (yield: 70.6%).
$^1H$ NMR (400 MHz, DMSO-$d_6$) δ 13.43 (s, 1H), 8.61 (dd, J= 4.8, 1.7 Hz, 1H), 8.57 - 8.48 (m, 1H), 8.05 (d, *J*= 16.3 Hz, 1H), 7.88 - 7.76 (m, 2H), 7.76 - 7.55 (m, 5H), 7.34 - 7.16 (m, 2H).
LCMS (ESI), m/z: 334.1 $[M+H]^+$

Example 35: Preparation of (E)-5-((3,5-difluorophenyl)sulfonyl)-3-(2-(pyridin-2-yl)vinyl)-1H-pyrazolo[3,4-c]pyridi ne (designated as DYX-149)

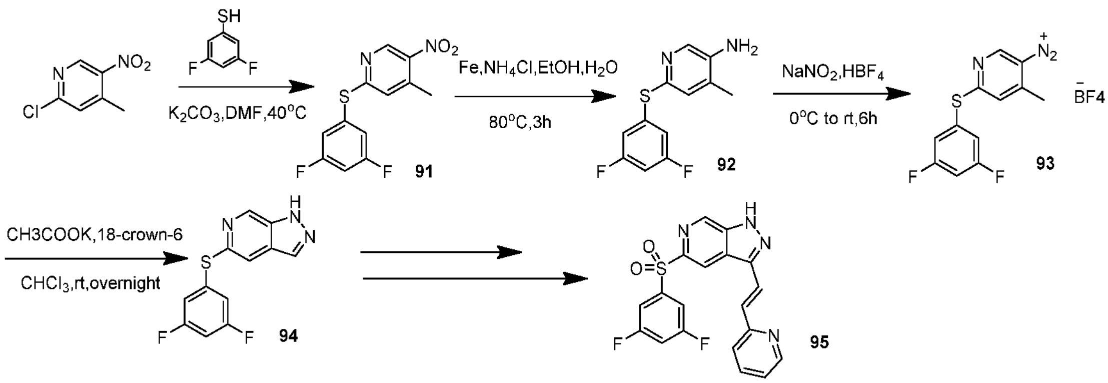


91 92 93 94 95

The synthesis method in Example 35 was referred to those in Example 17 and 19 (yield: 62.6%).

$^1H$ NMR (400 MHz, DMSO-$d_6$) $\delta$ 14.46 (s, 1H), 9.17 (s, 1H), 9.08 (s, 1H), 8.64 (dd, *J* = 4.8, 1.6 Hz, 1H), 8.08 (d, *J* = 16.4 Hz, 1H), 7.89 - 7.65 (m, 6H), 7.38 - 7.30 (m, 1H).

LCMS (ESI), m/z: 399.0 $[M+H]^+$

Example 36: Preparation of (E)-5-((3,5-difluorophenyl)sulfonyl)-3-(2-(pyridin-2-yl)vinyl)-1H-pyrazolo[4,3-b]pyridi ne (designated as DYX-150)

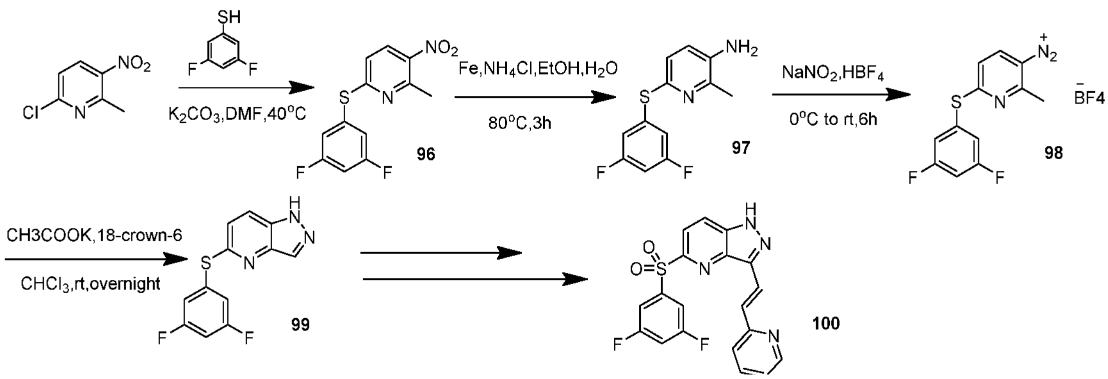


96 97 98 99 100

The synthesis method in Example 36 was referred to those in Example 17 and 19 (yield: 75.1%).

$^1H$ NMR (400 MHz, DMSO-$d_6$) $\delta$ 14.02 (s, 1H), 8.65 (dd, *J* = 4.9, 1.7 Hz, 1H), 8.38 (d, *J* = 8.8 Hz, 1H), 8.24 (d, *J* = 8.8 Hz, 1H), 7.99 - 7.72 (m, 6H), 7.59 (d, *J* = 7.8 Hz, 1H), 7.33 (ddd, *J* = 7.6, 4.7, 1.1 Hz, 1H).

LCMS (ESI), m/z: 399.0 $[M+H]^+$

Example 37: Preparation of 5-benzyl-3-(pyridin-2-ylethynyl)-1H-indazole (designated as DYX-014)

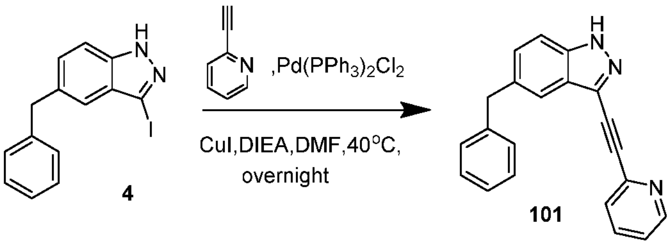


4 101

Compound 4 (200 mg, 0.6 mmol), bis(triphenylphosphine)palladium dichloride (21 mg, 0.03 mmol), and cuprous iodide (12 mg, 0.06 mmol) were added to a two-necked flask. Argon replacement was performed for three times. 2-ethynylpyridine (123 mg, 1.2 mmol), DIEA (232 mg, 1.8 mmol) and 10 mL of DMF were added sequentially with a syringe,

and a reaction was carried out overnight at 40°C. After the reaction was completed, an appropriate amount of water was added, followed by extraction with ethyl acetate for three times. The organic layer was washed with water for four times, washed once with saturated brine, dried over anhydrous sodium sulfate, filtered by suction, and evaporated to dryness. Column chromatography was performed to give 101 mg of compound 6 (yield 54.6%).

$^1$H NMR (400 MHz, DMSO-$d_6$) δ 13.60 (s, 1H), 8.66 (ddd, *J* = 4.9, 1.8, 1.0 Hz, 1H), 7.90 (td, *J* = 7.7, 1.8 Hz, 1H), 7.77 (dt, *J* = 7.8, 1.1 Hz, 1H), 7.73 - 7.69 (m, 1H), 7.56 (dd, *J* = 8.6, 0.8 Hz, 1H), 7.46 (ddd, *J* = 7.7, 4.9, 1.2 Hz, 1H), 7.33 (dd, *J* = 8.7, 1.6 Hz, 1H), 7.29 (d, *J* = 4.9 Hz, 4H), 7.23 - 7.14 (m, 1H), 4.11 (s, 2H).

LCMS (ESI), m/z: 310.1 $[M+H]^+$

Example 38: Preparation of 5-(2-fluorobenzyl)-3-(pyridin-2-ylethynyl)-1H-indazole (designated as DYX-021)

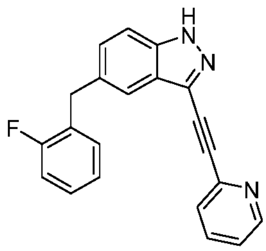

The synthesis was performed as same as Example 37 (yield: 54.5%).

$^1$H NMR (400 MHz, DMSO-$d_6$) δ 13.61 (s, 1H), 8.65 (ddd, *J*= 4.9, 1.8, 1.0 Hz, 1H), 7.90 (td, *J* = 7.7, 1.8 Hz, 1H), 7.75 (dt, *J* = 7.8, 1.1 Hz, 1H), 7.68 (s, 1H), 7.60 - 7.55 (m, 1H), 7.46 (ddd, *J* = 7.6, 4.9, 1.2 Hz, 1H), 7.38 - 7.23 (m, 3H), 7.20 - 7.11 (m, 2H), 4.13 (s, 2H).

LCMS (ESI), m/z: 328 $[M+H]^+$

Example 39: Preparation of 5-(3-fluorobenzyl)-3-(pyridin-2-ylethynyl)-1H-indazole (designated as DYX-025)

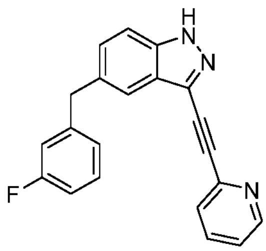

The synthesis was performed the same as Example 37 (yield: 47.6%).

$^1$H NMR (400 MHz, DMSO-$d_6$) δ 13.61 (s, 1H), 8.65 (ddd, *J* = 4.9, 1.8, 1.0 Hz, 1H), 7.90 (td, *J* = 7.7, 1.8 Hz, 1H), 7.80 - 7.72 (m, 2H), 7.57 (dd, *J* = 8.5, 0.8 Hz, 1H), 7.45 (ddd, *J* = 7.6, 4.8, 1.2 Hz, 1H), 7.38 - 7.27 (m, 2H), 7.18 - 7.07 (m, 2H), 7.06 - 6.95 (m, 1H), 4.12 (s, 2H).

LCMS (ESI), m/z: 328 $[M+H]^+$

Example 40: Preparation of 5-(4-fluorobenzyl)-3-(pyridin-2-ylethynyl)-1H-indazole (designated as DYX-027)

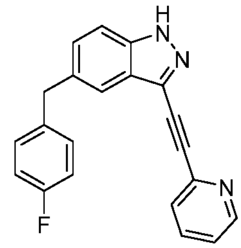

The synthesis was performed the same as Example 37 (yield: 50.7%).

$^1$H NMR (400 MHz, DMSO-$d_6$) δ 13.59 (s, 1H), 8.65 (ddd, *J* = 4.9, 1.8, 0.9 Hz, 1H), 7.90 (td, *J* = 7.7, 1.8 Hz, 1H), 7.78 - 7.69 (m, 2H), 7.56 (dd, *J* = 8.6, 0.8 Hz, 1H), 7.46 (ddd, *J* = 7.6, 4.9, 1.2 Hz, 1H), 7.38 - 7.26 (m, 3H), 7.15 - 7.02 (m, 2H), 4.09 (s, 2H).

LCMS (ESI), m/z: 328[M+H]$^+$

Example 41: Preparation of 5-(2,5-difluorobenzyl)-3-(pyridin-2-ylethynyl)-1H-indazole (designated DYX-041)

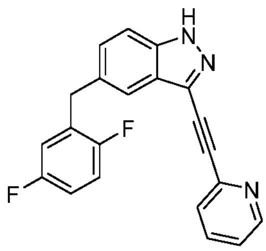

The synthesis was performed the same as Example 37 (yield: 53.1%).

$^1$H NMR (400 MHz, DMSO-$d_6$) δ 13.63 (s, 1H), 8.70 - 8.62 (m, 1H), 7.90 (td, *J* = 7.7, 1.9 Hz, 1H), 7.79 - 7.69 (m, 2H), 7.58 (d, *J* = 8.6 Hz, 1H), 7.46 (tt, *J* = 5.5, 2.7 Hz, 1H), 7.34 (dd, *J* = 8.6, 1.5 Hz, 1H), 7.23 (tdd, *J* = 9.3, 5.0, 3.3 Hz, 2H), 7.11 (td, *J* = 8.3, 4.4 Hz, 1H), 4.12 (s, 2H).

LCMS (ESI), m/z: 346.1 [M+H]$^+$

Example 42: Preparation of 5-(2,3-difluorobenzyl)-3-(pyridin-2-ylethynyl)-1H-indazole (designated as DYX-074)

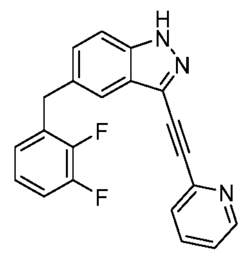

The synthesis was performed the same as Example 37 (yield: 54.5%).

$^1$H NMR (400 MHz, DMSO-$d_6$) δ 13.63 (s, 1H), 8.66 (dt, *J* = 4.7, 1.5 Hz, 1H), 7.90 (td, *J* = 7.8, 1.9 Hz, 1H), 7.78 - 7.69 (m, 2H), 7.59 (d, *J*= 8.6 Hz, 1H), 7.46 (ddd, *J* = 7.7, 4.8, 1.2 Hz, 1H), 7.37 - 7.21 (m, 2H), 7.21 - 7.10 (m, 2H), 4.19 (s, 2H).

LCMS (ESI), m/z: 346.1 [M+H]$^+$

Example 43: Preparation of 5-(3,5-difluorobenzyl)-3-(pyridin-2-ylethynyl)-1H-indazole (designated as CCB-484)

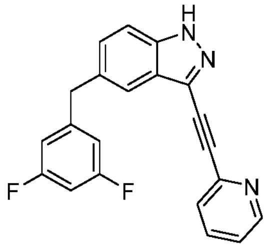

The synthesis was performed the same as Example 37 (yield: 52.2%).

$^1$H NMR (400 MHz, DMSO-$d_6$) $\delta$ 13.60 (s, 1H), 8.66 (ddd, *J* = 4.8, 1.7, 0.9 Hz, 1H), 7.90 (td, *J* = 7.7, 1.8 Hz, 1H), 7.80 - 7.73 (m, 2H), 7.61 - 7.54 (m, 1H), 7.46 (ddd, *J* = 7.6, 4.9, 1.2 Hz, 1H), 7.36 (dd, *J*= 8.6, 1.5 Hz, 1H), 7.08 - 6.98 (m, 3H).

LCMS (ESI), m/z: 346 $[M+H]^+$

Example 44: Preparation of 5-(3,4-difluorobenzyl)-3-(pyridin-2-ylethynyl)-1H-indazole (designated as DYX-044)

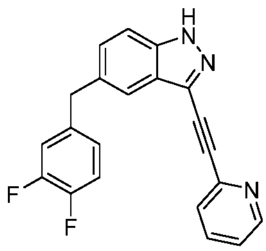

The synthesis was performed the same as Example 37 (yield: 49.6%).

$^1$H NMR (400 MHz, DMSO-$d_6$) $\delta$ 13.60 (s, 1H), 8.70 - 8.60 (m, 1H), 7.89 (td, *J* = 7.7, 1.8 Hz, 1H), 7.80 - 7.70 (m, 2H), 7.57 (d, *J* = 8.6 Hz, 1H), 7.45 (ddd, *J* = 7.6, 4.9, 1.3 Hz, 1H), 7.40 - 7.27 (m, 3H), 7.13 (ddd, *J* = 8.6, 4.4, 1.9 Hz, 1H), 4.09 (s, 2H).

LCMS (ESI), m/z: 346.1 $[M+H]^+$

Example 45: Preparation of 5-(2,4-difluorobenzyl)-3-(pyridin-2-ylethynyl)-1H-indazole (designated as DYX-051)

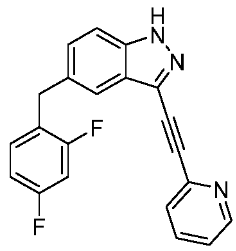

The synthesis was performed the same as Example 37 (yield: 50.6%).

$^1$H NMR (400 MHz, DMSO-$d_6$) $\delta$ 13.63 (s, 1H), 8.66 (d, *J* = 4.8 Hz, 1H), 7.90 (t, *J* = 7.8 Hz, 1H), 7.84 - 7.64 (m, 2H), 7.58 (d, *J* = 8.6 Hz, 1H), 7.52 - 7.14 (m, 4H), 7.05 (d, *J* = 8.7 Hz, 1H), 4.11 (s, 2H).

LCMS (ESI), m/z: 346 $[M+H]^+$

Example 46: Preparation of 5-(2,6-difluorobenzyl)-3-(pyridin-2-ylethynyl)-1H-indazole (designated as DYX-071)

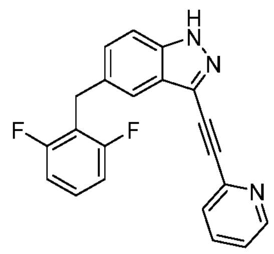

The synthesis was performed the same as Example 37 (yield: 62.6%).

$^1$H NMR (400 MHz, DMSO-$d_6$) δ 13.64 (s, 1H), 8.66 (dt, *J* = 4.6, 1.5 Hz, 1H), 7.90 (td, *J* = 7.7, 1.8 Hz, 1H), 7.78 - 7.71 (m, 1H), 7.63 (s, 1H), 7.58 (d, *J*= 8.6 Hz, 1H), 7.46 (ddd, *J* = 7.6, 4.9, 1.2 Hz, 1H), 7.40 - 7.27 (m, 2H), 7.13 (t, *J* = 7.8 Hz, 2H), 4.14 (s, 2H).

LCMS (ESI), m/z: 346 $[M+H]^+$

Example 47: Preparation of 3-(pyridin-2-ylethynyl)-5-(3,4,5-trifluorobenzyl)-1H-indazole (designated as DYX-075)

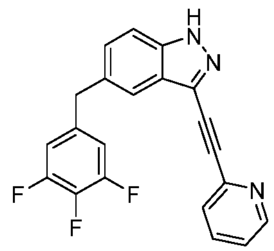

The synthesis was performed the same as Example 37 (yield: 53.1%).

$^1$H NMR (400 MHz, DMSO-$d_6$) δ 13.63 (s, 1H), 8.66 (d, *J* = 4.6 Hz, 1H), 7.91 (td, *J* = 7.7, 1.8 Hz, 1H), 7.81 - 7.73 (m, 2H), 7.58 (dd, *J* = 8.6, 0.8 Hz, 1H), 7.46 (ddd, *J* = 7.6, 4.9, 1.2 Hz, 1H), 7.36 (dd, *J* = 8.6, 1.6 Hz, 1H), 7.33 - 7.24 (m, 2H), 4.09 (s, 2H).

LCMS (ESI), m/z: 364.1 $[M+H]^+$

Example 48: Preparation of 5-(3,5-difluorophenoxy)-3-(pyridin-2-ylethynyl)-1H-indazole (designated as DYX-092)

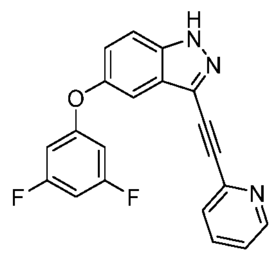

The synthesis was performed the same as Example 37 (yield: 47.7%).

$^1$H NMR (400 MHz, DMSO-$d_6$) δ 13.82 (s, 1H), 8.66 (s, 1H), 7.88 (t, *J* = 7.7 Hz, 1H), 7.77 (dd, *J* = 22.3, 8.4 Hz, 2H), 7.59 (d, *J* = 2.2 Hz, 1H), 7.50 - 7.40 (m, 1H), 7.29 (dd, *J* = 9.0, 2.1 Hz, 1H), 6.98 (tt, *J* = 9.3, 2.3 Hz, 1H), 6.77 - 6.66 (m, 2H).

LCMS (ESI), m/z: 348.1 $[M+H]^+$

Example 49: Preparation of 5-((3,5-difluorophenyl)thio)-3-(pyridin-2-ylethynyl)-1H-indazole (designated as DYX-086)

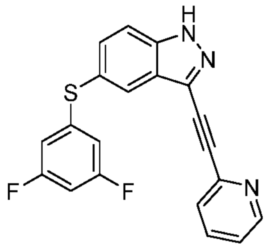

The synthesis was performed the same as Example 37 (yield: 40.4%).

$^1$H NMR (400 MHz, DMSO-$d_6$) δ 13.96 (s, 1H), 8.65 (d, *J* = 4.8 Hz, 1H), 8.14 (s, 1H), 7.94 - 7.73 (m, 3H), 7.60 - 7.52 (m, 1H), 7.46 (dd, *J* = 7.5, 4.9 Hz, 1H), 7.06 (tt, *J* = 9.2, 2.4 Hz, 1H), 6.85 - 6.74 (m, 2H).

LCMS (ESI), m/z: 364 $[M+H]^+$

Example 50: Preparation of 5-((3,5-difluorophenyl)sulfonyl)-3-(pyridin-2-ylethynyl)-1H-indazole (designated as DYX-108)

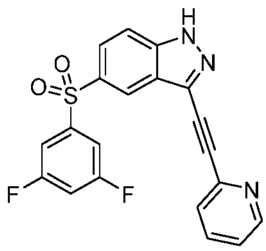

The synthesis was performed the same as Example 37 (yield: 44.2%).

$^1$H NMR (400 MHz, DMSO-$d_6$) δ 14.20 (s, 1H), 8.69 (d, *J* = 4.9 Hz, 1H), 8.60 (s, 1H), 8.02 (d, *J* = 8.9 Hz, 1H), 7.97 - 7.80 (m, 5H), 7.63 (q, *J* = 11.3, 10.1 Hz, 1H), 7.50 (dd, *J* = 7.6, 5.0 Hz, 1H).

LCMS (ESI), m/z: 396 $[M+H]^+$

Example 51: Preparation of N-(3,5-difluorophenyl)-3-(pyridin-2-ylethynyl)-1H-indazol-5-amine (designated as DYX-129)

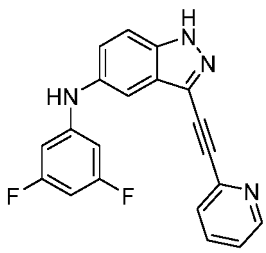

The synthesis was performed the same as Example 37 (yield: 43.7%).

$^1$H NMR (400 MHz, DMSO-$d_6$) δ 13.63 (s, 1H), 8.74 (s, 1H), 8.64 (d, *J* = 4.8 Hz, 1H), 7.88 (td, *J* = 7.8, 1.8 Hz, 1H), 7.69 (dd, *J* = 36.6, 8.3 Hz, 2H), 7.54 - 7.38 (m, 2H), 7.37 - 7.26 (m, 1H), 6.66 - 6.40 (m, 3H).

LCMS (ESI), m/z: 347.1 $[M+H]^+$

Example 52: Preparation of N-(3,5-difluorophenyl)-N-methyl-3-(pyridin-2-ylethynyl)-1H-indazol-5-amine (designated as DYX-130)

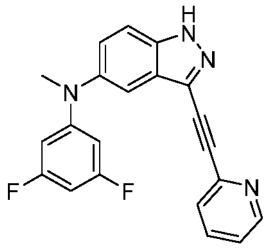

The synthesis was performed the same as Example 37 (yield: 45.8%).

$^1$H NMR (400 MHz, DMSO-$d_6$) δ 13.78 (s, 1H), 8.63 (ddd, *J* = 4.8, 1.8, 1.0 Hz, 1H), 7.88 (td, *J* = 7.7, 1.8 Hz, 1H), 7.78 (dt, *J* = 7.8, 1.2 Hz, 1H), 7.75 - 7.66 (m, 2H), 7.44 (ddd, *J* = 7.6, 4.9, 1.3 Hz, 1H), 7.32 (dd, *J* = 8.8, 2.0 Hz, 1H), 6.45 (tt, *J* = 9.3, 2.3 Hz, 1H), 6.32 - 6.19 (m, 2H), 3.32 (s, 3H).

LCMS (ESI), m/z: 361.1 $[M+H]^+$

Example 53: Preparation of 5-(3,5-difluorophenyl)-3-(pyridin-2-ylethynyl)-1H-indazole (designated as DYX-115)

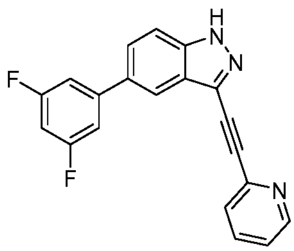

The synthesis was performed the same as Example 37 (yield: 42.9%).

$^1$H NMR (400 MHz, DMSO-$d_6$) δ 13.78 (s, 1H), 8.66 (d, *J* = 5.0 Hz, 1H), 8.16 (s, 1H), 7.95 - 7.68 (m, 4H), 7.60 - 7.41 (m, 3H), 7.21 (t, *J* = 9.3 Hz, 1H).

LCMS (ESI), m/z: 332 $[M+H]^+$

Example 54: Preparation of 5-((3,5-difluorobenzyl)oxo)-3-(pyridin-2-ylethynyl)-1H-indazole (designated as DYX-131)

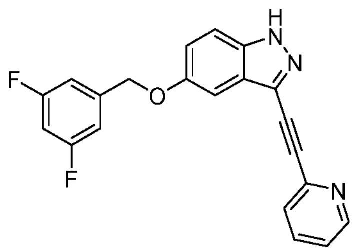

The synthesis was performed the same as Example 37 (yield: 50.6%).

$^1$H NMR (400 MHz, DMSO-$d_6$) δ 13.62 (s, 1H), 8.65 (dt, *J* = 4.8, 1.6 Hz, 1H), 7.90 (td, *J* = 7.7, 1.8 Hz, 1H), 7.77 (dt, *J* = 7.8, 1.2 Hz, 1H), 7.59 (d, *J* = 9.0 Hz, 1H), 7.45 (ddd, *J* = 7.7, 4.8, 1.2 Hz, 1H), 7.31 - 7.17 (m, 5H), 5.25 (s, 2H).

LCMS (ESI), m/z: 362.1 $[M+H]^+$

Example 55: Preparation of 5-(3-chlorobenzyl)-3-(pyridin-2-ylethynyl)-1H-indazole (designated as DYX-134Q)

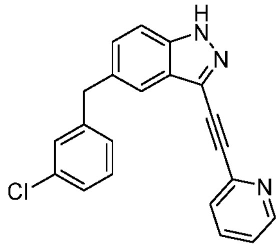

The synthesis was performed the same as Example 37 (yield: 40.3%).

$^1$H NMR (400 MHz, DMSO-$d_6$) δ 13.62 (s, 1H), 8.66 (dt, *J* = 4.9, 1.3 Hz, 1H), 7.90 (td, *J* = 7.7, 1.9 Hz, 1H), 7.76 (d, *J* = 7.7 Hz, 2H), 7.57 (d, *J* = 8.6 Hz, 1H), 7.46 (ddd, *J* = 7.6, 4.9, 1.2 Hz, 1H), 7.39 - 7.19 (m, 5H), 4.11 (s, 2H).

LCMS (ESI), m/z: 344 $[M+H]^+$

Example 56: Preparation of 3-(pyridin-2-ylethynyl)-5-(3-(trifluoromethylbenzyl)-1H-indazole (designated as DYX-138Q)

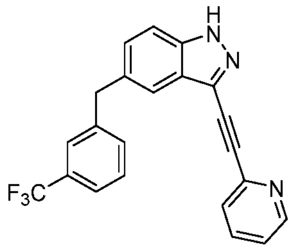

The synthesis was performed the same as Example 37 (yield: 45.8%).

$^1$H NMR (400 MHz, DMSO-$d_6$) δ 13.64 (s, 1H), 8.66 (d, *J* = 4.9 Hz, 1H), 7.96 - 7.85 (m, 1H), 7.83 - 7.72 (m, 2H), 7.71 - 7.43 (m, 6H), 7.36 (d, *J* = 8.6 Hz, 1H), 4.22 (s, 2H).

LCMS (ESI), m/z: 378.1 $[M+H]^+$

Example 57: Preparation of 5-(3-methoxybenzyl)-3-(pyridin-2-ylethynyl)-1H-indazole (designated as DYX-135Q)

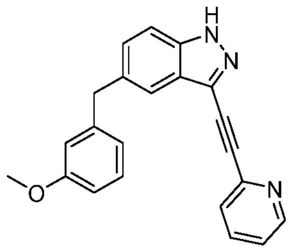

The synthesis was performed the same as Example 37 (yield: 47.3%).

$^1$H NMR (400 MHz, DMSO-$d_6$) δ 13.60 (s, 1H), 8.65 (ddd, *J* = 4.9, 1.8, 1.0 Hz, 1H), 7.90 (td, *J* = 7.7, 1.8 Hz, 1H), 7.76 (dt, *J* = 7.9, 1.2 Hz, 1H), 7.71 (s, 1H), 7.55 (d, *J* = 8.6 Hz, 1H), 7.45 (ddd, *J* = 7.7, 4.9, 1.2 Hz, 1H), 7.33 (dd, *J* = 8.6, 1.5 Hz, 1H), 7.19 (t, *J* = 7.8 Hz, 1H), 6.88 - 6.80 (m, 2H), 6.75 (ddd, *J* = 8.2, 2.6, 1.0 Hz, 1H), 4.06 (s, 2H), 3.70 (s, 3H).

LCMS (ESI), m/z: 339.1 $[M+H]^+$

Example 58: Preparation of 3-((3-(pyridin-2-ylethynyl)-1H-indazol-5-yl)methyl)benzonitrile (designated DYX-136Q)

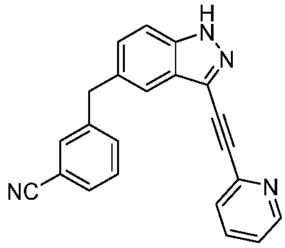

The synthesis was performed the same as Example 37 (yield: 44.5%).

$^1$H NMR (400 MHz, DMSO-$d_6$) δ 13.63 (s, 1H), 8.66 (d, *J* = 4.8 Hz, 1H), 7.90 (t, *J* = 7.7 Hz, 1H), 7.85 - 7.40 (m, 8H), 7.35 (d, *J* = 8.6 Hz, 1H), 4.17 (s, 2H).

LCMS (ESI), m/z: 335 $[M+H]^+$

Example 59: Preparation of 5-(3-methylbenzyl)-3-(pyridin-2-ylethynyl)-1H-indazole (designated as DYX-137Q)

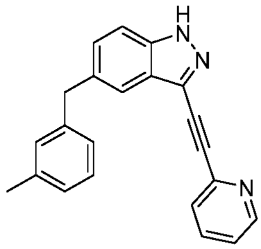

The synthesis was performed the same as Example 37 (yield: 43.2%).

$^1$H NMR (400 MHz, DMSO-$d_6$) δ 13.58 (s, 1H), 8.65 (d, *J* = 4.8 Hz, 1H), 7.89 (td, *J*= 7.7, 1.8 Hz, 1H), 7.75 (d, *J*= 7.8 Hz, 1H), 7.69 (s, 1H), 7.55 (d, *J* = 8.5 Hz, 1H), 7.45 (dd, *J* = 7.6, 4.9 Hz, 1H), 7.31 (d, *J* = 8.3 Hz, 1H), 7.16 (t, *J* = 7.4 Hz, 1H), 7.06 (d, *J*= 8.4 Hz, 2H), 6.98 (d, *J*= 7.5 Hz, 1H), 4.05 (s, 2H), 2.24 (s, 3H).

LCMS (ESI), m/z: 324 $[M+H]^+$

Example 60: Preparation of (E)-5-(3,5-difluorobenzyl)-3-(2-(pyridin-3-yl)vinyl)-1H-indazole (designated as DYX-03)

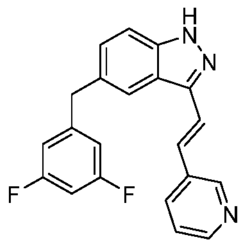

The synthesis was performed the same as Example 1 (yield: 67.6%).

$^1$H NMR (400 MHz, DMSO-$d_6$) δ 13.19 (s, 1H), 8.89 (d, *J* = 2.2 Hz, 1H), 8.48 (dd, *J* = 4.7, 1.6 Hz, 1H), 8.22 - 8.11 (m, 2H), 7.68 (d, *J* = 16.8 Hz, 1H), 7.58 - 7.39 (m, 3H), 7.29 (dd, *J* = 8.6, 1.5 Hz, 1H), 7.03 (td, *J* = 7.2, 6.8, 3.0 Hz, 3H), 4.12 (s, 2H).

LCMS (ESI), m/z: 348.1 $[M+H]^+$

Example 61: Preparation of (E)-5-(3,5-difluorobenzyl)-3-(2-(pyridin-4-yl)vinyl)-1H-indazole (designated as DYX-215)

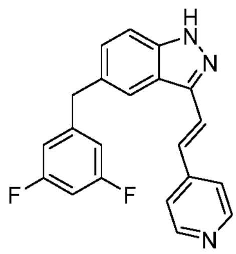

The synthesis was performed the same as Example 1 (yield: 65.9%).
$^1$H NMR (400 MHz, DMSO-$d_6$) δ 13.62 (s, 1H), 8.85 - 8.74 (m, 2H), 8.27-8.13 (m, 4H), 7.68 (d, *J* = 16.6 Hz, 1H), 7.57 (d, *J* = 8.6 Hz, 1H), 7.35 (dd, *J* = 8.6, 1.5 Hz, 1H), 7.09 - 7.00 (m, 3H), 4.15 (s, 2H).
LCMS (ESI), m/z: 348.1 [M+H]$^+$

Example 62: Preparation of (E)-5-(3,5-difluorobenzyl)-3-styryl-1H-indazole (designated as DYX-07)

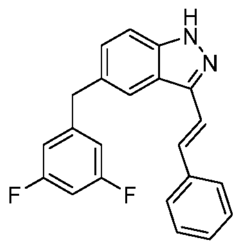

The synthesis was performed the same as Example 1 (yield: 70.2%).
$^1$H NMR (400 MHz, DMSO-$d_6$) δ 13.11 (s, 1H), 8.13 (s, 1H), 7.74 - 7.68 (m, 2H), 7.54 - 7.44 (m, 3H), 7.41 (t, *J* = 7.6 Hz, 2H), 7.32 - 7.25 (m, 2H), 7.08 - 6.99 (m, 3H), 4.12 (s, 2H).
LCMS (ESI), m/z: 347.1 [M+H]$^+$

Example 63: Preparation of (E)-5-(3,5-difluorobenzyl)-3-(2-fluorostyryl)-1H-indazole (designated as DYX-10)

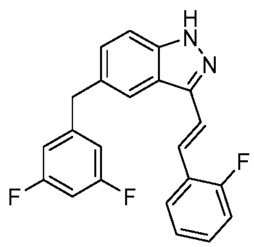

The synthesis was performed the same as Example 1 (yield: 71.1%).
$^1$H NMR (400 MHz, DMSO-$d_6$) δ 13.20 (s, 1H), 8.00 (s, 1H), 7.91 (td, *J* = 7.7, 1.8 Hz, 1H), 7.66 - 7.47 (m, 3H), 7.38 - 7.22 (m, 4H), 7.04 (dddd, *J* = 9.4, 5.8, 4.3, 2.4 Hz, 3H), 4.13 (s, 2H).
LCMS (ESI), m/z: 365.1 [M+H]$^+$

Example 64: Preparation of (E)-5-(3,5-difluorobenzyl)-3-(2-(pyrazin-2-yl)vinyl)-1H-indazole (designated as DYX-05)

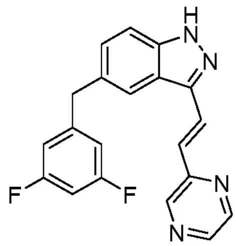

The synthesis was performed the same as Example 1 (yield: 73.1%).

$^1$H NMR (400 MHz, DMSO-$d_6$) δ 13.35 (s, 1H), 8.92 (d, *J* = 1.5 Hz, 1H), 8.65 (dd, *J* = 2.5, 1.5 Hz, 1H), 8.51 (d, *J* = 2.5 Hz, 1H), 8.17 (s, 1H), 8.06 (d, *J* = 16.4 Hz, 1H), 7.63 (d, *J* = 16.4 Hz, 1H), 7.52 (d, *J* = 8.5 Hz, 1H), 7.32 (dd, *J* = 8.6, 1.4 Hz, 1H), 7.11 - 6.97 (m, 3H), 4.13 (s, 2H).

LCMS (ESI), m/z: 349.1 $[M+H]^+$

Example 65: Preparation of (E)-2-(2-(5-(3,5-difluorobenzyl)-1H-indazol-3-yl)vinyl)quinoline (designated as DYX-06)

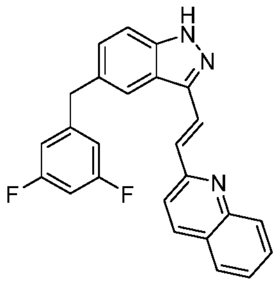

The synthesis was performed the same as Example 1 (yield: 54.7%).

$^1$H NMR (400 MHz, DMSO-$d_6$) δ 13.35 (s, 1H), 8.38 (d, *J* = 8.6 Hz, 1H), 8.21 (s, 1H), 8.13 (d, *J* = 16.6 Hz, 1H), 8.06 - 7.92 (m, 3H), 7.80 - 7.69 (m, 2H), 7.61 - 7.50 (m, 2H), 7.32 (dd, *J* = 8.6, 1.5 Hz, 1H), 7.11 - 6.99 (m, 3H), 4.16 (s, 2H).

LCMS (ESI), m/z: 398.1 $[M+H]^+$

Example 66: Preparation of (E)-5-(3,5-difluorobenzyl)-3-(2-(6-methylpyridin-2-yl)vinyl)-1H-indazole (designated as DYX-09)

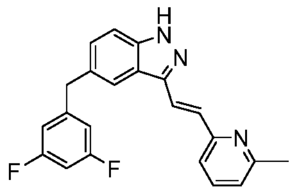

The synthesis was performed the same as Example 1 (yield: 77.8%).

$^1$H NMR (400 MHz, DMSO-$d_6$) δ 13.23 (s, 1H), 8.13 (s, 1H), 7.92 (d, *J* = 16.4 Hz, 1H), 7.70 (t, *J* = 7.7 Hz, 1H), 7.57 - 7.42 (m, 3H), 7.30 (dd, *J* = 8.6, 1.4 Hz, 1H), 7.14 (d, *J* = 7.6 Hz, 1H), 7.10 - 6.96 (m, 3H), 4.14 (s, 2H), 2.53 (s, 3H).

LCMS (ESI), m/z: 362.1 $[M+H]^+$

Example 67: Preparation of (E)-5-(3,5-difluorobenzyl)-3-(2-(thiophen-2-yl)vinyl)-1H-indazole (designated as DYX-04)

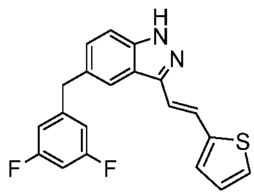

The synthesis was performed the same as Example 1 (yield: 49.9%).

$^1H$ NMR (400 MHz, DMSO-$d_6$) δ 13.12 (s, 1H), 8.10 (s, 1H), 7.68 (d, *J* = 16.5 Hz, 1H), 7.52 - 7.44 (m, 2H), 7.33 (d, *J*= 3.5 Hz, 1H), 7.27 (dd, *J*= 8.6, 1.4 Hz, 1H), 7.21 (d, *J* = 16.5 Hz, 1H), 7.10 (dd, *J* = 5.1, 3.5 Hz, 1H), 7.07 - 7.00 (m, 3H), 4.11 (s, 2H).

LCMS (ESI), m/z: 353.0 $[M+H]^+$

Example 68: Preparation of (E)-5-((3,5-difluorophenyl)sulfonyl)-3-(2-(pyridin-4-yl)vinyl)-1H-indazole (designated as E01F02)

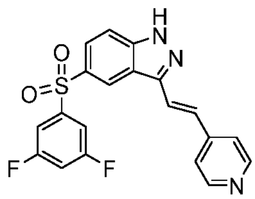

The synthesis was performed the same as Example 1 (yield: 70.2%).

$^1H$ NMR (400 MHz, DMSO-$d_6$) δ 8.97 (d, *J* = 1.6 Hz, 1H), 8.63 - 8.57 (m, 2H), 8.04 - 7.93 (m, 2H), 7.84 - 7.72 (m, 5H), 7.65 (tt, *J* = 9.2, 2.4 Hz, 1H), 7.56 (d, *J* = 16.5 Hz, 1H).

LCMS (ESI), m/z: 398.1 $[M+H]^+$

Example 69: Preparation of (E)-5-((3,5-difluorophenyl)sulfonyl)-3-styryl-1H-indazole (designated as E01F07)

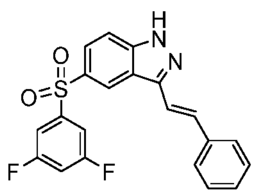

The synthesis was performed the same as Example 1 (yield: 73.6%).

$^1H$ NMR (400 MHz, DMSO-$d_6$) δ 13.70 (s, 1H), 8.93 (d, *J* = 1.6 Hz, 1H), 7.93 (dd, *J* = 8.9, 1.7 Hz, 1H), 7.85 - 7.69 (m, 6H), 7.65 (dd, *J* = 9.2, 2.5 Hz, 1H), 7.62 (s, 1H), 7.44 (t, *J* = 7.5 Hz, 2H), 7.33 (t, *J* = 7.3 Hz, 1H).

LCMS (ESI), m/z: 397.1 $[M+H]^+$

Example 70: Preparation of (E)-5-(3,5-difluorobenzyl)-3-(2-methylstyryl)-1H-indazole (designated as D01F08)

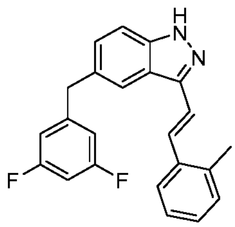

The synthesis was performed the same as Example 1 (yield: 73.1%).

$^1$H NMR (400 MHz, DMSO-$d_6$) δ 13.10 (s, 1H), 7.95 (s, 1H), 7.80 - 7.73 (m, 1H), 7.61 (d, *J* = 16.5 Hz, 1H), 7.49 (d, *J* = 8.5 Hz, 1H), 7.39 (d, *J* = 16.6 Hz, 1H), 7.31 (dd, *J* = 8.6, 1.4 Hz, 1H), 7.28 - 7.17 (m, 3H), 7.10 - 7.00 (m, 3H), 4.12 (s, 2H), 2.42 (s, 3H).

LCMS (ESI), m/z: 361.1 $[M+H]^+$

Example 71: Preparation of (E)-5-(3-chloro-5-fluorobenzyl)-3-(2-(pyridin-2-yl)vinyl)-1H-indazole (designated as D02F01)

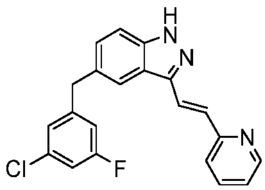

The synthesis was performed the same as Example 1 (yield: 65.8%).

$^1$H NMR (400 MHz, DMSO-$d_6$) δ 13.24 (s, 1H), 8.61 (dd, *J* = 5.0, 1.7 Hz, 1H), 8.14 (s, 1H), 7.95 (d, *J* = 16.4 Hz, 1H), 7.82 (td, *J* = 7.6, 1.8 Hz, 1H), 7.67 (d, *J* = 7.8 Hz, 1H), 7.60 - 7.48 (m, 2H), 7.34 - 7.16 (m, 5H), 4.13 (s, 2H).

LCMS (ESI), m/z: 364.1 $[M+H]^+$

Example 72: Preparation of (E)-5-(3-fluoro-5-methoxybenzyl)-3-(2-(pyridin-2-yl)vinyl)-1H-indazole (designated as D05F01)

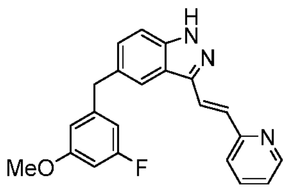

The synthesis was performed the same as Example 1 (yield: 60.9%).

LCMS (ESI),m/z: 360.1 $[M+H]^+$

Example 73: Preparation of (E)-5-(3,5-dichlorobenzyl)-3-(2-(pyridin-2-yl)vinyl)-1H-indazole (designated as D06F01)

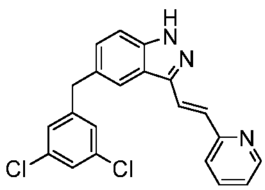

The synthesis was performed the same as Example 1 (yield: 72.0%).
$^1H$ NMR (400 MHz, DMSO-$d_6$) δ 13.25 (s, 1H), 8.62 - 8.58 (m, 1H), 8.15 (s, 1H), 7.95 (d, *J* = 16.4 Hz, 1H), 7.82 (td, *J* = 7.7, 1.9 Hz, 1H), 7.66 (d, *J* = 7.8 Hz, 1H), 7.60 - 7.48 (m, 2H), 7.40 (dd, *J* = 7.1, 1.9 Hz, 3H), 7.32 - 7.25 (m, 2H), 4.12 (s, 2H).
LCMS (ESI), m/z: 380.0 $[M+H]^+$

Example 74: Preparation of (E)-5-(3,5-dimethylbenzyl)-3-(2-(pyridin-2-yl)vinyl)-1H-indazole (designated as D07F01)

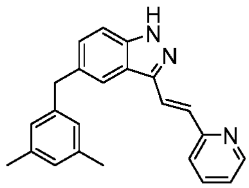

The synthesis was performed the same as Example 1 (yield: 66.3 %).
$^1H$ NMR (400 MHz, DMSO-$d_6$) δ 13.20 (s, 1H), 8.65 - 8.57 (m, 1H), 8.05 (s, 1H), 7.95 (d, *J* = 16.3 Hz, 1H), 7.81 (td, *J* = 7.7, 1.8 Hz, 1H), 7.68 (d, *J* = 7.8 Hz, 1H), 7.59 - 7.43 (m, 2H), 7.31 - 7.20 (m, 2H), 6.84 (d, *J*= 33.1 Hz, 3H), 4.02 (s, 2H), 2.21 (s, 6H).
LCMS (ESI), m/z: 340.2 $[M+H]^+$

Example 75: Preparation of (E)-5-(5-fluoro-2-methoxybenzyl)-3-(2-(pyridin-2-yl)vinyl)-1H-indazole (designated as D09F01)

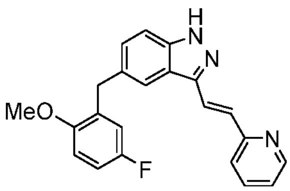

The synthesis was performed the same as Example 1 (yield: 67.4%).
LCMS (ESI), m/z: 360.1 $[M+H]^+$

Example 76: Preparation of (E)-N-(2,5-difluorobenzyl)-3-(2-(pyridin-2-yl)vinyl)-1H-indazol-5-amine (designated as DYX-216A)

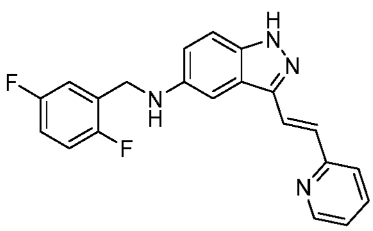

The synthesis was performed the same as Example 29 (yield: 74.8%).
$^1H$ NMR (400 MHz, DMSO-$d_6$) δ 12.94 (s, 1H), 8.63 - 8.53 (m, 1H), 7.93-7.75 (m, 2H), 7.56 (d, *J* = 7.9 Hz, 1H), 7.41 - 7.20 (m, 5H), 7.13 (tt, *J* = 8.0, 3.5 Hz, 1H), 7.05 - 6.89 (m, 2H), 6.21 (t, *J* = 6.4 Hz, 1H), 4.42 (d, *J* = 5.0 Hz, 2H).
LCMS (ESI), m/z: 363.1 $[M+H]^+$

Example 77: Preparation of (R,E)-5-(2-(2,5-difluorophenyl)pyrrolidin-1-yl)-3-(2-(pyridin-2-yl)vinyl)-1H-indazole (designated as DYX-170)

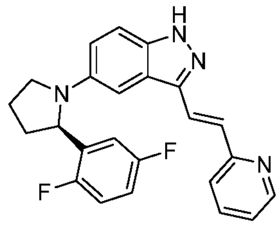

The synthesis was performed the same as Example 20 (yield: 66.2%).

$^1$H NMR (400 MHz, DMSO-$d_6$) δ 12.96 (s, 1H), 8.59 (ddd, *J* = 4.8, 1.8, 0.9 Hz, 1H), 7.89 (d, *J* = 16.3 Hz, 1H), 7.80 (td, *J* = 7.7, 1.9 Hz, 1H), 7.54 (d, *J* = 7.8 Hz, 1H), 7.41 - 7.29 (m, 2H), 7.28 - 7.19 (m, 2H), 7.17 - 7.08 (m, 1H), 6.97 - 6.88 (m, 2H), 6.72 (dd, *J* = 9.1, 2.1 Hz, 1H), 5.12 - 4.97 (m, 1H), 3.88 (td, *J* = 8.3, 7.6, 2.9 Hz, 1H), 3.44 (td, *J* = 8.8, 6.9 Hz, 1H), 2.49 - 2.41 (m, 1H), 2.11 - 1.98 (m, 2H), 1.95 - 1.85 (m, 1H).

LCMS (ESI), m/z: 403.1 [M+H]$^+$

Example 78: Preparation of (E)-3-(5-((3,5-difluorobenzyl)amino)-1H-indazol-3-yl)acrylamide (designated as DYX-223)

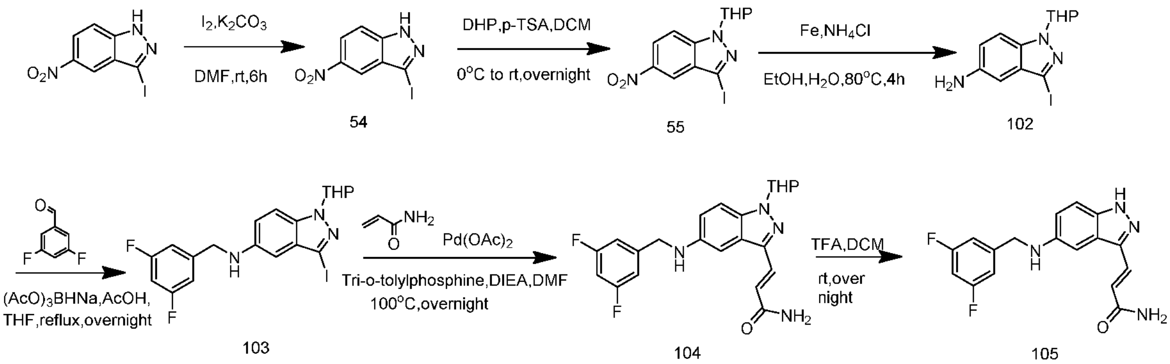


The specific steps of this synthetic route were the same as those in Example 26 and Example 29 (yield: 60.9%).

$^1$H NMR (400 MHz, DMSO-$d_6$) δ 13.07 (s, 1H), 7.63 - 7.50 (m, 2H), 7.35 (d, *J* = 8.9 Hz, 1H), 7.17 (qd, *J* = 6.2, 3.1 Hz, 2H), 7.07 (qd, *J* = 6.9, 6.1, 2.5 Hz, 2H), 6.93 (dd, *J* = 8.9, 2.1 Hz, 1H), 6.82 (s, 1H), 6.72 (d, *J* = 16.0 Hz, 1H), 6.33 (s, 1H), 4.39 (s, 2H).

LCMS (ESI), m/z: 329.1 [M+H]$^+$

Example 79: Preparation of (E)-3-(5-((3,5-difluorobenzyl)amino)-1H-indazol-3-yl)-N,N-dimethylacrylamide (designated as DYX-220)

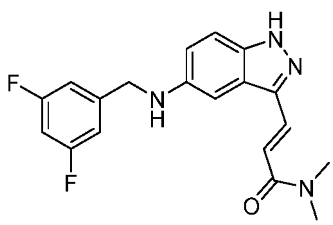

The synthesis was performed the same as Example 78 (yield: 56.7%).

$^1H$ NMR (400 MHz, DMSO-$d_6$) δ 13.12 (s, 1H), 7.63 (d, *J* = 15.6 Hz, 1H), 7.36 (d, *J* = 8.9 Hz, 1H), 7.12 (h, *J* = 4.0 Hz, 2H), 7.05 (tt, *J* = 9.3, 2.4 Hz, 1H), 6.98-6.89 (m, 2H), 6.69 (s, 1H), 6.46 (t, *J* = 6.1 Hz, 1H), 4.42 (d, *J* = 4.7 Hz, 2H), 3.09 (s, 3H), 2.95 (s, 3H).

LCMS (ESI), m/z: 357.1 $[M+H]^+$

Example 80: Preparation of (E)-3 -(5-((3,5-difluorobenzyl)amino)-1H-indazol-3-yl)-1-morpholinoprop-2-en-1-one (designated as DYX-222)

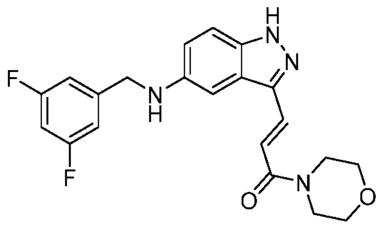

The synthesis was performed the same as Example 78 (yield: 63.4%).

$^1H$ NMR (400 MHz, DMSO-$d_6$) δ 13.16 (s, 1H), 7.71 (d, *J* = 15.6 Hz, 1H), 7.37 (d, *J* = 8.9 Hz, 1H), 7.16 - 7.03 (m, 3H), 7.00 - 6.92 (m, 2H), 6.73 (s, 1H), 6.45 (s, 1H), 4.43 (s, 2H), 3.71 - 3.53 (m, 8H).

LCMS (ESI), m/z: 399.2 $[M+H]^+$

Example 81: Preparation of (R,E)-3-(5-((3,5-difluorobenzyl)amino)-1H-indazol-3-yl)-1-(3-hydroxylpyrrolidin-1-yl) prop-2-en-1-one (designated as DYX-227)

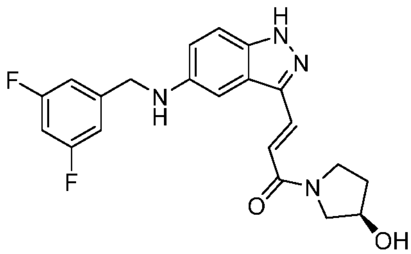

The synthesis was performed the same as Example 78 (yield: 64.4%).

$^1H$ NMR (400 MHz, DMSO-$d_6$) δ 13.13 (s, 1H), 7.63 (dd, *J* = 15.7, 3.2 Hz, 1H), 7.37 (d, *J* = 8.9 Hz, 1H), 7.17 - 7.02 (m, 3H), 6.96 (ddd, *J* = 9.0, 4.9, 2.0 Hz, 1H), 6.77 - 6.63 (m, 2H), 6.46 (dt, *J* = 17.2, 5.5 Hz, 1H), 5.00 (dd, *J* = 23.2, 3.5 Hz, 1H), 4.46 - 4.26 (m, 3H), 3.58 (dtt, *J* = 16.6, 8.4, 4.9 Hz, 2H), 3.48 - 3.38 (m, 2H), 2.08-1.76 (m, 2H).

LCMS (ESI), m/z: 399.1 $[M+H]^+$

Example 82: Preparation of (S,E)-3-(5-((3,5-difluorobenzyl)amino)-1H-indazol-3-yl)-1-(3-hydroxylpyrrolidin-1-yl) prop-2-en-1-one (designated as DYX-228)

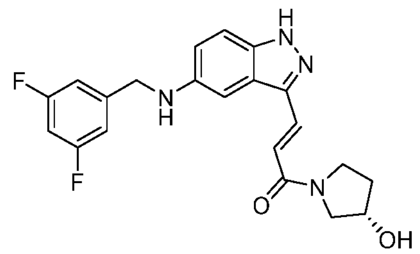

The synthesis was performed the same as Example 78 (yield: 61.7%).

$^1$H NMR (400 MHz, DMSO-$d_6$) δ 13.14 (s, 1H), 7.64 (dd, *J* = 15.7, 3.5 Hz, 1H), 7.37 (dd, *J* = 8.9, 1.9 Hz, 1H), 7.17 - 7.01 (m, 3H), 6.96 (ddd, *J* = 9.1, 4.9, 2.1 Hz, 1H), 6.77 - 6.63 (m, 2H), 6.46 (dt, *J* = 17.2, 6.1 Hz, 1H), 5.00 (dd, *J* = 23.0, 3.4 Hz, 1H), 4.48 - 4.25 (m, 3H), 3.58 (tdt, *J* = 15.4, 11.1, 6.4 Hz, 2H), 3.42 (d, *J* = 12.5 Hz, 2H), 2.07 - 1.76 (m, 2H).

LCMS (ESI), m/z: 399.1 [M+H]$^+$

Example 83: Preparation of (E)-3-(5-((3,5-difluorobenzyl)amino)-1H-indazol-3-yl)-1-(4-methylpiperazin-1-yl)prop-2-en-1-one (designated as DYX-226)

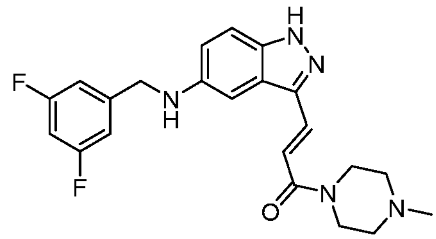

The synthesis was performed the same as Example 78 (yield: 59.8%).

$^1$H NMR (400 MHz, DMSO-$d_6$) δ 13.16 (s, 1H), 7.67 (d, *J* = 15.4 Hz, 1H), 7.36 (d, *J* = 8.8 Hz, 1H), 7.24 - 6.80 (m, 5H), 6.59 (d, *J* = 102.2 Hz, 2H), 4.43 (s, 2H), 3.59 (s, 4H), 2.31 (d, *J* = 53.1 Hz, 7H).

LCMS (ESI), m/z: 412.2 [M+H]$^+$

Example 84: Preparation of (E)-3-(5-((3,5-difluorobenzyl)amino)-1H-indazol-3-yl)-N-isopropylacrylamide (designated as DYX-231)

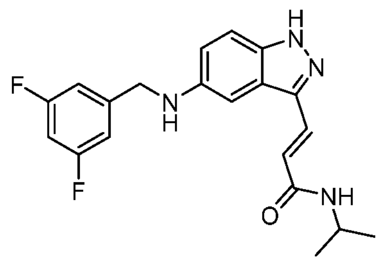

The synthesis was performed the same as Example 78 (yield: 63.9%).

$^1$H NMR (400 MHz, DMSO-$d_6$) δ 13.04 (s, 1H), 7.93 (d, *J* = 7.7 Hz, 1H), 7.55 (d, *J* = 15.9 Hz, 1H), 7.34 (d, *J* = 8.8 Hz, 1H), 7.19 (qd, *J* = 6.2, 3.1 Hz, 2H), 7.07 (tt, *J* = 9.3, 2.4 Hz, 1H), 6.93 (dd, *J* = 8.9, 2.0 Hz, 1H), 6.82 (s, 1H), 6.72 (d, *J* = 16.0 Hz, 1H), 6.31 (s, 1H), 4.39 (s, 2H), 4.07 - 3.90 (m, 1H), 1.14 (d, *J* = 6.6 Hz, 6H).

LCMS (ESI), m/z: 412.2 [M+H]$^+$

Example 85: Preparation of (E)-N-tert-butyl-3-(5-((3,5-difluorobenzyl)amino)-1H-indazol-3-yl)acrylamide (designated as DYX-230)

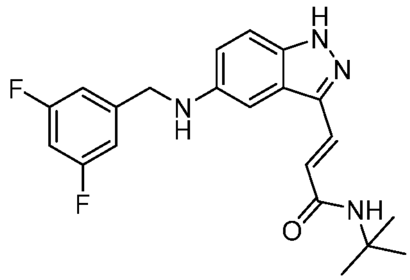

The synthesis was performed the same as Example 78 (yield: 67.6%).

$^1$H NMR (400 MHz, DMSO-$d_6$) δ 13.01 (s, 1H), 7.66 (s, 1H), 7.49 (d, *J* = 15.9 Hz, 1H), 7.33 (d, *J* = 8.8 Hz, 1H), 7.23 - 7.15 (m, 2H), 7.07 (tt, *J* = 9.3, 2.4 Hz, 1H), 6.92 (dd, *J* = 8.9, 2.0 Hz, 1H), 6.85 - 6.72 (m, 2H), 6.30 (t, *J* = 6.2 Hz, 1H), 4.39 (d, *J* = 6.0 Hz, 2H), 1.35 (s, 9H).

LCMS (ESI), m/z: 385.2 $[M+H]^+$

Example 86: Preparation of (E)-3-(5-((3,5-difluorobenzyl)amino)-1H-indazol-3-yl)-N-methylacrylamide (designated as DYX-235)

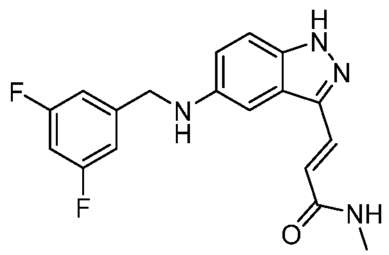

The synthesis was performed the same as Example 78 (yield: 69.0%).

$^1$H NMR (400 MHz, DMSO-$d_6$) δ 13.06 (s, 1H), 8.06 (q, *J* = 4.6 Hz, 1H), 7.56 (d, *J* = 15.9 Hz, 1H), 7.34 (d, *J* = 8.9 Hz, 1H), 7.17 (qd, *J* = 6.2, 3.1 Hz, 2H), 7.07 (tt, *J* = 9.3, 2.4 Hz, 1H), 6.94 (dd, *J* = 8.9, 2.0 Hz, 1H), 6.85 - 6.79 (m, 1H), 6.73 (d, *J* = 16.0 Hz, 1H), 6.31 (s, 1H), 4.39 (s, 2H), 2.74 (d, *J* = 4.6 Hz, 3H).

LCMS (ESI), m/z: 343.1 $[M+H]^+$

Example 87: Preparation of (E)-3-(5-((3,5-difluorobenzyl)amino)-1H-indazol-3-yl)-N-ethylacrylamide (designated as DYX-236)

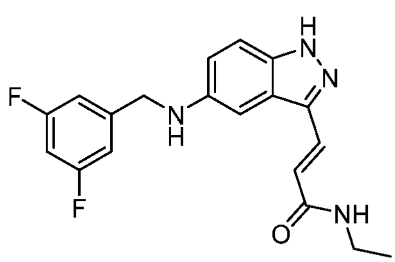

The synthesis was performed the same as Example 78 (yield: 64.1%).

$^1H$ NMR (400 MHz, DMSO-$d_6$) δ 13.05 (s, 1H), 8.09 (t, *J* = 5.6 Hz, 1H), 7.56 (d, *J* = 15.9 Hz, 1H), 7.34 (d, *J* = 8.9 Hz, 1H), 7.18 (tq, *J* = 6.2, 4.1, 3.1 Hz, 2H), 7.07 (tt, *J* = 9.3, 2.4 Hz, 1H), 6.93 (dd, *J* = 8.9, 2.1 Hz, 1H), 6.82 (s, 1H), 6.72 (d, *J* = 16.0 Hz, 1H), 6.31 (s, 1H), 4.46 - 4.33 (m, 2H), 3.23 (qd, *J* = 7.2, 5.4 Hz, 2H), 1.10 (t, *J* = 7.2 Hz, 3H).

LCMS (ESI), m/z: 357.2 [M+H]+

Example 88: Preparation of (R,E)-N-(1-(3,5-difluorophenyl)ethyl)-3-(2-(pyridin-2-yl)vinyl)-1H-indazol-5-amine (designated DYX-244-1)

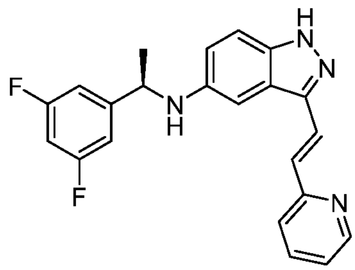

The synthesis was performed the same as Example 20 (yield: 72.4%).

$^1H$ NMR (400 MHz, DMSO-$d_6$) δ 12.90 (s, 1H), 8.58 (dd, *J* = 4.9, 1.8 Hz, 1H), 7.86 - 7.75 (m, 2H), 7.51 (d, *J* = 7.8 Hz, 1H), 7.27 (dd, *J* = 23.9, 8.2 Hz, 4H), 7.16 (d, *J* = 16.3 Hz, 1H), 7.01 (tt, *J* = 9.3, 2.4 Hz, 1H), 6.92 (dd, *J* = 8.9, 2.0 Hz, 1H), 6.87 - 6.79 (m, 1H), 6.23 (d, *J* = 7.1 Hz, 1H), 4.70 (p, *J* = 6.7 Hz, 1H), 1.47 (d, *J* = 6.6 Hz, 3H). LCMS (ESI), m/z: 377.1 [M+H]+

Example 89: Preparation of (S,E)-N-(1-(3,5-difluorophenyl)ethyl)-3-(2-(pyridin-2-yl)vinyl)-1H-indazol-5-amine (designated DYX-244-2)

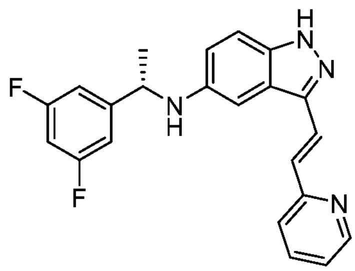

The synthesis was performed the same as Example 20 (yield: 71.0%).

$^1H$ NMR (400 MHz, DMSO-$d_6$) δ 12.92 (s, 1H), 8.58 (d, *J* = 4.7 Hz, 1H), 7.87 - 7.76 (m, 2H), 7.50 (d, *J* = 7.9 Hz, 1H), 7.28 (dd, *J* = 26.2, 8.1 Hz, 4H), 7.15 (d, *J* = 16.3 Hz, 1H), 7.05 - 6.96 (m, 1H), 6.92 (d, *J* = 8.9 Hz, 1H), 6.82 (s, 1H), 6.23 (d, *J* = 7.1 Hz, 1H), 4.70 (p, *J* = 6.9 Hz, 1H), 1.47 (d, *J* = 6.7 Hz, 3H). LCMS (ESI), m/z: 377.1 [M+H]+

Example 90: Preparation of (E)-3-(5-(((R)-1-(3,5-difluorophenyl)ethyl)amino)-1H-indazol-3-yl)-1-((S)-3-hydroxylp rrolidin-1-lro-2-en-1-one (designated as DYX-303)

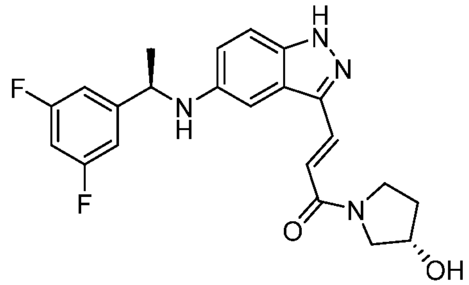

The synthesis was performed the same as Example 20 (yield: 66.0%).

$^1$H NMR (400 MHz, DMSO-$d_6$) δ 13.15 (s, 1H), 7.62 (dd, *J* = 15.7, 2.2 Hz, 1H), 7.35 (dd, *J* = 9.0, 2.1 Hz, 1H), 7.16 (h, *J* = 4.0 Hz, 2H), 6.99 (m, 2H), 6.72 - 6.54 (m, 2H), 6.36 (s, 1H), 4.72 - 4.63 (m, 1H), 4.37 (m, 1H), 3.82 (td, *J* = 9.0, 3.0 Hz, 1H), 3.65 - 3.45 (m, 4H), 2.11 - 1.77 (m, 2H), 1.45 (dd, *J* = 6.8, 3.8 Hz, 3H). LCMS (ESI), m/z: 413.2 [M+H]$^+$

Example 91: Preparation of (E)-N-(3-fluorobenzyl)-3-(2-(pyridin-2-yl)vinyl)-1H-indazol-5-amine (designated as DYX-305)

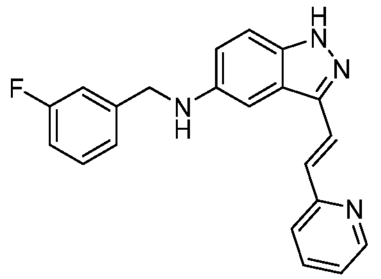

The synthesis was performed the same as Example 29 (yield: 73.9%).

$^1$H NMR (400 MHz, DMSO-$d_6$) δ 12.93 (s, 1H), 8.58 (dd, *J* = 4.9, 1.7 Hz, 1H), 7.86 (d, *J* = 16.3 Hz, 1H), 7.78 (td, *J* = 7.7, 1.9 Hz, 1H), 7.55 (d, *J* = 7.9 Hz, 1H), 7.42-7.26 (m, 5H), 7.25 - 7.21 (m, 1H), 7.04 (ddd, *J* = 10.0, 6.0, 2.3 Hz, 1H), 6.96 (dt, *J* = 7.0, 2.0 Hz, 2H), 6.28 (d, *J* = 6.0 Hz, 1H), 4.42 (d, *J* = 4.6 Hz, 2H).

LCMS (ESI), m/z: 345.1 [M+H]$^+$

Example 92: Preparation of (E)-N-(3-fluoro-5-chlorobenzyl)-3-(2-(pyridin-2-yl)vinyl)-1H-indazol-5-amine (designated as DYX-400)

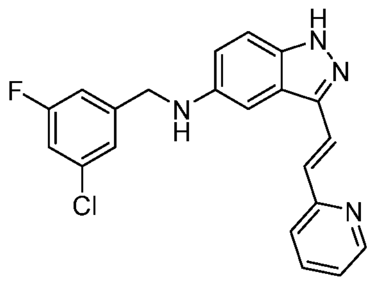

The synthesis was performed the same as Example 29 (yield: 72.9%).

$^1$H NMR (400 MHz, DMSO-$d_6$) δ 12.93 (s, 1H), 8.58 (dd, *J*= 5.0, 1.7 Hz, 1H), 7.85 (d, *J* = 16.3 Hz, 1H), 7.79 (td, *J* = 7.7, 1.9 Hz, 1H), 7.55 (d, *J* = 7.8 Hz, 1H), 7.42 (d, *J* = 1.6 Hz, 1H), 7.37 - 7.21 (m, 5H), 6.94 (dd, *J* = 6.9, 2.5 Hz, 2H), 6.34 (t, *J* = 6.1 Hz, 1H), 4.43 (d, *J* = 4.7 Hz, 2H).

LCMS (ESI), m/z: 379.1 [M+H]$^+$

Example 93: Preparation of (E)-N-(3-fluoro-5-methylbenzyl)-3-(2-(pyridin-2-yl)vinyl)-1H-indazol-5-amine (designated as DYX-401)

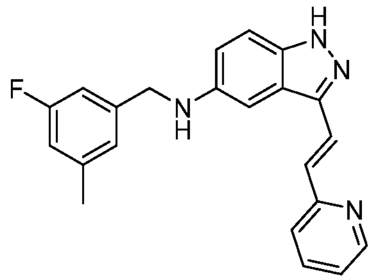

The synthesis was performed the same as Example 29 (yield: 68.3%).
$^1$H NMR (400 MHz, DMSO-$d_6$) δ 12.91 (s, 1H), 8.57 (d, *J* = 4.7 Hz, 1H), 7.89 - 7.75 (m, 2H), 7.55 (d, *J*= 7.9 Hz, 1H), 7.36 - 7.20 (m, 3H), 7.18 - 7.04 (m, 2H), 6.91 (dd, *J*= 26.2, 8.9 Hz, 3H), 6.24 (s, 1H), 4.35 (s, 2H), 2.30 (s, 3H).
LCMS (ESI), m/z: 359.1 $[M+H]^+$

Example 94: Preparation of (E)-3-fluoro-5-(((3-(2-(pyridin-2-yl)vinyl)-1H-indazol-5-yl)amino)methyl)benzonitrile (designated as DYX-402)

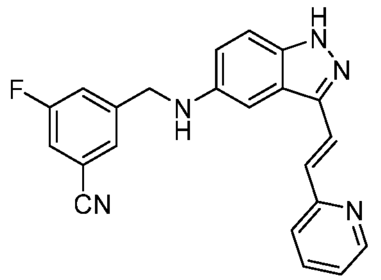

The synthesis was performed the same as Example 29 (yield: 74.0%).
$^1$H NMR (400 MHz, DMSO-$d_6$) δ 12.94 (s, 1H), 8.61 - 8.55 (m, 1H), 7.85 (d, *J* = 16.4 Hz, 2H), 7.78 (td, *J* = 7.7, 1.9 Hz, 1H), 7.70 (tdd, *J* = 10.0, 2.6, 1.3 Hz, 2H), 7.56 (d, *J* = 7.8 Hz, 1H), 7.35 (d, *J* = 8.8 Hz, 1H), 7.28 - 7.20 (m, 2H), 6.97 - 6.90 (m, 2H), 6.36 (t, *J* = 6.2 Hz, 1H), 4.48 (d, *J* = 5.7 Hz, 2H).
LCMS (ESI), m/z: 370.2 $[M+H]^+$

Example 95: Preparation of (E)-N-(3-fluoro-5-methoxybenzyl)-3-(2-(pyridin-2-yl)vinyl)-1H-indazol-5-amine (designated as DYX-403)

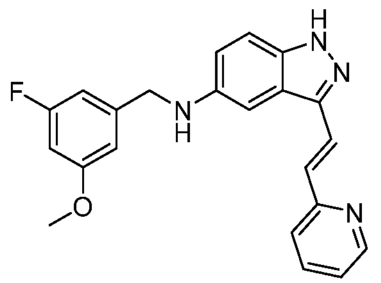

The synthesis was performed the same as Example 29 (yield: 69.0%).
$^1$H NMR (400 MHz, DMSO-$d_6$) δ 12.92 (s, 1H), 8.58 (dd, *J*= 4.8, 1.7 Hz, 1H), 7.89 - 7.75 (m, 2H), 7.54 (d, *J* =

7.8 Hz, 1H), 7.33 (d, *J* = 8.9 Hz, 1H), 7.27 - 7.20 (m, 2H), 6.97 - 6.90 (m, 3H), 6.90 - 6.84 (m, 1H), 6.67 (dt, *J* = 11.0, 2.3 Hz, 1H), 6.28 (s, 1H), 4.36 (s, 2H), 3.75 (s, 3H).

LCMS (ESI), m/z: 375.1 $[M+H]^+$

Example 96: Preparation of (R,E)-N-(1-(3,5-difluorophenyl)ethyl)-3-(2-(pyridin-3-yl)vinyl)-1H-indazol-5-amine (designated as DYX-409)

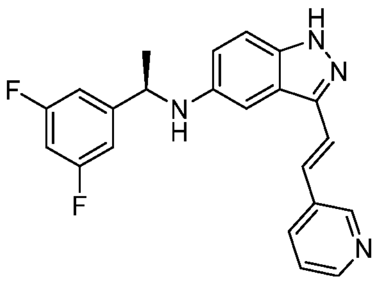

The synthesis was performed the same as Example 20 (yield: 70.0%).

$^1H$ NMR (400 MHz, DMSO-$d_6$) δ 12.86 (s, 1H), 8.77 (d, *J* = 2.2 Hz, 1H), 8.45 (dd, *J* = 4.6, 1.6 Hz, 1H), 8.07 (dt, *J* = 8.2, 2.0 Hz, 1H), 7.52 (d, *J* = 16.8 Hz, 1H), 7.40 (dd, *J* = 8.0, 4.7 Hz, 1H), 7.33 - 7.22 (m, 3H), 7.08 (d, *J* = 16.8 Hz, 1H), 7.00 (tt, *J* = 9.3, 2.4 Hz, 1H), 6.93 (dd, *J* = 8.9, 2.0 Hz, 1H), 6.83 (d, *J* = 2.0 Hz, 1H), 6.22 (d, *J* = 7.1 Hz, 1H), 4.69 (p, *J*= 6.7 Hz, 1H), 1.47 (d, *J*= 6.7 Hz, 3H).

LCMS (ESI), m/z: 377.1 $[M+H]^+$

Example 97: Preparation of (R,E)-N-(1-(3,5-difluorophenyl)ethyl)-3-(2-(pyridin-4-yl)vinyl)-1H-indazol-5-amine (designated as DYX-410)

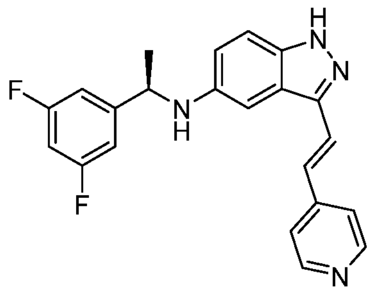

The synthesis was performed the same as Example 20 (yield: 71.4%).

$^1H$ NMR (400 MHz, DMSO-$d_6$) δ 12.98 (s, 1H), 8.55 (d, *J* = 5.0 Hz, 2H), 7.67 (d, *J*= 16.7 Hz, 1H), 7.57 (d, *J*= 5.1 Hz, 2H), 7.35 - 7.21 (m, 3H), 7.07 - 6.97 (m, 2H), 6.94 (dd, *J* = 8.9, 2.0 Hz, 1H), 6.83 (s, 1H), 6.26 (d, *J* = 6.9 Hz, 1H), 4.70 (p, *J* = 6.8 Hz, 1H), 1.47 (d, *J*= 6.6 Hz, 3H).

LCMS (ESI), m/z: 377.1 $[M+H]^+$

Example 98: Preparation of (R,E)-N-(1-(3,5-difluorophenyl)ethyl)-3-(2-(pyrazin-2-yl)vinyl)-1H-indazol-5-amine (designated as DYX-411)

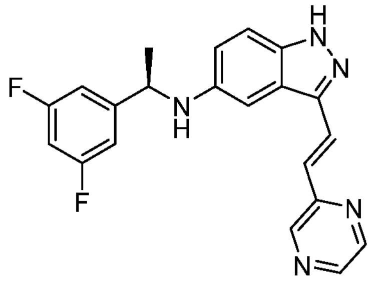

The synthesis was performed the same as Example 20 (yield: 75.0%).

$^1$H NMR (400 MHz, DMSO-$d_6$) δ 12.85 (s, 1H), 8.79 (s, 1H), 8.67 - 8.59 (m, 1H), 8.47 (d, *J* = 2.5 Hz, 1H), 7.92 (d, *J* = 16.3 Hz, 1H), 7.38 - 7.16 (m, 4H), 7.05 - 6.89 (m, 2H), 6.84 (s, 1H), 6.28 (d, *J*= 7.2 Hz, 1H), 4.71 (p, *J*= 6.8 Hz, 1H), 1.47 (d, *J* = 6.7 Hz, 3H).

LCMS (ESI), m/z: 378.1 $[M+H]^+$

Example 99: Preparation of (R,E)-N-(1-(3,5-difluorophenyl)ethyl)-3-styryl-1H-indazol-5-amine (designated as DYX-412)

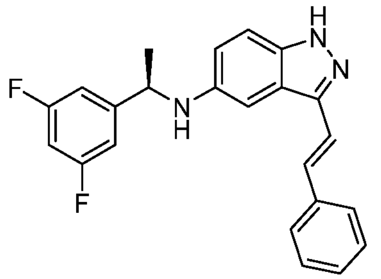

The synthesis was performed the same as Example 20 (yield: 67.5%).

$^1$H NMR (400 MHz, DMSO-$d_6$) δ 12.78 (s, 1H), 7.62 - 7.56 (m, 2H), 7.42 - 7.35 (m, 3H), 7.31 - 7.23 (m, 4H), 7.09 - 6.99 (m, 2H), 6.93 (dd, *J* = 8.9, 2.0 Hz, 1H), 6.80 (d, *J*= 2.0 Hz, 1H), 6.21 (s, 1H), 4.67 (q, *J*= 6.9 Hz, 1H), 1.47 (d, *J*= 6.7 Hz, 3H).

LCMS (ESI), m/z: 376.1 $[M+H]^+$

Example 100: Preparation of (E)-N-(2-fluorobenzyl)-3-(2-(pyridin-2-yl)vinyl)-1H-indazol-5-amine (designated as DYX-413)

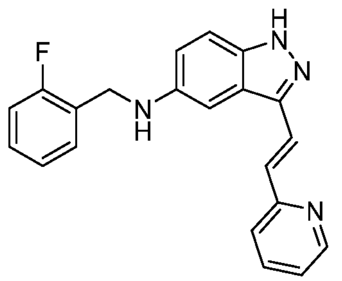

The synthesis was performed the same as Example 29 (yield: 70.4%).

$^1$H NMR (400 MHz, DMSO-$d_6$) δ 12.92 (s, 1H), 8.58 (dd, *J*= 4.8, 1.7 Hz, 1H), 7.90 - 7.76 (m, 2H), 7.59 - 7.48 (m, 2H), 7.35 - 7.21 (m, 5H), 7.17 (qd, *J* = 7.4, 6.7, 1.3 Hz, 1H), 7.03 - 6.91 (m, 2H), 6.16 (s, 1H), 4.41 (d, *J* = 3.3 Hz, 2H).

LCMS (ESI), m/z: 345.1 $[M+H]^+$

Example 101: Preparation of (E)-N-(3-fluoro-5-(trifluoromethyl)benzyl)-3-(2-(pyridin-2-yl)vinyl)-1H-indazol-5-amin e (designated as DYX-414)

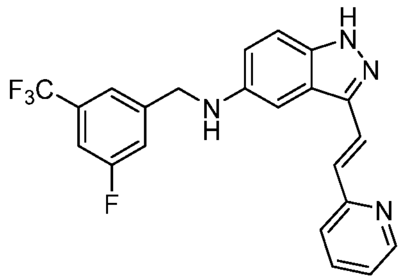

The synthesis was performed the same as Example 29 (yield: 70.0%).

$^1$H NMR (400 MHz, DMSO-$d_6$) δ 12.95 (s, 1H), 8.57 (dd, *J*= 4.8, 1.7 Hz, 1H), 7.86 (d, *J* = 16.3 Hz, 1H), 7.79 (td, *J* = 7.7, 1.8 Hz, 1H), 7.74 (s, 1H), 7.68 - 7.61 (m, 1H), 7.58 - 7.49 (m, 2H), 7.35 (d, *J* = 9.2 Hz, 1H), 7.30 - 7.21 (m, 2H), 6.95 (d, *J* = 7.3 Hz, 2H), 6.39 (s, 1H), 4.52 (s, 2H).

LCMS (ESI), m/z: 413.1 $[M+H]^+$

Example 102: Preparation of (E)-N-(3-bromo-5-fluorobenzyl)-3-(2-(pyridin-2-yl)vinyl)-1H-indazol-5-amine (designated as DYX-415)

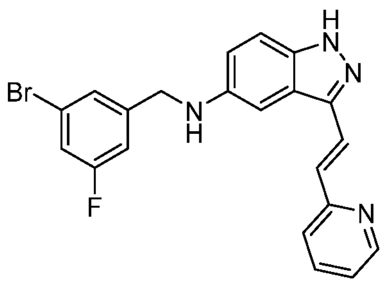

The synthesis was performed the same as Example 29 (yield: 67.3%).

$^1$H NMR (400 MHz, DMSO-$d_6$) δ 12.94 (s, 1H), 8.58 (dd, *J*= 4.8, 1.7 Hz, 1H), 7.91 - 7.76 (m, 2H), 7.57 (d, *J*= 6.4 Hz, 2H), 7.37 (ddd, *J*= 16.6, 9.1, 2.4 Hz, 3H), 7.30 - 7.20 (m, 2H), 6.94 (dd, *J* = 6.9, 2.7 Hz, 2H), 6.35 (s, 1H), 4.43 (s, 2H).

LCMS (ESI), m/z: 423.0 $[M+H]^+$

Example 103: Preparation of (E)-N-(2-chloro-5-fluorobenzyl)-3-(2-(pyridin-2-yl)vinyl)-1H-indazol-5-amine (designated as DYX-416)

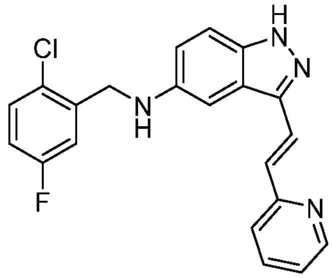

The synthesis was performed the same as Example 29 (yield: 62.6%).

$^1$H NMR (400 MHz, DMSO-$d_6$) δ 12.96 (s, 1H), 8.58 (dd, *J*= 4.9, 1.7 Hz, 1H), 7.89 - 7.76 (m, 2H), 7.58 - 7.50

(m, 2H), 7.40 - 7.30 (m, 2H), 7.29 - 7.21 (m, 2H), 7.15 (td, $J$= 8.4, 3.2 Hz, 1H), 6.99 - 6.87 (m, 2H), 6.34 (s, 1H), 4.46 (s, 2H).
LCMS (ESI), m/z: 379.1 $[M+H]^+$

Example 104: Preparation of (E)-N-(4-fluorobenzyl)-3-(2-(pyridin-2-yl)vinyl)-1H-indazol-5-amine (designated as DYX-417)

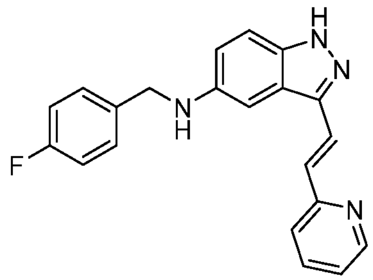

The synthesis was performed the same as Example 29 (yield: 65.9%).
$^1H$ NMR (400 MHz, DMSO-$d_6$) δ 12.91 (s, 1H), 8.58 (dd, $J$= 4.9, 1.7 Hz, 1H), 7.86 (d, $J$ = 16.3 Hz, 1H), 7.79 (td, $J$ = 7.7, 1.8 Hz, 1H), 7.56 (d, $J$ = 7.8 Hz, 1H), 7.54 - 7.47 (m, 2H), 7.32 (d, $J$ = 8.8 Hz, 1H), 7.29 - 7.21 (m, 2H), 7.20 - 7.13 (m, 2H), 6.95 (d, $J$= 8.6 Hz, 2H), 6.20 (s, 1H), 4.36 (s, 2H).
LCMS (ESI), m/z: 345.1 $[M+H]^+$

Example 105: Preparation of (E)-N-(5-fluoro-2-methoxybenzyl)-3-(2-(pyridin-2-yl)vinyl)-1H-indazol-5-amine (designated as DYX-418)

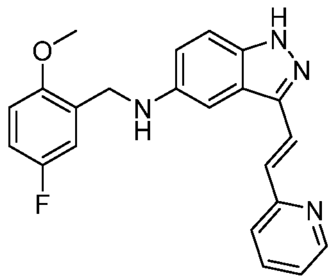

The synthesis was performed the same as Example 29 (yield: 67.4%).
$^1H$ NMR (400 MHz, DMSO-$d_6$) δ 12.94 (s, 1H), 8.57 (dd, $J$= 4.9, 1.7 Hz, 1H), 7.85 (d, $J$ = 16.4 Hz, 1H), 7.78 (td, $J$ = 7.7, 1.9 Hz, 1H), 7.57 (d, $J$ = 7.8 Hz, 1H), 7.35 (d, $J$ = 8.9 Hz, 1H), 7.30 - 7.20 (m, 2H), 7.16 (dt, $J$ = 9.4, 1.8 Hz, 1H), 7.02 (dd, $J$ = 6.4, 1.8 Hz, 2H), 6.94 (d, $J$ = 8.5 Hz, 2H), 6.13 (s, 1H), 4.34 (s, 2H), 3.88 (s, 3H).
LCMS (ESI), m/z: 375.1 $[M+H]^+$

Example 106: Preparation of (E)-N-((5-fluoropyridin-3-yl)methyl)-3-(2-(pyridin-2-yl)vinyl)-1H-indazol-5-amine (designated as DYX-419)

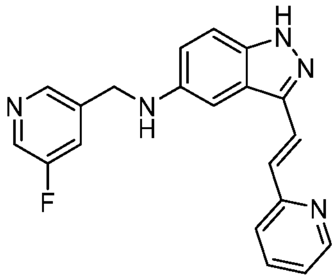

The synthesis was performed the same as Example 29 (yield: 64.1%).

$^1$H NMR (400 MHz, DMSO-$d_6$) δ 12.96 (s, 1H), 8.63 - 8.54 (m, 2H), 8.45 (d, *J* = 2.8 Hz, 1H), 7.88 (d, *J* = 16.3 Hz, 1H), 7.83 - 7.75 (m, 2H), 7.59 (d, *J* = 7.8 Hz, 1H), 7.36 (d, *J* = 8.9 Hz, 1H), 7.32 - 7.21 (m, 2H), 7.03 - 6.92 (m, 2H), 6.31 (s, 1H), 4.48 (s, 2H).

LCMS (ESI), m/z: 346.1 $[M+H]^+$

Example 107: Preparation of (R,E)-N-(1-(3-fluorophenyl)ethyl)-3-(2-(pyridin-2-yl)vinyl)-1H-indazol-5-amine (designated as DYX-320)

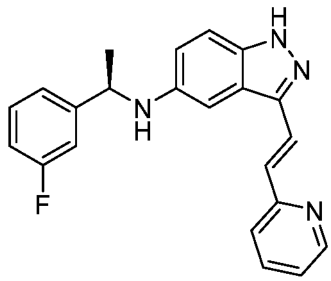

The synthesis was performed the same as Example 29 (yield: 52.1%).

$^1$H NMR (400 MHz, DMSO-$d_6$) δ 12.88 (s, 1H), 8.58 (dd, *J*= 4.8, 2.3 Hz, 1H), 7.86 - 7.74 (m, 2H), 7.51 (d, *J* = 7.8 Hz, 1H), 7.38 - 7.21 (m, 5H), 7.13 (d, *J* = 16.3 Hz, 1H), 7.03 - 6.90 (m, 2H), 6.83 (s, 1H), 6.20 (d, *J*= 7.0 Hz, 1H), 4.65 (t, *J*= 6.6 Hz, 1H), 1.47 (d, *J* = 6.7 Hz, 3H).

Example 108: Preparation of (R,E)-N-(1-(5-fluoropyridin-3-yl)ethyl)-3-(2-(pyridin-2-yl)vinyl)-1H-indazol-5-amine

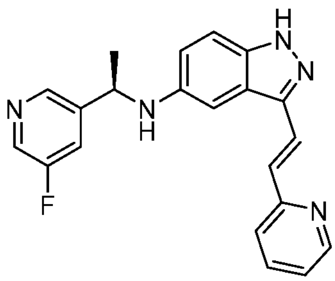

Example 109: Preparation of (R,E)-N-(1-(3,5-difluorophenyl)ethyl)-3-(2-(pyridin-2-yl)vinyl)-1H-pyrazolo[3,4-b] pyri din-5-amine (designated as DYX-318)

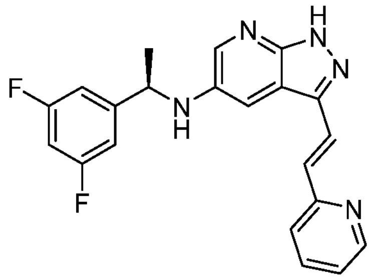

The synthesis was performed the same as Example 20 (yield: 60.6%).

$^1H$ NMR (400 MHz, DMSO-$d_6$) $\delta$ 13.43 (s, 1H), 8.59 (dd, *J*= 4.8, 1.8 Hz, 1H), 8.18 (d, *J* = 2.5 Hz, 1H), 7.85 - 7.75 (m, 2H), 7.54 (d, *J* = 7.8 Hz, 1H), 7.34 - 7.20 (m, 5H), 7.02 (tt, *J* = 9.3, 2.4 Hz, 1H), 6.51 (d, *J* = 7.5 Hz, 1H), 4.76 (p, *J* = 6.8 Hz, 1H), 1.49 (d, *J* = 6.6 Hz, 3H).

Example 110: Preparation of (R,E)-N-(1-(3,5-difluorophenyl)ethyl)-3-(2-(3-methylpyridin-2-yl)vinyl)-1H-indazol-5-amine

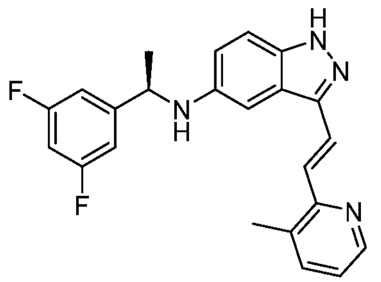

Example 111: Preparation of (E)-5-(3,5-difluorobenzyl)-3-(2-(trifluoromethyl)styryl)-1H-indazole (designated as D01F11)

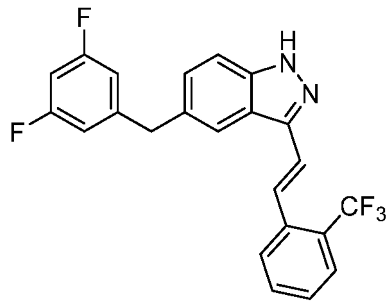

The synthesis was performed the same as Example 1 (yield: 70.3%).

LCMS (ESI), m/z: 415.1 [M+H]$^+$

Example 112: Preparation of (E)-5-((3,5-difluorophenyl)sulfonyl)-3-(2-methylstyryl)-1H-indazole (designated as E01F08)

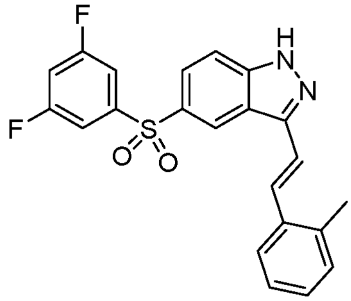

The synthesis was performed the same as Example 1 (yield: 65.6%)

LCMS (ESI), m/z: 411.1 $[M+H]^+$

Example 113: Preparation of (E)-5-((3,5-difluorophenyl)sulfonyl)-3-(2-fluorostyryl)-1H-indazole (designated as E01F10)

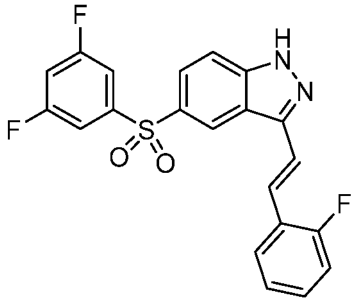

The synthesis was performed the same as Example 1 (yield: 63.2%).

LCMS (ESI), m/z: 415.0 [M+H]

Example 114: Preparation of (R,E)-2-(4-(2-(5-((1-(3,5-difluorophenyl)ethyl)amino)-1H-indazol-3-yl)vinyl)-1H-pyraz ol-1-yl)-1-ethanol (designated as DYX-327)

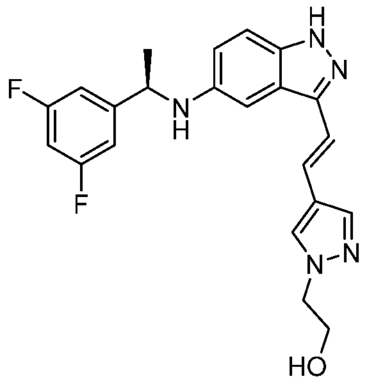

The synthesis was performed the same as Example 20 (yield: 29.5%).

$^1H$ NMR (400 MHz, DMSO-$d_6$) $\delta$ 12.60 (s, 1H), 7.90 (s, 1H), 7.70 (s, 1H), 7.27 - 7.20 (m, 3H), 7.05 - 6.98 (m, 2H), 6.91 - 6.85 (m, 2H), 6.75 - 6.70 (m, 1H), 6.13 (d, $J$ = 6.9 Hz, 1H), 4.95 (s, 1H), 4.63 (p, $J$ = 6.8 Hz, 1H), 4.14 (t, $J$ = 5.6 Hz, 2H), 3.75 (t, $J$= 5.6 Hz, 2H), 1.46 (d, $J$= 6.7 Hz, 3H).

LCMS (ESI), m/z: 410.4 $[M+H]^+$

Example 115: Preparation of (E)-N-benzyl-3-(2-(pyridin-2-yl)vinyl)-1H-indazol-5-amine (designated as DYX-314)

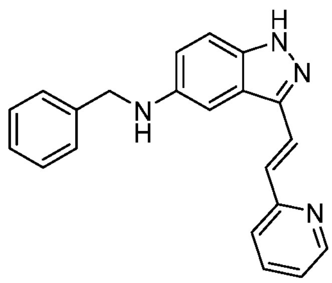

The synthesis was performed the same as Example 29 (yield: 77.3%).

$^1$H NMR (400 MHz, DMSO-$d_6$) δ 12.92 (s, 1H), 8.58 (dd, *J*= 4.8, 1.8 Hz, 1H), 7.87 (d, *J* = 16.4 Hz, 1H), 7.79 (td, *J* = 7.7, 1.9 Hz, 1H), 7.55 (d, *J* = 7.8 Hz, 1H), 7.51 - 7.45 (m, 2H), 7.38 - 7.30 (m, 3H), 7.29 - 7.19 (m, 3H), 7.01 - 6.92 (m, 2H), 6.21 (s, 1H), 4.38 (s, 2H).

LCMS (ESI), m/z: 327.1 [M+H]$^+$

Example 116: Preparation of (R,E)-2-(4-(2-(5-((1-(3-fluorophenyl)ethyl)amino)-1H-indazol-3-yl)vinyl)-1H-pyrazol-1 -yl)-1-ethanol (designated as DYX-335)

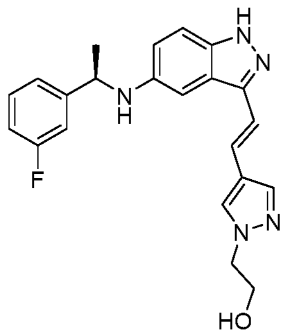

The synthesis was performed the same as Example 20 (yield: 37.2%).

$^1$H NMR (400 MHz, DMSO-$d_6$) δ 12.55 (s, 1H), 7.90 (s, 1H), 7.70 (s, 1H), 7.40 - 7.26 (m, 3H), 7.22 (d, *J* = 8.9 Hz, 1H), 7.06 - 6.95 (m, 2H), 6.92 - 6.79 (m, 2H), 6.72 (s, 1H), 6.09 (d, *J* = 6.7 Hz, 1H), 4.94 (t, *J* = 5.3 Hz, 1H), 4.58 (p, *J* = 6.9 Hz, 1H), 4.15 (t, *J*= 5.6 Hz, 2H), 3.76 (q, *J*= 5.5 Hz, 2H), 1.46 (d, *J*= 6.7 Hz, 3H).

LCMS (ESI), m/z: 392.4 [M+H]$^+$

Example 117: Preparation of (R,E)-N-(1-(3,5-difluorophenyl)ethyl)-3-(2-(pyridin-2-yl)vinyl)-1H-pyrazolo[4,3-b]pyri din-5-amine (designated as DYX-332)

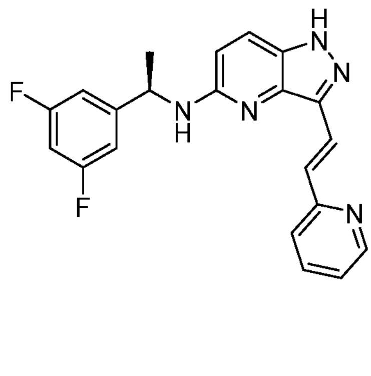

The synthesis was performed the same as Example 20 (yield: 64.5%).

$^1H$ NMR (400 MHz, DMSO-$d_6$) δ 12.90 (s, 1H), 8.58 (dd, *J*= 4.8, 1.8 Hz, 1H), 7.92 (d, *J*= 16.0 Hz, 1H), 7.81 - 7.64 (m, 3H), 7.51 (d, *J*= 7.9 Hz, 1H), 7.37 (d, *J* = 6.7 Hz, 1H), 7.28 - 7.17 (m, 3H), 6.95 (tt, *J* = 9.3, 2.4 Hz, 1H), 6.77 (d, *J* = 9.1 Hz, 1H), 5.17 (p, *J*= 6.9 Hz, 1H), 1.49 (d, *J*= 7.0 Hz, 3H).

LCMS (ESI), m/z: 375.1 $[M+H]^+$

Example 118: Preparation of (R,E)-N-(1-(3,5-difluorophenyl)ethyl)-3-(2-(pyridin-2-yl)vinyl)-1H-pyrazolo[3,4-c]pyri din-5-amine (designated as DYX-340)

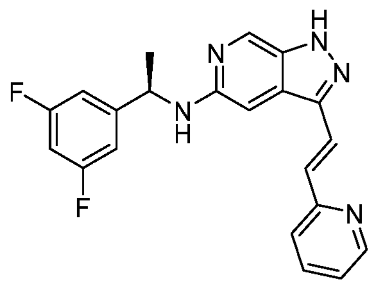

The synthesis was performed the same as Example 20 (yield: 32. 1%).

$^1H$ NMR (400 MHz, DMSO-$d_6$) δ 13.32 (s, 1H), 8.62 - 8.54 (m, 2H), 7.89 - 7.77 (m, 2H), 7.59 (d, *J*= 7.8 Hz, 1H), 7.33 - 7.18 (m, 4H), 7.03 - 6.95 (m, 1H), 6.93 (s, 1H), 6.74 (d, *J*= 8.0 Hz, 1H), 5.00 (p, *J*= 7.0 Hz, 1H), 1.47 (d, *J*= 6.8 Hz, 3H).

Example 119: Preparation of (R,E)-N-(1-(3,5 -difluorophenyl)ethyl)-3-(2-(1 -methyl-1H-pyrazol-4-yl)vinyl)-1H-indazol-5-amine (designated as DYX-346)

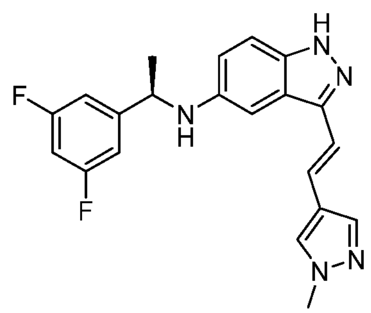

The synthesis was performed the same as Example 20 (yield: 49%).

$^1H$ NMR (400 MHz, DMSO-$d_6$) δ 12.58 (s, 1H), 7.87 (s, 1H), 7.68 (s, 1H), 7.27 - 7.19 (m, 3H), 7.06 - 6.98 (m, 2H), 6.91 - 6.84 (m, 2H), 6.73 (s, 1H), 6.13 (d, *J* = 6.9 Hz, 1H), 4.62 (p, *J*= 6.8 Hz, 1H), 3.85 (s, 3H), 1.46 (d, *J*= 6.7 Hz, 3H).

Example 120: Preparation of (R,E)-N-(1-(3,5-difluorophenyl)ethyl)-3-(2-(1-ethyl-1H-pyrazol-4-yl)vinyl)-1H-indazol -5-amine (designated as DYX-349)

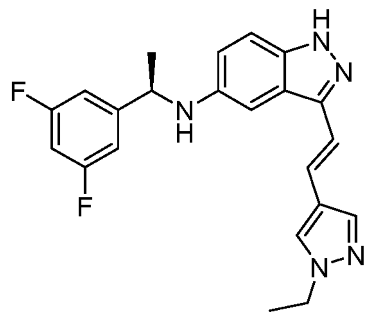

The synthesis was performed the same as Example 20 ( yield: 52%).

$^1$H NMR (400 MHz, DMSO-$d_6$) δ 12.57 (s, 1H), 7.92 (s, 1H), 7.69 (s, 1H), 7.29 - 7.19 (m, 3H), 7.06 - 6.98 (m, 2H), 6.92 - 6.83 (m, 2H), 6.73 (s, 1H), 6.13 (d, *J* = 6.8 Hz, 1H), 4.62 (p, *J*= 6.5 Hz, 1H), 4.13 (q, *J*= 7.3 Hz, 2H), 1.46 (d, *J*= 6.7 Hz, 3H), 1.40 (t, *J* = 7.3 Hz, 3H).

Example 121: Preparation of (E)-3,5-difluoro- N-(3-(2-(1-(2-(2-methoxyethyl)-1H-pyrazol-4-yl)vinyl)-1H-indazol-5-yl)benzenesulfonamide (designated as CHW-1)

Preparation of an intermediate 121 was carried out as follows:

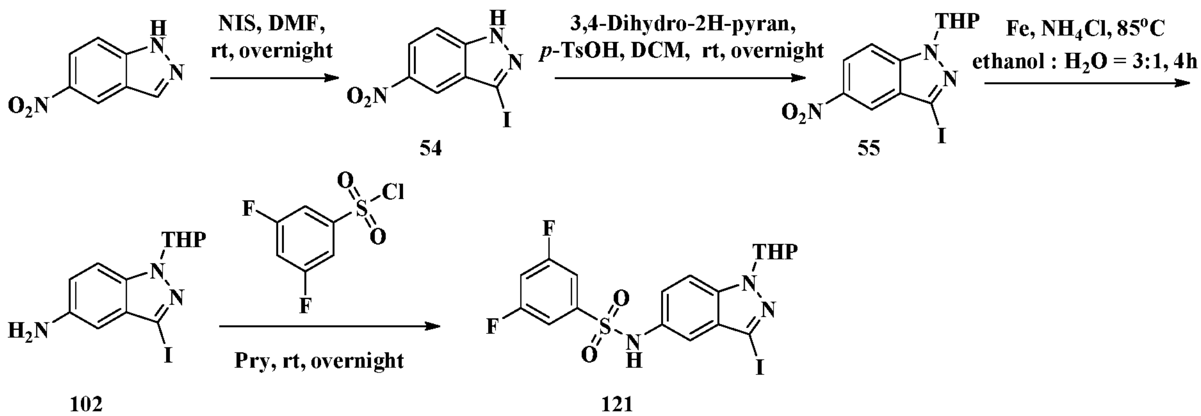


Step 1: Synthesis of 3-iodo-5-nitro-1H-indazole (intermediate 54)

5-Nitro-1H-indazole (10 g, 61.3 mmol) was added to a 250 mL dry flask. After the system was stirred to be clarified, N-iodosuccinimide (16.5 g, 73.6 mmol) was added, and reaction was carried out overnight at room temperature. After the completion of the reaction was detected by TLC, 100 mL of water was added to precipitate a large amount of yellow solid. Suction filtration was performed by using a Buchner funnel, and washed with water (30 mL*3) for three times. The obtained yellow solid was placed in a vacuum oven at 50 °C for 5 hours to obtain 16.8 g of compound 54 (yield: 94.7%), LCMS (ESI), m/z: 290.0, $[M+H]^+$

Step 2: Synthesis of 3-iodo-5-nitro-1-(tetrahydro-2H-pyran-2-yl)-1H-indazole (intermediate 55)

3-Iodo-5-nitro-1H-indazole (10 g, 34.6 mmol) and p-toluenesulfonic acid monohydrate (1 g, 5.2 mmol) were added to a 250 mL single-necked flask, then 80 mL of dry DCM was added, and the mixture was stirred to be dissolved. The solution of 3,4-dihydro-2H-pyran was added in DCM at 0 °C, and the system was gradually clarified, slowly heated, and stirred overnight at room temperature. After the completion of the reaction was detected by TLC, the reaction was quenched by adding saturated aqueous sodium bicarbonate solution. DCM was added for extraction for three times. Drying was performed with anhydrous sodium sulfate, then concentration was performed. Column chromatography was performed to obtain 8.3 g of compound 55 (yield: 64%), LCMS (ESI), m/z: 374.0, $[M+H]^+$

Step 3: Synthesis of 3-iodo-1-(tetrahydro-2H-pyran-2-yl)-1H-indazol-5-amine (intermediate 102)

3 -Iodo-5-nitro-1-(tetrahydro-2H-pyran-2-yl)-1H-indazole (6 g, 16 mmol), reduced iron powder (4.5 g, 80 mmol),

and ammonium chloride (2.58 g, 48 mmol) were added to a 250 mL single-necked flask, followed by addition of 160 mL of a mixed solution of ethanol:water (3:1). The mixture was heated to react at 80°C for 4 hours. After the completion of the reaction was detected by TLC, suction filtration was performed with diatomite, and ethyl acetate was added for extraction. The organic phase was dried, and concentrated. Column chromatography was performed to give 4.7 g of compound 102 (yield: 86%), LCMS (ESI), m/z: 344.0, $[M+H]^+$

Step 4: Synthesis of 3,5-difluoro-N-(3-iodo-1-(tetrahydro-2H-pyran-2-yl)-1H-indazol-5-yl)benzenesulfonam ide (an intermediate 121)

3-Iodo-1-(tetrahydro-2H-pyran-2-yl)-1H-indazol-5-amine (2.5 g, 7.3 mmol) was added to a 100 mL double-necked flask. 30 mL of pyridine was added under $N_2$ protection until dissolved. 3,5-difluorobenzenesulfonyl chloride (1.7 g, 8 mmol) was added with stirring so that the system become wine red, and the reaction was carried out overnight at room temperature. After the completion of the reaction was detected by TLC, saturated brine was added, followed by extraction with ethyl acetate for three times. The organic phase was dried over anhydrous sodium sulfate, and concentrated. Column chromatography was performed to give 2.0 g of compound 121 as pink solid (yield: 53%), $^1$H NMR (500 MHz, DMSO-$d_6$) $\delta$ 10.47 (s, 1H), 7.69 (d, *J* = 9.0 Hz, 1H), 7.60 (tt, *J*= 9.2, 2.4 Hz, 1H), 7.42 - 7.35 (m, 2H), 7.25 (dd, *J*= 8.9, 2.0 Hz, 1H), 7.08 (d, *J* = 1.9 Hz, 1H), 5.78 (dd, *J* = 9.7, 2.5 Hz, 1H), 3.84 (m, 1H), 3.69 (m 1H), 2.31 (m, 1H), 2.09 - 1.87 (m, 2H), 1.70 (m, 1H), 1.55 (m, 2H). LCMS (ESI), m/z: 520.0, $[M+H]^+$

Preparation of compound CHW-1 was carried out as followed:

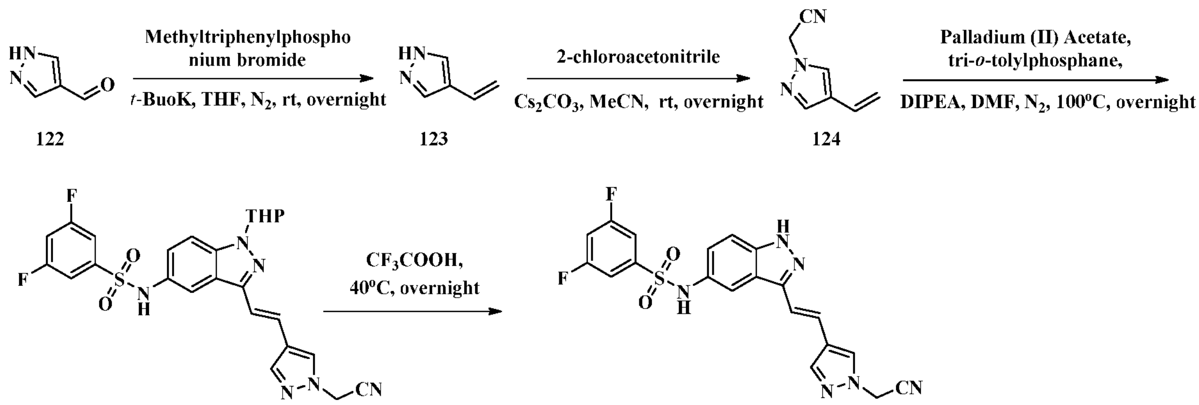


122 123 124

125 126

Step 1: Synthesis of 4-vinyl-1H-pyrazole (Intermediate 123)

Methyltriphenylphosphonium bromide (5.57 g, 15.6 mmol) was added to a 100 mL dry double-necked flask with $N_2$ replacement for five times. Solution of potassium tert-butoxide in tetrahydrofuran was added at 0°C, and a reaction was carried out for 1 hour while maintaining the temperature. Then solution of 4-Vinyl-1H-pyrazolein tetrahydrofuran was added, and the reaction was carried out overnight at room temperature. After the completion of the reaction was detected by TLC, saturated aqueous ammonium chloride solution was added with stirring for 10 minutes. Ethyl acetate was added for extraction for three times. The organic phase was dried and concentrated. Column chromatography was performed to give compound 123 (0.68 g, 69%).

$^1$H NMR (500 MHz, Chloroform-*d*) $\delta$ 7.64 (s, 1H), 7.26 (s, 1H), 6.58 (m, 1H), 5.50 (dd, *J* = 17.7, 1.4 Hz, 1H), 5.11 (dd, *J* = 11.0, 1.4 Hz, 1H). LCMS (ESI), m/z: 95.1, $[M+H]^+$

Step 2: Synthesis of 2-(4-vinyl-1H-pyrazol-1-yl)acetonitrile (intermediate 124)

4-vinyl-1H-pyrazole (0.8 g, 8 mmol), cesium carbonate (4.1 g, 12 mmol), and chloroacetonitrile (0.77 g, 10.1 mmol) were added to a 50 mL single-necked flask, followed by adding 10 mL of acetonitrile. The reaction was carried out overnight at room temperature. After the completion of the reaction was detected by TLC, the resulting reaction solution was filtrated by suction with diatomite, and the obtained filter residue was washed with acetonitrile for three times. The resulting filtrate was concentrated and column chromatography was performed to give compound 124 (1 g, 94%). $^1$H NMR (500 MHz, DMSO-$d_6$) $\delta$ 7.92 (s, 1H), 7.76 (s, 1H), 6.56 (dd, *J* = 17.7, 11.0 Hz, 1H), 5.54 (dd, *J* = 17.7, 1.5 Hz, 1H), 5.45 (s, 2H), 5.08 (dd, *J* = 11.0, 1.5 Hz, 1H). LCMS (ESI), m/z: 134.6, $[M+H]^+$

Step 3: Synthesis of (E)-N-(3-(2-(1-(cyanomethyl)-1H-pyrazol-4-yl)vinyl)-1-(tetrahydro-2H-pyran-2-yl)-1H -indazol-5-yl)-3,5-difluorobenzenesulfonamide (intermediate 125)

2-(4-vinyl-1H-pyrazol-1-yl)acetonitrile (0.199 g, 1.5 mmol), palladium acetate (11.2 mg, 0.07 mmol), tris(2-tolyl)phosphine (30 mg, 0.15 mmol), and 3,5-difluoro-N-(3-iodo-1-(tetrahydro-2H-pyran-2-yl)-1H-indazol-5-yl)benzenesulfonam ide (the intermediate 121) (500 mg, 0.99 mmol) were added to a 50 mL dry double-necked flask with $N_2$ replacement for five times, followed by addition of N,N-diisopropylethylamine (387 mg, 4.5 mmol) and 15 mL of N,N-dimethylformamide. The mixture was heated to 100 °C, and the reaction was carried out overnight. After the completion of the reaction was detected by TLC, saturated brine (150 mL) was added, and extraction was performed with ethyl acetate (20 mL). The organic phase was dried over anhydrous sodium sulfate, concentrated, and column chromatography was performed to give compound 125 (242 mg, 50%).

$^1$H NMR (500 MHz, DMSO-*d*$_6$) $\delta$ 10.39 (s, 1H), 8.14 (s, 1H), 8.04 (s, 1H), 7.96 (s, 2H), 7.75 (d, *J*= 1.9 Hz, 1H), 7.65 (d, *J*= 9.0 Hz, 1H), 7.59 (dt, *J*= 9.1, 2.3 Hz, 1H), 7.46 - 7.41 (m, 2H), 7.29 - 7.17 (m, 2H), 7.13 (dd, *J* = 8.9, 2.0 Hz, 1H), 5.53 (s, 2H), 3.87 (m, 1H), 3.71 (m, 1H), 2.51 (m, 1H), 2.43 - 2.33 (m, 1H), 2.08 - 1.93 (m, 3H), 1.81 - 1.66 (m, 1H), 1.59 (m, 2H). LCMS (ESI), m/z: 525.1, $[M+H]^+$

Step 4: Synthesis of (E)-N-(3-(2-(1-(cyanomethyl)-1H-pyrazol-4-yl)vinyl)-1H-indazol-5-yl)-3,5-difluoroben zenesulfonamide

Compound 125 (78 mg, 0.15 mmol) and trifluoroacetic acid (0.343 g, 3 mmol) were added to a 50 mL single-necked flask, and then 15 mL of dichloromethane was added. The reaction was carried out overnight at 40°C. After the completion of the reaction was detected by TLC, a large amount of trifluoroacetic acid were removed by rotary evaporation under vacuum. The reaction was quenched by adding saturated sodium bicarbonate, and extraction was performed with ethyl acetate for three times. The organic phase was dried over anhydrous sodium sulfate, and concentrated. Column chromatography was performed to give compound 126 (designated as CHW-1) (32 mg, 50%).

$^1$H NMR (500 MHz, DMSO-*d*$_6$) $\delta$ 13.10 (s, 1H), 8.12 (s, 1H), 8.01 (s, 1H), 7.72 (d, *J* = 1.8 Hz, 1H), 7.59 (dt, *J* = 9.1, 2.4 Hz, 1H), 7.51 - 7.37 (m, 3H), 7.32 - 7.11 (m, 2H), 7.06 (dd, *J* = 8.9, 1.9 Hz, 1H), 5.52 (d, *J* = 7.1 Hz, 2H). LCMS (ESI), m/z: 441.1, $[M+H]^+$

Example 122: Preparation of (E)-N-(3-(2-(1-acetyl-1H-pyrazol-4-yl)vinyl)-1H-indazol-5-yl)-3,5-difluorobenzenesulf onamide (designated as CHW-2)

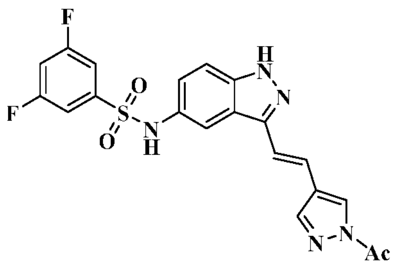

The synthesis was performed the same as Example 121 (yield: 35%).

$^1$H NMR (500 MHz, DMSO-*d*$_6$) $\delta$ 10.27 (s, 1H), 7.94 (s, 2H), 7.69 (d, *J* = 1.8 Hz, 1H), 7.60 (dt, *J*= 9.0, 2.4 Hz, 1H), 7.48 - 7.36 (m, 3H), 7.16 (m, 2H), 7.05 (dd, *J* = 8.9, 1.9 Hz, 1H), 2.50 (p, *J*= 1.9 Hz, 3H). LCMS (ESI), m/z: 444.1, $[M+H]^+$

Example 123: Preparation of (E)-3,5-difluoro-N-(3-(2-(1-(1-(2,2,2-trifluoroethyl)-1H-pyrazol-4-yl)vinyl)-1H-indazol -5-yl)benzenesulfonamide (designated as CHW-3)

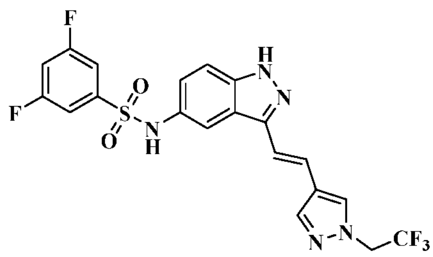

The synthesis was performed the same as Example 121 (yield: 61%).

$^1H$ NMR (500 MHz, DMSO-$d_6$) $\delta$ 10.30 (s, 1H), 8.12 (s, 1H), 8.00 (s, 1H), 7.73 (d, *J* = 1.8 Hz, 1H), 7.59 (dt, *J*= 9.1, 2.4 Hz, 1H), 7.49 - 7.37 (m, 3H), 7.29 - 7.11 (m, 2H), 7.06 (dd, *J* = 8.9, 1.9 Hz, 1H), 5.15 (q, *J* = 9.1 Hz, 2H). LCMS (ESI), m/z: 484.1, $[M+H]^+$

Example 124: Preparation of (E)-N-(3-(2-(1-(cyclopropyl-1H-pyrazol-4-yl)vinyl)-1H-indazol-5-yl)-3,5-difluorobenze nesulfonamide (designated as CHW-4)

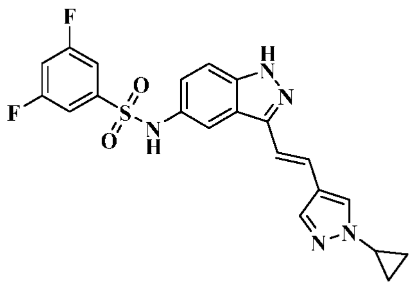

The synthesis was performed the same as Example 121 (yield 73%).

$^1H$ NMR (500 MHz, DMSO-$d_6$) $\delta$ 13.05 (s, 1H), 10.29 (s, 1H), 8.08 (s, 1H), 7.78 (s, 1H), 7.70 (s, 1H), 7.60 (dt, *J* = 9.2, 2.4 Hz, 1H), 7.47 - 7.37 (m, 2H), 7.21 - 7.07 (m, 2H), 7.05 (dd, *J*= 8.9, 1.9 Hz, 1H), 3.74 (tt, *J*= 7.5, 3.9 Hz, 1H), 1.08 (m, 1H), 0.99 (m, 1H). LCMS (ESI), m/z: 442.1, $[M+H]^+$

Example 125: Preparation of (E)-3-fluoro-N-(3-(2-(pyridin-2-yl)vinyl)-1H-indazol-5-yl)benzene sulfonamide (designated as CHW-5)

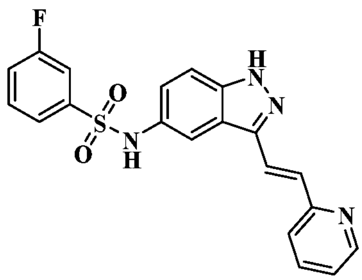

The synthesis was performed the same as Example 27 (yield: 55%).

$^1H$ NMR (500 MHz, DMSO-$d_6$) $\delta$ 13.30 (s, 1H), 10.25 (s, 1H), 8.61 (d, *J*= 5.0, 1H), 7.90 - 7.78 (m, 2H), 7.74 (m, 1H), 7.65 (d, *J* = 7.9 Hz, 1H), 7.62 - 7.43 (m, 6H), 7.34 (s, 1H), 7.33 - 7.25 (m, 1H), 7.13 (m, 1H). LCMS (ESI), m/z: 495.1, $[M+H]^+$

Example 126: Preparation of (E)-N-(3-(2-(1-(1-( difluoromethyl)-1H-pyrazol-4-yl)vinyl)-1H-indazol-5-yl)-3,5-difluor obenzene sulfonamide (designated as CHW-6)

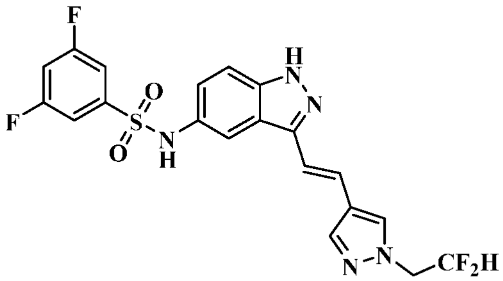

The synthesis was performed the same as Example 121 (yield: 85%).

$^1$H NMR (500 MHz, DMSO-$d_6$) $\delta$ 13.07 (s, 1H), 10.28 (s, 1H), 8.06 (s, 1H), 7.93 (s, 1H), 7.71 (s, 1H), 7.60 (dt, *J* = 9.2, 2.4 Hz, 1H), 7.47 - 7.35 (m, 3H), 7.25 - 7.09 (m, 2H), 7.05 (dd, *J* = 8.9, 1.8 Hz, 1H), 6.39 (tt, *J*= 54.9, 3.8 Hz, 1H), 4.65 (td, *J* = 15.1, 3.8 Hz, 2H). LCMS (ESI), m/z: 466.1, $[M+H]^+$

Example 127: Preparation of (E)-3,5-difluoro-N-(3-(2-(1-isopropyl-1H-pyrazol-4-yl)vinyl)-1H-indazol-5-yl)benzenes ulfonamide (designated as CHW-7)

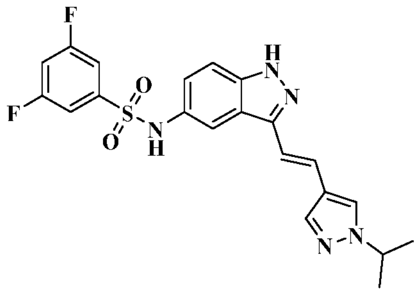

The synthesis was performed the same as Example 121 (yield: 36%).

$^1$H NMR (500 MHz, DMSO-$d_6$) $\delta$ 13.03 (s, 1H), 10.29 (s, 1H), 8.07 (s, 1H), 7.78 (s, 1H), 7.69 (s, 1H), 7.60 (td, *J* = 9.1, 4.6 Hz, 1H), 7.47 - 7.35 (m, 3H), 7.22 - 7.08 (m, 2H), 7.05 (dd, *J* = 8.8, 1.8 Hz, 1H), 4.50 (m, 1H), 1.46 (d, *J* = 6.7 Hz, 6H). LCMS (ESI), m/z: 444.1, $[M+H]^+$

Example 128: Preparation of (E)-3,5-difluoro-N-(3-(2-(pyridin-3-yl)vinyl)-1H-indazol-5-yl)benzenesulfonamide (designated as CHW-8)

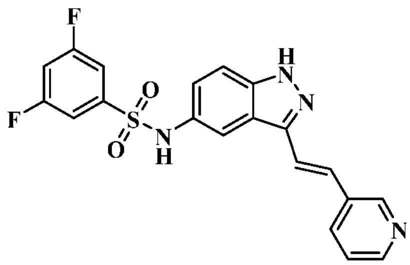

The synthesis was performed the same as Example 121 (yield 41%).

$^1$H NMR (500 MHz, DMSO-$d_6$) $\delta$ 13.28 (s, 1H), 10.33 (s, 1H), 8.83 (m, 1H), 8.48 (dd, *J*= 4.7, 1.5 Hz, 1H), 8.15

(d, $J$ = 7.8 Hz, 1H), 7.83 (m, 1H), 7.66 (m, 1H), 7.58 (tt, $J$ = 9.0, 2.4 Hz, 1H), 7.51 - 7.30 (m, 6H), 7.08 (dd, $J$ = 8.8, 1.9 Hz, 1H). LCMS (ESI), m/z: 413.1, $[M+H]^+$

Example 129: Preparation of (E)-5-fluoro-N-(3-(2-(pyridin-2-yl)vinyl)-1H-indazol-5-yl)pyridine-3-sulfonamide (designated as CHW-9)

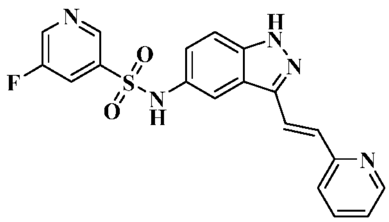

The synthesis was performed the same as the Example27 (yield: 43%).

$^1$H NMR (500 MHz, DMSO-$d_6$) $\delta$ 13.36 (s, 1H), 10.47 (d, $J$ = 2.4 Hz, 1H), 8.83 (d, $J$ = 2.7 Hz, 1H), 8.72 (q, $J$= 1.6 Hz, 1H), 8.67 - 8.56 (m, 1H), 8.03 (dt, $J$= 8.3, 2.2 Hz, 1H), 7.99 - 7.76 (m, 3H), 7.69 (d, $J$ = 7.8 Hz, 1H), 7.52 (d, $J$ = 8.9 Hz, 1H), 7.38 (m, 1H), 7.33 - 7.26 (m, 1H), 7.14 (m, 1H). LCMS (ESI), m/z: 396.1, $[M+H]^+$

Example 130: Preparation of (E)-2-(4-(2-(5-((3,5-difluorophenyl)sulfonamido)-1H-indazol-3-yl)vinyl)-1H-pyrazol-1-yl)-N,N-dimethylacetamide (designated CHW-10)

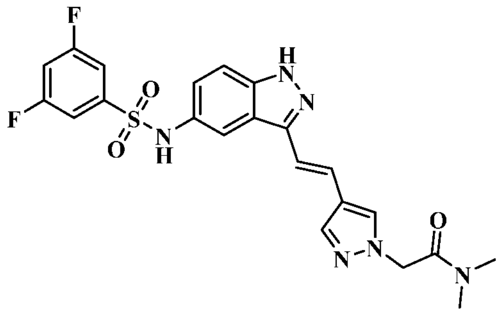

The synthesis was performed the same as Example 121 (yield: 91%).

$^1$H NMR (500 MHz, DMSO-$d_6$) $\delta$ 10.27 (s, 1H), 7.91 (s, 1H), 7.80 (s, 1H), 7.69 (s, 1H), 7.63 - 7.57 (td, $J$= 9.1, 4.5 Hz, 1H), 7.46 - 7.37 (m, 3H), 7.19 - 7.08 (m, 2H), 7.05 (dd, $J$ = 8.8, 1.8 Hz, 1H), 5.11 (s, 2H), 3.05 (s, 3H), 2.87 (s, 3H). LCMS (ESI), m/z: 487.1, $[M+H]^+$

Example 131: Preparation of (E)-N-(3-(2-(1-(1-(difluoromethyl)-1H-pyrazol-4-yl)vinyl)-1H-indazol-5-yl)-3,5-difluor obenzenesulfonamide (designated as CHW-11)

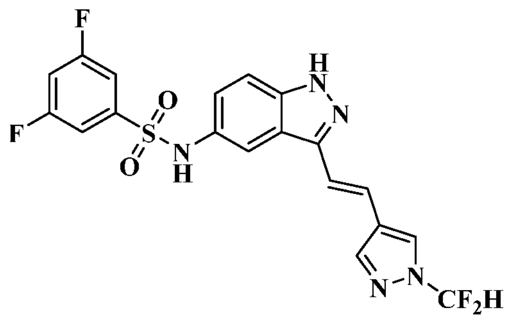

The synthesis was performed the same as Example 121 (yield: 78%).

$^1H$ NMR (500 MHz, DMSO-$d_6$) $\delta$ 13.07 (s, 1H), 10.28 (s, 1H), 8.06 (s, 1H), 7.93 (s, 1H), 7.71 (d, *J*= 1.8 Hz, 1H), 7.60 (dt, *J*= 9.2, 2.4 Hz, 1H), 7.49 - 7.33 (m, 3H), 7.26 - 7.09 (m, 2H), 7.05 (dd, *J* = 8.9, 1.8 Hz, 1H), 6.39 (tt, *J* = 54.9, 3.8 Hz, 1H). LCMS (ESI), m/z: 452.1, $[M+H]^+$

Example 132: Preparation of (E)-3,5-difluoro-N-(3-(2-(1-(2-(2-isopropoxyethyl)-1H-pyrazol-4-yl)vinyl)-1H-indazol-5-yl)benzenesulfonamide (designated as CHW-12)

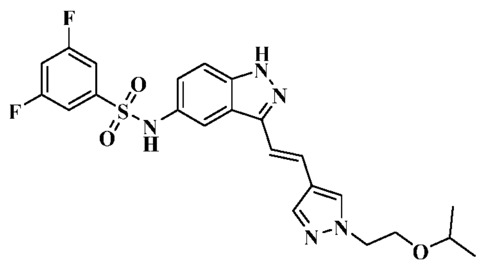

The synthesis was performed the same as Example 121 (yield: 87%).

$^1H$ NMR (500 MHz, DMSO-$d_6$) $\delta$ 10.29 (s, 1H), 7.99 (s, 1H), 7.82 (s, 1H), 7.69 (s, 1H), 7.60 (td, *J* = 9.1, 4.5 Hz, 1H), 7.47 - 7.36 (m, 3H), 7.19 - 7.08 (m, 2H), 7.06 (dd, *J* = 8.9, 1.8 Hz, 1H), 4.23 (t, *J* = 5.5 Hz, 2H), 3.75 (t, *J* = 5.5 Hz, 2H), 2.00 (s, 1H), 1.07 (d, *J* = 6.1 Hz, 6H). LCMS (ESI), m/z: 488.2, $[M+H]^+$

Example 133: Preparation of (E)-N-(3-(2-(1-(cyanomethyl)-1H-pyrazol-4-yl)vinyl)-1H-indazol-5-yl)-3,5-difluoroben zenesulfonamide (designated as CHW-13)

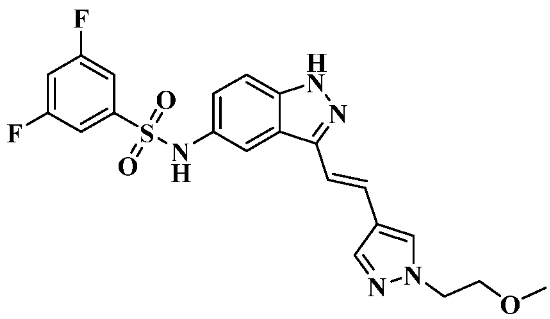

The synthesis was performed the same as Example 121 (yield: 50%).

$^1H$ NMR (500 MHz, DMSO-$d_6$) $\delta$ 13.04 (s, 1H), 10.29 (s, 1H), 8.00 (s, 1H), 7.81 (s, 1H), 7.69 (s, 1H), 7.60 (dt, *J*= 9.2, 2.4 Hz, 1H), 7.47 - 7.38 (m, 3H), 7.19 - 7.08 (m, 2H), 7.06 (dd, *J*= 8.9, 1.9 Hz, 1H), 4.28 (t, *J*= 5.3 Hz, 2H), 3.72 (t, *J*= 5.3 Hz, 2H), 3.27 (s, 3H). LCMS (ESI), m/z: 460.1, $[M+H]^+$

Example 134: Preparation of (E)-2-(4-(2-(5-((3,5-difluorophenyl)sulfonamido)-1H-indazol-3-yl)vinyl)-1H-pyrazol-1-yl)-N-methylacetamide (designated as CHW-14)

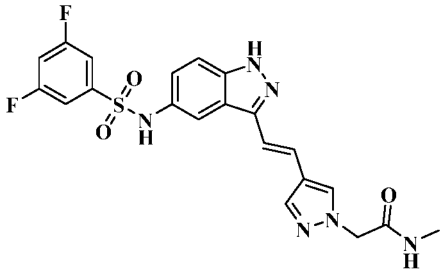

The synthesis was performed the same as Example 121 (yield: 80%).

$^1$H NMR (500 MHz, DMSO-$d_6$) $\delta$ 13.04 (s, 1H), 10.27 (s, 1H), 7.99 (s, 1H), 7.83 (s, 1H), 7.69 (s, 1H), 7.60 (dt, *J* = 9.1, 2.4 Hz, 1H), 7.45 - 7.36 (m, 3H), 7.22 - 7.08 (m, 2H), 7.05 (dd, *J* = 8.8, 1.9 Hz, 1H), 4.78 (s, 2H), 2.64 (d, *J* = 4.6 Hz, 3H). LCMS (ESI), m/z: 473.1, $[M+H]^+$

Example 135: Preparation of (E)-3,5-difluoro- N-(3-(2-(1-methyl-1H-pyrazol-4-yl)vinyl)-1H-indazol-5-yl)benzenesul fonamide (designated as D5217)

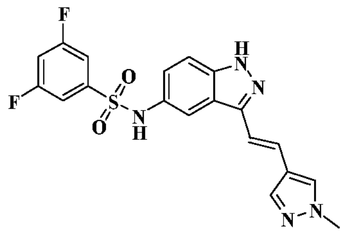

The synthesis was performed the same as Example 121 (yield: 51%).

$^1$H NMR (400 MHz, DMSO-$d_6$) $\delta$ 13.02 (s, 1H), 10.26 (s, 1H), 7.96 (s, 1H), 7.77 (s, 1H), 7.66 (s, 1H), 7.61 (dt, *J* = 9.2, 2.4 Hz, 1H), 7.47 (m, 1H), 7.25 - 7.09 (m, 2H), 7.05 (dd, *J*= 8.9, 1.8 Hz, 1H), 6.39 (tt, *J*= 54.9, 3.8 Hz, 1H), 3.85 (td, *J*= 15.1, 3.8 Hz, 3H).

LCMS (ESI), m/z: 414.4 $[M+H]^+$

Example 136: Preparation of (E)-3,5-difluoro-N-(3-(2-(1-ethyl-1H-pyrazol-4-yl)vinyl)-1H-indazol-5-yl)benzenesulfo namide (designated as D5218)

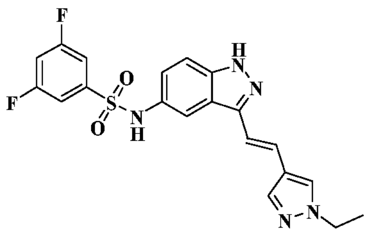

The synthesis was performed the same as Example 121 (yield 46%).

$^1$H NMR (400 MHz, DMSO-$d_6$) $\delta$ 13.02 (s, 1H), 10.26 (s, 1H), 7.96 (s, 1H), 7.77 (s, 1H), 7.66 (s, 1H), 7.61 (dt, *J*= 9.2, 2.4 Hz, 1H), 7.47 (m, 1H), 7.25 - 7.09 (m, 2H), 7.05 (dd, *J*= 8.9, 1.8 Hz, 1H), 6.39 (tt, *J*= 54.9, 3.8 Hz, 1H), 4.15 (td, *J*= 15.1, 3.8 Hz, 2H), 1.40 (td, *J*= 15.1, 3.8 Hz, 3H).

LCMS (ESI), m/z: 428.4 $[M+H]^+$

Example 137: Preparation of (E)-3,5-difluoro-N-(3-(2-(1H-pyrazol-4-yl)vinyl)-1H-indazol-5-yl)benzenesulfonamide (designated as D5220)

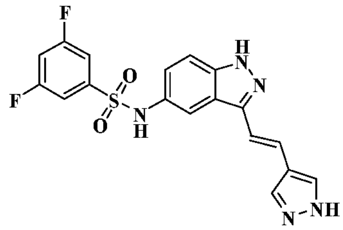

The synthesis was performed the same as Example 121 (yield: 47%).
$^1H$ NMR (400 MHz, DMSO-$d_6$) $\delta$ 13.02 (s, 1H), 10.26 (s, 1H), 7.96 (s, 1H), 7.77 (s, 1H), 7.66 (s, 1H), 7.61 (dt, *J* = 9.2, 2.4 Hz, 1H), 7.47 (m, 1H), 7.25 - 7.09 (m,3H), 7.05 (dd, *J*= 8.9, 1.8 Hz, 1H), 6.39 (tt, *J*= 54.9, 3.8 Hz, 1H).
LCMS (ESI), m/z: 400.4 $[M+H]^+$

Example 138: Preparation of (E)-3,5-difluoro-N-(3-{2-[1-(2-hydroxyl-ethyl)-1H-pyrazol-4-yl]-vinyl}-1H-indazol-5-y 1)-benzenesulfonamide (designated as D5221)

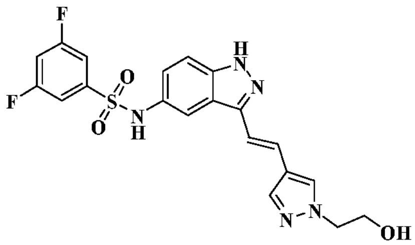

The synthesis was performed the same as Example 121 (yield: 42%).
$^1H$ NMR (400 MHz, DMSO-$d_6$) $\delta$ 13.02 (s, 1H), 10.26 (s, 1H), 7.96 (s, 1H), 7.77 (s, 1H), 7.66 (s, 1H), 7.61 (dt, *J*= 9.2, 2.4 Hz, 1H), 7.47 (m, 1H), 7.25 - 7.09 (m, 2H), 7.05 (dd, *J*= 8.9, 1.8 Hz, 1H), 6.39 (tt, *J*= 54.9, 3.8 Hz, 1H), 4.15 (td, *J*= 15.1, 3.8 Hz, 2H), 1.40 (td, *J* = 15.1, 3.8 Hz, 2H).
LCMS (ESI), m/z: 444.4 $[M+H]^+$

Example 139 $IC_{50}$ test of compounds on TRKs

Kinase activity assay: Z'-LYTE™ technology (detection by fluorescence, enzyme-conjugated format, based on the difference in sensitivity of phosphorylated and non-phosphorylated peptides to proteolytic cleavage) was applied, and using the principle of fluorescence resonance energy transfer (FRET), a Z'-LYTE™ FRET peptide substrate (Z'-LYTE™ Tyrosine 1 Peptide Substrate, Invitrogen, PV3190), and a secondary reaction were used to detect the inhibitory activity of the compounds on TRKs (TRK1, TRK2, TRK3) (life technologies, PV3144, PV3616, PV3617).
Enzymatic reaction: In a 384-well plate, 5 μL of an enzyme-substrate system (50 mM HEPES pH 7.5, 0.01% BRIJ-35, 10 mM $MgCl_2$, 1 mM EGTA, 2 μM Tyr 01 Peptide Substrate) was added and 5 nL of compound (concentration gradient) was transferred thereto by using the echo520 ultramicro liquid pipetting system. After shaking for 10-20 min at room temperature, 200 nL, 12.5 nL and 25 nL of ATP (final concentrations are 400 uM, 25 uM and 50 uM, respectively) were transferred by using the echo520 ultramicro liquid pipetting system. After shaking and mixing, centrifuging was performed. A reaction was carried out at 30 °C in the dark for 1.5 hours.
Assay reaction: 2.5 μL of Development Solution (1:128 dilution) was added in each well, and incubated at 37 °C in the dark for 1 hour, and then 5 μL of Stop Reagent was added.
Plate reading: fluorescence signals were detected using a Perkin Elmer EnVision Multimode Plate Reader (excitation at 400 nm, and emission at 460 nm, and 535 nm).

Calculation: the inhibition rate of each well was calculated by the fully active well and control signal wells. The data were analyzed as follows:

$$\text{Phosphorylation ratio} = 1-\{(\text{emission ratio} \times F100\%-C100\%)/[C0\%-C100\% + \text{emission ratio} \times (F100\%-F0\%)]\} \times 100;$$

$$\text{Inhibition rate} = 100 \times (1-\text{compound phosphorylation ratio}/\text{negative control phosphorylation ratio}).$$

$IC_{50}$ values were calculated by using GraphPad Prism 5.0 software.

The results of the kinase activity test are shown in Table 1.

Table 1 Compounds kinase activity test results ($IC_{50}$: nM)

| Compound number | TRKA | TRKB | TRKC |
|---|---|---|---|
| DYX-048 | 33.2 | 15.3 | 9.7 |
| DYX-032 | 14.5 | 56.1 | 12.0 |
| DYX-038 | 9.3 | 32.4 | 1.9 |
| DYX-042 | 251.2 | 281.2 | 113.7 |
| DYX-082 | 15.2 | 67.8 | 8.5 |
| DYX-060 | 817.9 | 374.7 | 619.6 |
| DYX-054 | 7.1 | 12.2 | 5.4 |
| DYX-081 | 39.8 | 170.0 | 26.6 |
| DYX-059 | 250.3 | > 1000 | 160.1 |
| CCB-489 | 4.6 | 14.4 | 2.7 |
| DYX-083 | 627.5 | > 1000 | 190.6 |
| DYX-134X | 70.9 | 211.0 | 28.3 |
| DYX-135X | 49.4 | 70.5 | 22.5 |
| DYX-136X | 4.8 | 91.0 | 5.8 |
| DYX-137X | 33.8 | 80.8 | 21.9 |
| DYX-138X | 757.0 | 356.0 | 262.0 |
| DYX-101 | 21.8 | 36.6 | 24.8 |
| DYX-090 | 9.9 | 79.9 | 35.7 |
| DYX-114 | 1.2 | 7.0 | 2.8 |
| DYX-105 | 105.8 | 353.3 | 226.4 |
| DYX-139 | 15.0 | 25.7 | 18.1 |
| DYX-132 | 3.3 | 9.8 | 3.0 |
| DYX-176 | 7.1 | 21.4 | 6.5 |
| DYX-191 | 9.1 | 37.1 | 16.6 |
| DYX-193 | 45.4 | 120.0 | 33.2 |
| DYX-164 | 196.0 | 741.0 | 226.0 |
| DYX-165 | 1.3 | 3.2 | 2.4 |

(continued)

| Compound number | TRKA | TRKB | TRKC |
|---|---|---|---|
| DYX-174 | 46.8 | 242.0 | 94.1 |
| DYX-185 | 1.7 | 10.0 | 1.9 |
| DYX-187 | 18.7 | 20.3 | 6.2 |
| DYX-171 | 577.0 | >1000 | 532.0 |
| DYX-183 | 598.2 | >1000 | 456.2 |
| DYX-180 | 3.9 | 9.9 | 4.7 |
| DYX-119 | 648.3 | >1000 | 226.4 |
| DYX-149 | 317.7 | >1000 | 155.3 |
| DYX-150 | 3.3 | 9.4 | 4.1 |
| DYX-014 | 100 | 321 | 95 |
| DYX-021 | 110 | 360 | 79 |
| DYX-025 | 26 | 82 | 12 |
| DYX-027 | >1000 | >1000 | >1000 |
| DYX-041 | 44 | 40 | 8 |
| DYX-074 | 72 | 435 | 41 |
| CCB-484 | 34.0 | 83.8 | 11.8 |
| DYX-044 | 501.0 | >1000 | 652.0 |
| DYX-051 | >1000 | >1000 | >1000 |
| DYX-071 | 217 | 385 | 113 |
| DYX-075 | >1000 | >1000 | >1000 |
| DYX-092 | 51.7 | 310.2 | 199.9 |
| DYX-086 | 54.2 | 241.2 | 111.0 |
| DYX-108 | 8.1 | 43.1 | 21.4 |
| DYX-129 | >1000 | >1000 | 574.7 |
| DYX-130 | 319.3 | 993.1 | 235.3 |
| DYX-115 | > 1000 | > 1000 | > 1000 |
| DYX-131 | 31.3 | 71.3 | 20.0 |
| DYX-134Q | 91.9 | 124.1 | 40.3 |
| DYX-135Q | 291.9 | 817.2 | 211.5 |
| DYX-137Q | 213.4 | 396.7 | 91.1 |
| DYX-03 | 4.0 | 10.9 | 8.6 |
| DYX-215 | 3.3 | 22.4 | 20.9 |
| DYX-07 | 10.6 | 40.3 | 104.3 |
| DYX-10 | 9.2 | 3.2 | 18.8 |
| DYX-05 | 1.6 | 10.9 | 6.0 |
| DYX-06 | 14.1 | 87.1 | 77.4 |
| DYX-09 | 10.6 | 14.8 | 4.2 |
| DYX-04 | 21.8 | 32.4 | 5.1 |

(continued)

| Compound number | TRKA | TRKB | TRKC |
|---|---|---|---|
| DYX-216A | 1.1 | 6.5 | 1.3 |
| DYX-170 | 8.1 | 18.4 | 1.9 |
| DYX-244-1 | 2.6 | 4.4 | 0.8 |
| DYX-244-2 | 16.3 | 22.1 | 1.9 |
| DYX-303 | 3.0 | 4.8 | 0.8 |
| DYX-305 | 1.8 | 9.5 | 1.3 |

It can be seen from the data in Table 1 that the indazole derivatives of the present disclosure have strong inhibitory activity on TRKs.

Example 140: Cell proliferation inhibitory activity study based on Ba/F3-TRK stable strain

The BaF3 cells (mouse pre-B cells) used in this experiment were purchased from the Japanese Cell Bank, and BaF3-CD74-NTRK1, BaF3-ETV6-NTRK2, and BaF3-ETV6-NTRK3 monoclonal stable strains were constructed in our laboratory and were confirmed as completely correct by positive drug activity, protein expression, gene sequencing and other experiments.

The brief steps of stable strain construction are as follows: construct a pCDNA3.1(+) plasmid vector carrying genes such as CD74-NTRK1, ETV6-NTRK2 and ETV6-NTRK3; the plasmid was electroporated into Ba/F3 cells using the Amaxa® Cell Line Nucleofector® Kit V; 48 hours after electroporated, G418 with a final concentration of 1000 μg/ml was added for screening for two weeks, and then IL3 was removed, and continued screening to obtain polyclonal stable strains; then single clones were selected by a limiting dilution method. The stable strains were then identified using positive drugs, WB and gene sequencing; and the monoclonal clones that were identified as completely correct may be used for the study of cell proliferation inhibitory activity of inhibitors.

Cell proliferation inhibition activity study: the cells in the logarithmic growth phase were seeded into 96-well plates at 8000-12000 cells/well, and inhibitors at different concentrations (0-10 μM) were added the next day, and incubation was continued for 72 hours; then 10 μL of CCK-8 reagent was added to each well, and the incubation was continued for 1-3 hours; and then use a super microplate reader to measure their absorbance values at 450 nm and 650 nm. The half-inhibition concentration (IC50) was calculated using GraphPad Prism 8.0.0.

The test results are shown in Table 2.

Table 2 Test Results of Compound Cell Activity ($IC_{50}$: nM)

| Cpd | CD74-NTRK1 | ETV6-NTRK2 | ETV6-NTRK3 |
|---|---|---|---|
| DYX-048 | 803.0 | 167.6 | 959.0 |
| DYX-032 | 1916.0 | 2342.0 | 717.6 |
| DYX-038 | 239.0 | 335.3 | 133.4 |
| DYX-042 | 8714.0 | > 10000 | 8385.0 |
| DYX-082 | 583.5 | 247.7 | 159.3 |
| DYX-060 | > 10000 | > 10000 | > 10000 |
| DYX-054 | 546.0 | 328.8 | 145.3 |
| DYX-081 | 1441.0 | 1016.0 | 819.7 |
| DYX-059 | 7935.0 | 8818.0 | 3253.0 |
| CCB-489 | 145.7 | 165.4 | 56.2 |
| DYX-083 | 7503.0 | 3020.0 | 3311.0 |
| DYX-134X | 2358.0 | 1876.0 | 846.2 |
| DYX-135X | 2991.0 | 2980.0 | 2398.0 |

(continued)

| Cpd | CD74-NTRK1 | ETV6-NTRK2 | ETV6-NTRK3 |
|---|---|---|---|
| DYX-136X | 602.6 | 1072.0 | 733.3 |
| DYX-137X | 2418.0 | 3723.0 | 3179.0 |
| DYX-138X | 6648.0 | 4077.0 | > 10000 |
| DYX-101 | 594.7 | 570.7 | 500.0 |
| DYX-090 | 479.7 | 294.9 | 157.8 |
| DYX-114 | 410.7 | 174.7 | 155.1 |
| DYX-105 | 2457.0 | 2616.0 | 2272.0 |
| DYX-139 | 2358.0 | 1787.0 | 1267.0 |
| DYX-132 | 156.3 | 178.5 | 96.2 |
| DYX-176 | 627.4 | 2525.0 | 1037.0 |
| DYX-191 | 264.3 | 667.3 | 502.8 |
| DYX-193 | 136.2 | 315.8 | 83.0 |
| DYX-164 | 1930.0 | 2576.0 | 2243.0 |
| DYX-165 | 126.2 | 48.9 | 43.7 |
| DYX-174 | 1181.0 | 1912.0 | 623.1 |
| DYX-185 | 32.6 | 43.9 | 23.7 |
| DYX-187 | 172.1 | 624.6 | 277.7 |
| DYX-171 | 8039.0 | 2728.0 | 2643.0 |
| DYX-183 | > 10000 | > 10000 | > 10000 |
| DYX-180 | 497.6 | 693.7 | 414.6 |
| DYX-119 | > 10000 | > 10000 | > 10000 |
| DYX-149 | 3078.0 | 2464.0 | 3099.0 |
| DYX-150 | 65.24 | 163.5 | 120.5 |
| DYX-014 | 8311.0 | 4698.0 | 3029.0 |
| DYX-021 | 4933.0 | 3087.0 | 2601.0 |
| DYX-025 | 2329.0 | 2560.0 | 916.7 |
| DYX-027 | > 10000 | > 10000 | > 10000 |
| DYX-041 | 724.0 | 1989.0 | 795.7 |
| DYX-074 | 2499.0 | 2826.0 | 2197.0 |
| CCB-484 | 820.4 | 1970.0 | 477.3 |
| DYX-044 | > 10000 | > 10000 | > 10000 |
| DYX-051 | > 10000 | > 10000 | > 10000 |
| DYX-071 | 7726.0 | 2891.0 | 2745.0 |
| DYX-075 | 2483.0 | > 10000 | > 10000 |
| DYX-092 | 3060.0 | 2609.0 | 2766.0 |
| DYX-086 | 3597.0 | 2603.0 | 3713.0 |
| DYX-108 | 888.1 | 914.3 | 748.8 |
| DYX-129 | 9984.0 | 2798.0 | 3151.0 |

(continued)

| Cpd | CD74-NTRK1 | ETV6-NTRK2 | ETV6-NTRK3 |
|---|---|---|---|
| DYX-130 | 3815.0 | 9838.0 | 3086.0 |
| DYX-115 | > 10000 | > 10000 | > 10000 |
| DYX-131 | 2898.0 | 1611.0 | 2206.0 |
| DYX-134Q | > 10000 | 4620.0 | 2647.0 |
| DYX-138Q | > 10000 | > 10000 | > 10000 |
| DYX-135Q | > 10000 | > 10000 | > 10000 |
| DYX-136Q | 4598.0 | 3097.0 | 2094.0 |
| DYX-137Q | > 10000 | > 10000 | > 10000 |
| DYX-03 | 126.0 | 157.5 | 75.2 |
| DYX-215 | 146.5 | 170.3 | 109.6 |
| DYX-07 | 211.3 | 967.8 | 284.1 |
| DYX-10 | 630.0 | 776.5 | 393.9 |
| DYX-05 | 580.2 | 678.4 | 418.8 |
| DYX-06 | 640.1 | 723.0 | 492.1 |
| DYX-09 | 69.0 | 98.4 | 167.7 |
| DYX-04 | 173.8 | 728.5 | 240.0 |
| E01F02 | 18.9 | 133.3 | 40.7 |
| E01F07 | 35.76 | 441.1 | 92.9 |
| D01F08 | 112.2 | 399.8 | 158.0 |
| D02F0 1 | 204.1 | 361.7 | 150.9 |
| D05F01 | 202.7 | 462.2 | 366.5 |
| D06F01 | 564.4 | > 10000 | 851.0 |
| D07F01 | 2309.0 | 3024.0 | 2045.0 |
| D09F0 1 | 40.6 | 56.1 | 18.1 |
| DYX-216A | 12.6 | 8.7 | 7.2 |
| DYX-170 | 99.4 | 242.4 | 166.8 |
| DYX-223 | 37.7 | 24.3 | 35.6 |
| DYX-220 | 21.2 | 31.7 | 22.6 |
| DYX-222 | 37.3 | 54.7 | 41.5 |
| DYX-227 | 37.5 | 56.0 | 33.2 |
| DYX-228 | 11.9 | 20.5 | 11.6 |
| DYX-226 | 48.7 | 48.1 | 34.4 |
| DYX-231 | 619.2 | 779.5 | 547.3 |
| DYX-230 | 682.4 | 302.9 | 359.5 |
| DYX-235 | 20.0 | 32.6 | 34.2 |
| DYX-236 | 111.6 | 116.4 | 196.1 |
| DYX-244-1 | 9.8 | 20.3 | 6.9 |
| DYX-244-2 | 129.7 | 230.2 | 255.7 |

(continued)

| Cpd | CD74-NTRK1 | ETV6-NTRK2 | ETV6-NTRK3 |
|---|---|---|---|
| DYX-303 | 10.1 | 20.0 | 7.1 |
| DYX-305 | 28.2 | 51.3 | 22.9 |
| DYX-400 | 59.0 | 154.1 | 41.1 |
| DYX-401 | 113.2 | 447.5 | 111.8 |
| DYX-402 | 28.2 | 53.5 | 22.6 |
| DYX-403 | 52.6 | 137.0 | 180.0 |
| DYX-409 | 6.4 | 36.9 | 7.7 |
| DYX-410 | 10.3 | 11.7 | 3.5 |
| DYX-411 | 35.7 | 38.0 | 11.0 |
| DYX-412 | 109.9 | 115.0 | 35.6 |
| DYX-413 | 40.8 | 375.9 | 160.7 |
| DYX-414 | 195.7 | 2524.0 | 1177.0 |
| DYX-415 | 50.8 | 510.3 | 155.7 |
| DYX-416 | 21.0 | 88.7 | 34.3 |
| DYX-417 | 157.8 | 2528.0 | 578.7 |
| DYX-418 | 7.6 | 41.0 | 16.2 |
| DYX-419 | 2.4 | 11.9 | 4.0 |
| D01F11 | 12.9 | 159.5 | 25.6 |
| E01F08 | 43.8 | 219.9 | 82.9 |
| E01F10 | 68.9 | 610.0 | 156.2 |
| DYX-346 | 6.5 | 9.5 | 3.1 |
| DYX-349 | 7.4 | 10.7 | 4.3 |
| DYX-320 | 37.6 | 32.4 | 10.4 |
| DYX-318 | 9.6 | 33.6 | 9.1 |
| DYX-327 | 1.4 | 17.6 | 5.4 |
| DYX-314 | 154.1 | 165.3 | 148.6 |
| DYX-335 | 0.6 | 10.7 | 3.3 |
| DYX-332 | 9.9 | 146.0 | 46.5 |
| DYX-340 | 18.7 | 160.0 | 68.0 |
| CHW-1 | 35.5 | 43.1 | 39.2 |
| CHW-2 | 106.6 | 185.4 | 126.7 |
| CHW-3 | 92.0 | 165.3 | 122.8 |
| CHW-4 | 3.1 | 8.2 | 7.0 |
| CHW-5 | 27.3 | 70.1 | 59.5 |
| CHW-6 | 23.0 | 60.1 | 53.5 |
| CHW-7 | 33.4 | 57.8 | 40.9 |
| CHW-8 | 143.0 | 247.9 | 173.9 |
| CHW-9 | 60.8 | 134.4 | 120.6 |

(continued)

| Cpd | CD74-NTRK1 | ETV6-NTRK2 | ETV6-NTRK3 |
|---|---|---|---|
| CHW-10 | 274.1 | 439.3 | 389.6 |
| CHW-11 | 53.8 | 103.4 | 111.2 |
| CHW-12 | 33.8 | 55.8 | 48.1 |
| CHW-13 | 40.2 | 52.3 | 41.3 |
| CHW-14 | 331.4 | 427.1 | 350.4 |
| D5217 | 90.9 | 170.9 | 124.7 |
| D5218 | 10.6 | 19.1 | 14.1 |
| D5220 | 120.3 | 214.8 | 175.3 |
| D5221 | 144.5 | 195.9 | 128.4 |

It can be seen from the data in Table 2 that the indazole derivatives of the present disclosure have strong inhibitory activity on the cell proliferation of Ba/F3-TRKs stable strains..

Example 141: Rat hepatocyte stability experiment

In William medium E, dilute the 37 °C rat hepatocyte suspension to an appropriate density (viable cell density = 2 × $10^6$ cells/mL). In addition, a 2x quantification solution (the concentration of the test compound was 2 μM) was additionally prepared in William's medium E. The hepatocyte solution and 2x quantification solution were preheated in a water bath of 37 °C. 40 μL of the preheated hepatocyte solution (2 × 10 $^6$cells/ mL) was added into the designated wells for 5, 15, 30, 60, and 120 min, and add 40 μL of the preheated 2x quantification solution and start timing. An assay plate was placed in a 37°C, 5% $CO_2$ incubator with shaking at 110 rpm. At 5, 15, 30, 60, and 120 minutes, 240 μL of ACN containing IS was added to each well. The plate was then sealed and stored in a -35 °C freezer. After collecting at all time points, the plate was shaken for 2 minutes and then centrifuging was performed for 15 minutes at 6000 rmp. 100 μL of supernatant from each well was transferred to a 96-well sample plate containing 100 μL of water for LC/MS analysis, determination of compound concentrations, calculation of rate constants and half-lives. The results are shown in Table 3.

Table 3

| Compound | Genus | | Residual percentage (%) | | | | | | Half-life (min) |
|---|---|---|---|---|---|---|---|---|---|
| | | | 0 min | 5 min | 15 min | 30 min | 60 min | 120 min | |
| Testosterone | Rats | Average value | 100.00 | 21.21 | 5.78 | BQL | BQL | BQL | 2.23 |
| | | Relative standard deviation of area ratio | 0.05 | 0.13 | 0.10 | N/A | N/A | N/A | |
| DYX165 | Rats | Average value | 100.00 | 92.73 | 84.16 | 73.15 | 59.81 | 58.02 | 154.14 |
| | | Relative standard deviation of area ratio | 0.01 | 0.03 | 0.03 | 0.07 | 0.02 | 0.03 | |
| DYX244-1 | Rats | Average value | 100.00 | 71.83 | 48.63 | 14.84 | 8.20 | 5.40 | 11.12 |
| | | Relative standard deviation of area ratio | 0.01 | 0.08 | 0.05 | 0.02 | 0.17 | 0.34 | |

(continued)

| Compound | Genus | | Residual percentage (%) | | | | | | Half-life (min) |
|---|---|---|---|---|---|---|---|---|---|
| | | | 0 min | 5 min | 15 min | 30 min | 60 min | 120 min | |
| DYX318 | Rats | Average value | 100.00 | 27.83 | 12.24 | 1.36 | 0.27 | 0.20 | 2.71 |
| | | Relative standard deviation of area ratio | 0.03 | 0.03 | 0.00 | 0.10 | 0.10 | 0.37 | |

Compounds DYX165, DYX244-1 and DYX318 all showed strong inhibitory activity against TRKs kinase and cell proliferation of the Ba/F3-TRK stable strains. X in Compound DYX165 is sulfonamide group, which has better metabolic stability.

Example 142: Pharmacokinetic evaluation

Pharmacokinetics and oral bioavailability were tested in SD rats. According to the solubility of the drug, a single dose was administered orally, intravenously or by intraperitoneal injection.. Animal blood samples were collected at different time points (0, 0.5, 1, 2, 4, 6, 8, and 24 hours), anticoagulated with heparin, and centrifuged to take the supernatant. Blood samples were analyzed by HPLC-MS. DAS2.1 was used for data analysis to detect half-life ($T_{1/2}$), maximum blood concentration ($C_{max}$), time to peak ($T_{max}$), area under the drug-time curve (AUC), and bioavailability (BA) and other pharmacokinetic data. The results are shown in Table 4.

Table 4

| Compound | DYX244-1 | | DYX165 |
|---|---|---|---|
| Administration mode | Intravenous injection | Oral administration | Oral administration |
| Dose (mg/kg) | 2 | 10 | 25 |
| Half-life (h) | 1.39 | 1.13 | 1.54 |
| Time to Peak (h) | 0.08 | 2.00 | 4.00 |
| Maximum blood concentration (μM) | 6.77 | 0.36 | 91.56 |
| Area under the drug-time curve (0-t) (μM·h) | 3.68 | 1.62 | 1035.70 |
| Area under the drug-time curve (0-∞) (μM·h) | 3.69 | 1.64 | 1035.78 |
| Removal rate (L/h/kg) | 1.44 | nd | nd |
| Bioavailability (%) | 8.79 | | |

Compounds DYX165 and DYX244-1 have strong inhibitory activity against TRKs kinase and cell proliferation of the Ba/F3-TRK stable strains. X in compound DYX165 is sulfonamido, which has better oral absorption properties. At an oral dose of 25 mg/kg in rats, the highest blood concentration of compound DYX165 was up to 91.56 μM , and the area under the drug-time curve was up to 1035.70 μM·h. The results indicate that when X in the compound of the formula (I) of the present disclosure is sulfonamido, it has better pharmacokinetic properties.

The technical features of the above-described examples can be combined arbitrarily. For the sake of brevity, not all possible combinations of the technical features of the above-described examples are described. However, as long as there is no contradiction between the combinations of these technical features, they should be considered to fall within the scope of the present description.

The above-described examples only express several embodiments of the present disclosure, and their descriptions are specific and detailed, but should not to be construedas limitation of the patent scope of the present invention. It should be noted that for a person of ordinary skill in the art, several modifications and improvements can be made without departing from the concept of the present disclosure, which all belong to the protection scope of the present invention. Therefore, the protection scope of the present invention shall be subject to the appended claims.

## Claims

1. Indazole derivatives or their pharmaceutically acceptable salts or stereoisomers having the structure shown in formula (I):

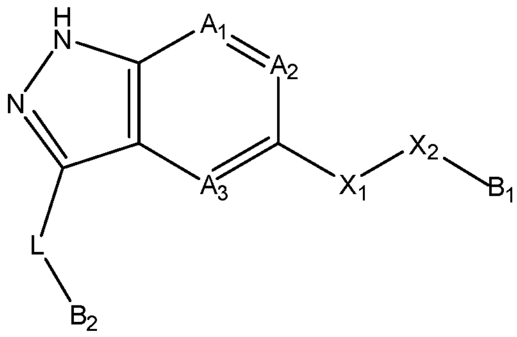

(I)

wherein,

0-2 of $A_1$, $A_2$, and $A_3$ are selected from N, and the rest are CH;
$X_1$ is selected from: $-(CR_1R_2)_n-$, -O-, -S-, -S(O)-, $-S(O_2)-$, -C(O)-, $-N(R_3)-$, -CH=CH-, -C=C-, $-C(O)N(R_3)-$, or none;
$X_2$ is selected from: $-(CR_1R_2)n-$, -O-, -S-, -S(O)-, $-S(O_2)-$, -C(O)-, $-N(R_3)-$, -CH=CH-, -C=C-, $-C(O)N(R_3)-$, or none;
alternatively, $X_1$ and $X_2$ together form one or more $R_1$ substituted or unsubstituted $C_3$-$C_8$ cycloalkyl, or one or more $R_1$ substituted or unsubstituted $C_3$-$C_8$ heterocyclyl;
$B_1$ is selected from: one or more $R_4$ substituted or unsubstituted $C_6$-$C_{10}$ aryl, and one or more $R_4$ substituted or unsubstituted $C_5$-$C_{10}$ heteroaryl;
L is selected from: -CH=CH-, -C=C-;
$B_2$ is selected from: one or more $R_5$ substituted or unsubstituted $C_6$-$C_{10}$ aryl, one or more $R_5$ substituted or unsubstituted $C_5$-$C_{10}$ heteroaryl, $-C(O)N(R_6R_7)$;
$R_1$ and $R_2$ are each independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthiol, $C_1$-$C_6$ alkoxy $C_1$-$C_6$ alkyl;
when either $X_1$ or $X_2$ is selected from -O-, -S-, and the other is selected from $-(CR_1R_2)n-$, both $R_1$ and $R_2$ are hydrogen;
each $R_3$ is independently selected from: hydrogen, $C_1$-$C_6$ alkyl;
each $R_4$ is independently selected from: hydrogen, $C_1$-$C_6$ alkyl, cyano, halogen, halogenated $C_1$-$C_6$ alkyl, hydroxyl, nitro, amino, mercapto, $C_1$-$C_6$ alkoxy, halogenated $C_1$-$C_6$ alkoxy, $C_1$-$C_6$ alkylamino, $C_1$-$C_6$ alkylamido, $C_1$-$C_6$ alkylthio, $C_1$-$C_6$ alkoxy $C_1$-$C_6$ alkyl, $C_1$-$C_6$ alkylsulfonyl, and $C_1$-$C_6$ alkylsulfonamido;
each $R_5$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$;
$R_6$ and $R_7$ are each independently selected from: hydrogen and $C_1$-$C_{10}$ alkyl, or $R_6$ and $R_7$ together with a nitrogen atom which they are attached to, form one or more $R_8$ substituted or unsubstituted 3-8 membered heterocyclyl;
each $R_8$ is independently selected from: hydrogen, $C_1$-$C_6$ alkyl, cyano, halogen, halogenated $C_1$-$C_6$ alkyl, hydroxyl, amino, mercapto, $C_1$-$C_6$ alkoxy, $C_1$-$C_6$ alkylamino, $C_1$-$C_6$ alkylthio, $C_1$-$C_6$ alkoxy $C_1$-$C_6$ alkyl, hydroxyl-substituted $C_1$-$C_6$ alkyl, cyano-substituted $C_1$-$C_6$ alkyl, $-C(O)-C(R_3)_3$, cyclopropyl, $N(R_3)_2C(O)-(C(R_3)_2)_n-$; and
n is selected from: an integer between 1-8.

2. The indazole derivatives or their pharmaceutically acceptable salts or stereoisomers according to claim 1, wherein both $A_1$ and $A_2$ are CH, and $A_3$ is N or CH.

3. The indazole derivatives or their pharmaceutically acceptable salts or stereoisomers according to claim 1, wherein $A_1$ is N or CH, and both $A_2$ and $A_3$ are CH.

4. The indazole derivatives or their pharmaceutically acceptable salts or stereoisomers according to claim 1, wherein $X_1$-$X_2$ formed by $X_1$ and $X_2$ together is selected from: $-(CR_1R_2)_n-$, -O-, -S-, -S(O)-, $-S(O_2)-$, -C(O)-, $-N(C_1\text{-}C_6\ alkyl)-$,

$-(CH_2)_n-O-$, $-O-(CH_2)_n-$, $-(CR_1R_2)_n-N(R_3)-$, $-N(R_3)-(CR_1R_2)_n-$, $-(CH_2)_n-S-$, $-S-(CH_2)n-$, $-S(O_2)N(R_3)-$, $-N(R_3)S(O_2)-$, $-N(R_3)C(O)N(R_3)-$, one or more $R_1$ substituted or unsubstituted $C_3$-$C_8$ cycloalkyl, and one or more $R_1$ substituted or unsubstituted $C_3$-$C_8$ heterocycloalkyl;

$R_1$ and $R_2$ are each independently selected from: hydrogen, $C_1$-$C_6$ alkyl;
each $R_3$ is independently selected from: H, $C_1$-$C_6$ alkyl; and
n is selected from an integer between 1-4.

**5.** The indazole derivatives or their pharmaceutically acceptable salts or stereoisomers according to claim 4, wherein $X_1$-$X_2$ formed by $X_1$ and $X_2$ together is selected from: $-(CR_1R_2)n-$, -O-, -S-, $-S(O_2)-$, $-N(C_1$-$C_3$ alkyl$)-$, $-(CH_2)_n-O-$, $-O-(CH_2)_n-$, $-(CR_1R_2)_n-N(R_3)-$, $-NR_3-(CR_1R_2)_n-$, $-S(O_2)N(R_3)-$, $-N(R_3)S(O_2)$, and $C_5$-$C_6$ heterocycloalkyl;

$R_1$ and $R_2$ are each independently selected from: hydrogen, $C_1$-$C_3$ alkyl;
each $R_3$ is independently selected from: H, $C_1$-$C_3$ alkyl; and
n is selected from: an integer between 1-2.

**6.** The indazole derivatives or their pharmaceutically acceptable salts or stereoisomers according to claim 5, wherein $X_1$-$X_2$ formed by $X_1$ and $X_2$ together is selected from: $-CH_2-$, -O-, -S-, $-S(O_2)-$, $-OCH_2-$, $-N(CH_3)-$, $-CH_2O-$, $-CH_2NH-$, $-CH_2N(CH_3)-$, $-NH-S(O_2)-$, $-S(O_2)-NH-$, $-NHCH_2-$, $-NHCH(CH_3)-$, $-CH(CH_3)NH-$, $-N(CH_3)CH_2-$, $-CH_2CH_2-$,

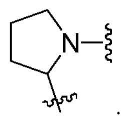

.

**7.** The indazole derivatives or their pharmaceutically acceptable salts or stereoisomers according to claim 6, wherein $X_1$-$X_2$ formed by $X_1$ and $X_2$ together is selected from: $-S(O_2)-$, $-CH_2CH_2-$; or $X_1$ is -O- and $X_2$ is $CH_2-$.

**8.** The indazole derivatives or their pharmaceutically acceptable salts or stereoisomers according to claim 6, wherein $X_1$ is -NH- and $X_2$ is selected from: $-S(O_2)-$, $-CH_2-$, $-CH(CH_3)-$.

**9.** The indazole derivatives or their pharmaceutically acceptable salts or stereoisomers according to claim 1, having a structure shown in formula (II) or formula (III):

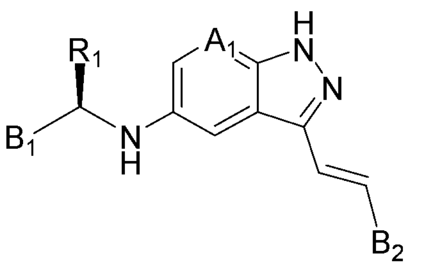

(II)

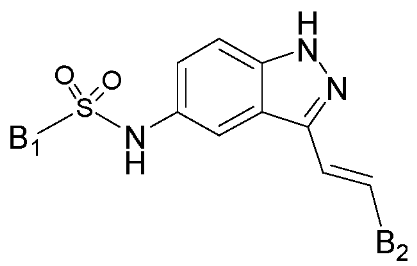

(III).

**10.** The indazole derivatives or their pharmaceutically acceptable salts or stereoisomers according to claim 1, wherein $B_1$ is selected from: one or more $R_4$ substituted or unsubstituted phenyl, one or more $R_4$ substituted or unsubstituted pyridinyl, one or more $R_4$ substituted or unsubstituted pyrimidinyl, one or more $R_4$ substituted or unsubstituted naphthyl, one or more $R_4$ substituted or unsubstituted quinolinyl, one or more $R_4$ substituted or unsubstituted pyrazolyl, one or more $R_4$ substituted or unsubstituted pyrrolyl, one or more $R_4$ substituted or unsubstituted thienyl, one or more $R_4$ substituted or unsubstituted furanyl, one or more $R_4$ substituted or unsubstituted pyrazinyl.

**11.** The indazole derivatives or their pharmaceutically acceptable salts or stereoisomers according to claim 1, wherein each $R_4$ is independently selected from: hydrogen, $C_1$-$C_3$ alkyl, halogen, halogenated $C_1$-$C_3$ alkyl, cyano, $C_1$-$C_3$ alkoxy, $C_1$-$C_4$ alkyl sulfonyl, $C_1$-$C_3$ alkylsulfonamido.

**12.** The indazole derivatives or their pharmaceutically acceptable salts or stereoisomers according to claim 1, wherein each $R_4$ is independently selected from: hydrogen, halogen, nitro, amino, hydroxyl, mercapto, trifluoromethyl, cyano, formamido, methylamino, trifluoromethoxy, difluoromethoxy, difluoromethyl, methyl, ethyl, n-propyl, isopropyl, n-butyl, isobutyl, tert-butyl, n-pentyl, isopentyl, neopentyl, methoxy, ethoxy, isopropoxy, tert-butoxy, methoxyethyl, ethoxyethyl, methylthio, ethylthio, isopropylthio, tert-butylthio, methylsulfonyl, ethylsulfonyl, isopropylsulfonyl, tert-butyl sulfonyl, methylsulfonamido.

**13.** The indazole derivatives or their pharmaceutically acceptable salts or stereoisomers according to any one of claims 1-12, wherein $B_1$ is selected from:

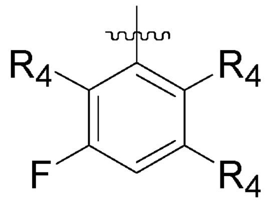 , 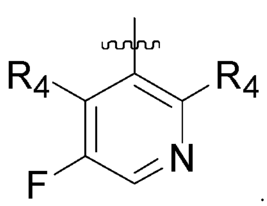 .

**14.** The indazole derivatives or their pharmaceutically acceptable salts or stereoisomers according to claim 13, wherein $B_1$ is selected from:

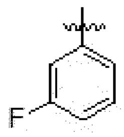 , 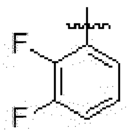 , 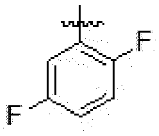 , 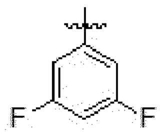 , 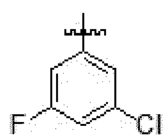 ,

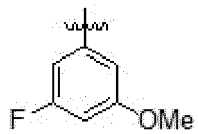 , 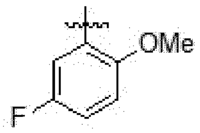 , 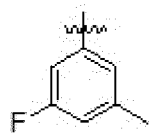 , 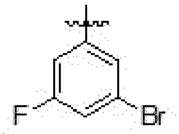 , 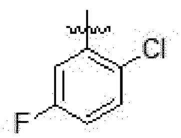 ,

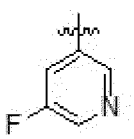 , 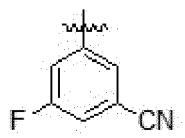 , 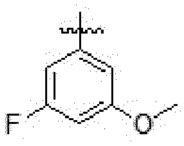 , 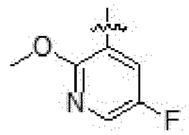 , 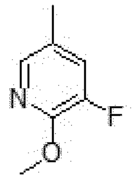 ,

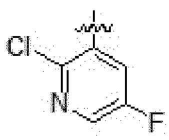 , 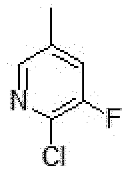

**15.** The indazole derivatives or their pharmaceutically acceptable salts or stereoisomers according to claim 1, wherein $B_2$ is selected from: one or more $R_5$ substituted or unsubstituted phenyl, one or more $R_5$ substituted or unsubstituted naphthyl, one or more $R_5$ substituted or unsubstituted quinolinyl, one or more $R_5$ substituted or unsubstituted pyrazolyl, one or more $R_5$ substituted or unsubstituted pyrrolyl, one or more $R_5$ substituted or unsubstituted thienyl, one or more $R_5$ substituted or unsubstituted furanyl, one or more $R_5$ substituted or unsubstituted pyridyl, one or more $R_5$ substituted or unsubstituted pyrimidinyl, one or more $R_5$ substituted or unsubstituted pyrazinyl, one or more $R_5$ substituted or unsubstituted triazolyl, $-C(O)NR_6R_7$.

**16.** The indazole derivatives or theirs pharmaceutically acceptable salts or stereoisomers according to claim 15, wherein $B_2$ is selected from:

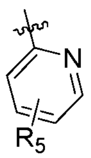, 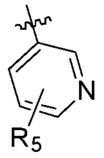, 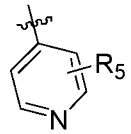, 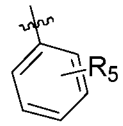, 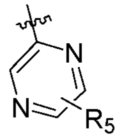, 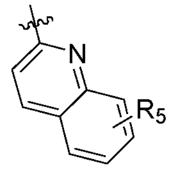, 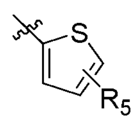, 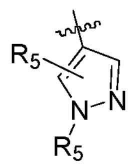,

$-C(O)NR_6R_7$.

**17.** The indazole derivatives or their pharmaceutically acceptable salts or stereoisomers according to claim 16, wherein $B_2$ is selected from:

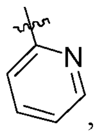, 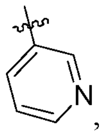, 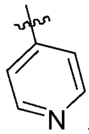, 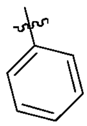, 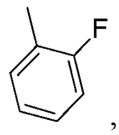,

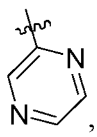, 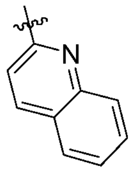, 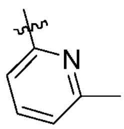, 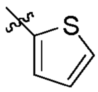, 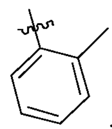, 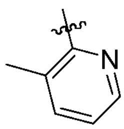, 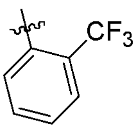, 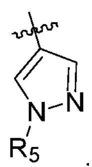;

, $-C(O)NR_6R_7$.

**18.** The indazole derivatives or their pharmaceutically acceptable salts or stereoisomers according to any one of claims 1-12 or claims 15-17, wherein each $R_5$ is independently selected from: hydrogen, $C_1$-$C_3$ alkyl, halogen, halogenated $C_1$-$C_3$ alkyl, cyano, $C_1$-$C_3$ alkoxy, hydroxyl-substituted $C_1$-$C_3$ alkyl, cyano-substituted $C_1$-$C_3$ alkyl, $C_1$-$C_3$ alkoxy $C_1$-$C_3$ alkyl, $-C(O)-C(R_3)_3$, cyclopropyl, $N(R_3)_2C(O)-(C(R_3)_2)_n-$, wherein each $R_3$ is independently selected from: H, $C_1$-$C_3$ alkyl.

**19.** The indazole derivatives or their pharmaceutically acceptable salts or stereoisomers according to claim 18, wherein each $R_5$ is independently selected from: hydrogen, hydroxyl-substituted $C_1$-$C_3$ alkyl, $C_1$-$C_3$ alkyl, cyano-substituted $C_1$-$C_3$ alkyl, cyclopropyl, $C_1$-$C_3$ alkoxy $C_1$-$C_3$ alkyl.

**20.** The indazole derivatives or their pharmaceutically acceptable salts or stereoisomers according to any one of claims 1-12 or claims 15-17, wherein each $R_5$ is independently selected from: hydrogen, hydroxyl, amino, mercapto, halogen, trifluoromethyl, cyano, trifluoromethoxy, difluoromethoxy, methyl, ethyl, n-propyl, isopropyl, n-butyl, isobutyl, tert-butyl, n-pentyl, isopentyl, neopentyl, methoxy, ethoxy, isopropoxy, tert-butoxy, methylthio, ethylthio, isopropylthio, tert-butylthio, methoxyethyl, ethoxyethyl, hydroxylmethyl, hydroxylethyl, hydroxylpropyl, cyano-substituted methyl, cyano-substituted ethyl, acetyl, 2,2,2-trifluoroethyl, cyclopropyl, 2,2-difluoroethyl, N,N-dimethylacetamido, difluoromethyl, isopropoxy-substituted ethyl, N-methylacetamido.

**21.** The indazole derivatives or their pharmaceutically acceptable salts or stereoisomers according to any one of claims 1-12 or claims 15-17, wherein $R_6$ and $R_7$ are each independently selected from: hydrogen, $C_1$-$C_6$ alkyl, or $R_6$ and $R_7$ together with the nitrogen atom to which they are attached form one or more $R_8$ substituted or unsubstituted 4-8 membered heterocyclyl.

**22.** The indazole derivatives or their pharmaceutically acceptable salts or stereoisomers according to claim 21, wherein $R_6$ and $R_7$ are each independently selected from: hydrogen, $C_1$-$C_3$ alkyl, or $R_6$ and $R_7$ together with the nitrogen atom to which they are attached form a heterocyclyl having the following structure:

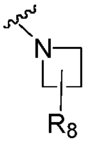 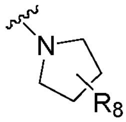 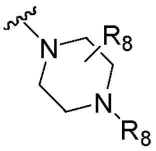 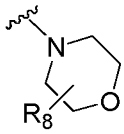 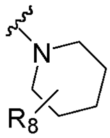 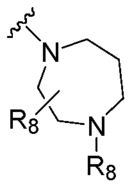 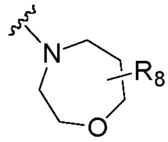 ,

wherein each $R_8$ is independently selected from: hydrogen, hydroxyl, $C_1$-$C_3$ alkyl, halogen, halogenated $C_1$-$C_3$ alkyl, $C_1$-$C_3$ alkoxy.

**23.** The indazole derivatives or their pharmaceutically acceptable salts or stereoisomers according to claim 22, wherein $R_6$ and $R_7$ are each independently selected from: hydrogen and methyl, or $R_6$ and $R_7$ together with the nitrogen atom to which they are attached form a heterocyclyl having the following structure:

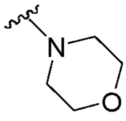 , 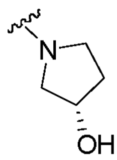 , 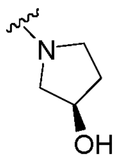 , 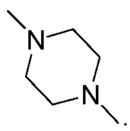 .

**24.** The indazole derivatives or their pharmaceutically acceptable salts or stereoisomers according to claim 1, having a structure shown in formula (II):

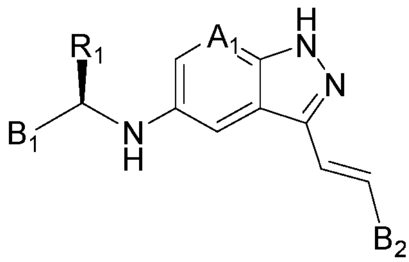

(II)

wherein $A_1$ is selected from: CH, N;

$R_1$ is selected from: H, methyl;

$B_1$ is selected from:

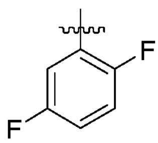 , 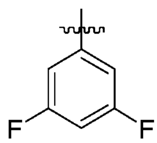 , 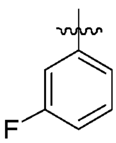 , 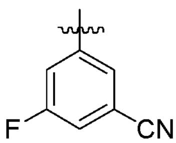 , 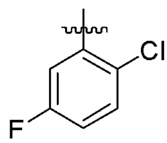 ,

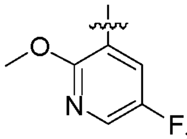 , 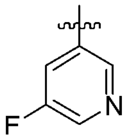 ;

$B_2$ is selected from:

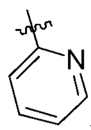, 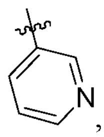, 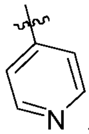, 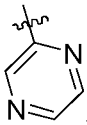, 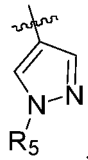,

$-C(O)NR_6R_7$;

$R_5$ is selected from: hydrogen, methyl, ethyl, and hydroxylethyl; and

$R_6$ and $R_7$ are each independently selected from: hydrogen and methyl, or $R_6$ and $R_7$ together with the nitrogen atom to which they are attached form a heterocyclyl having the following structure:

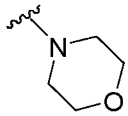, 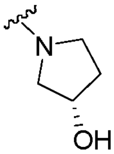, 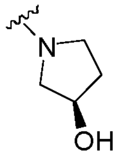, 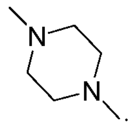.

**25.** The indazole derivatives or their pharmaceutically acceptable salts or stereoisomers according to claim 1, having a structure shown in formula (III):

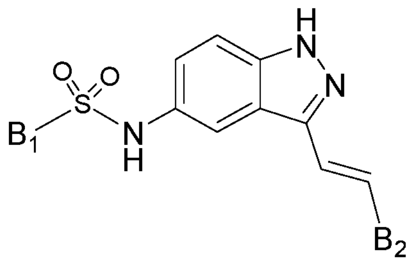

(III)

wherein $B_1$ is selected from:

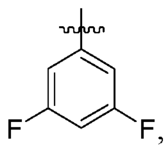, 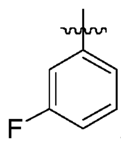, 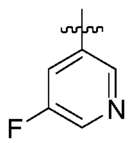;

$B_2$ is selected from:

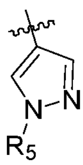, 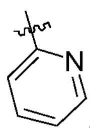, 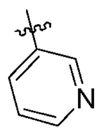;

and

$R_5$ is selected from: hydrogen, cyano-substituted methyl, acetyl, 2,2,2-trifluoroethyl, cyclopropyl, 2,2-difluoroethyl, isopropyl, difluoromethyl, isopropoxyethyl, methoxyethyl, hydroxylethyl, methyl, ethyl.

**26.** The indazole derivatives or their pharmaceutically acceptable salts or stereoisomers according to claim 1, is selected from the following compounds:

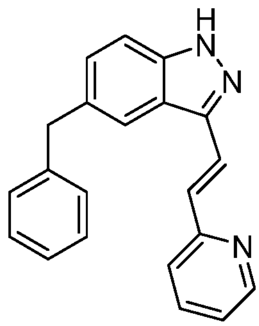
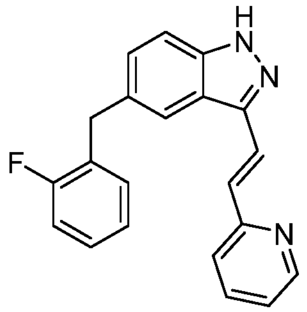
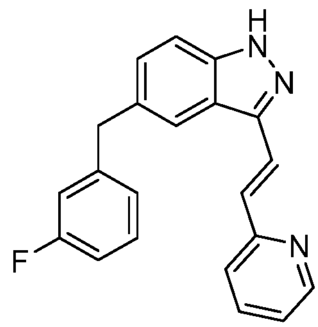

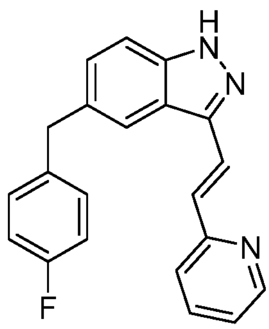
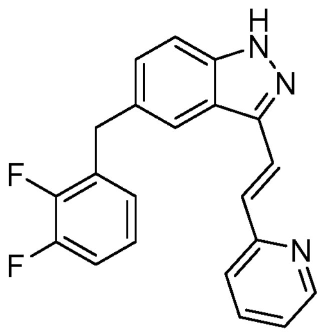
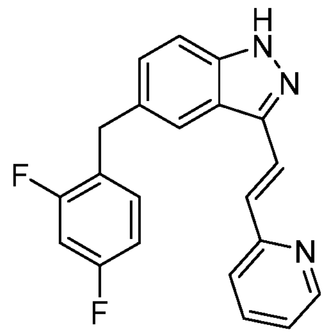

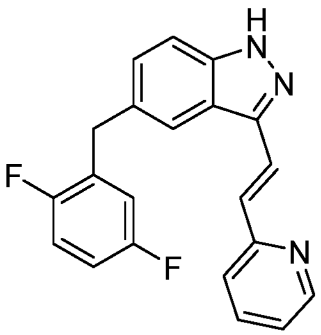
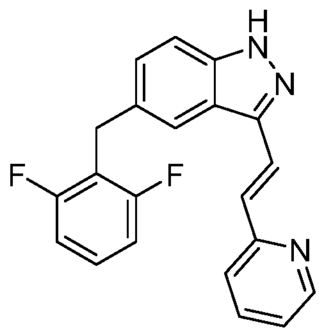
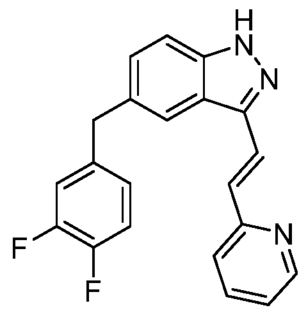

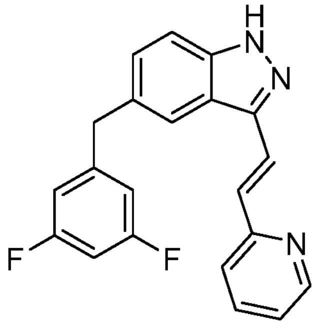
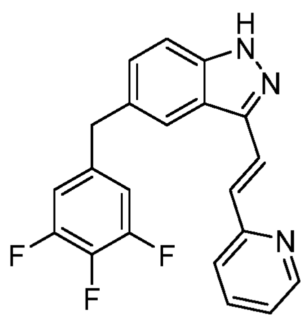
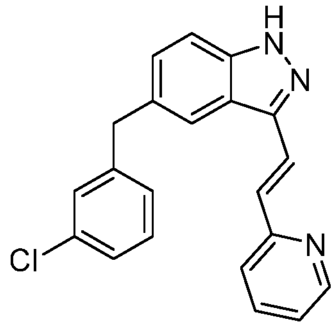

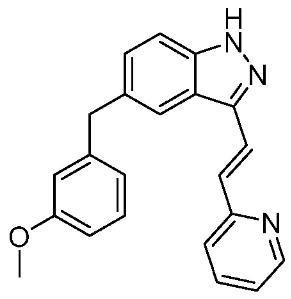
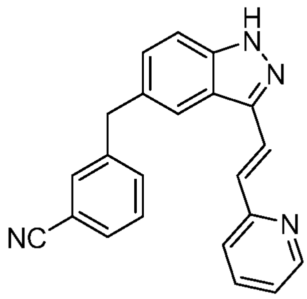
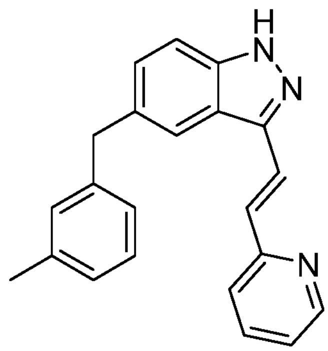

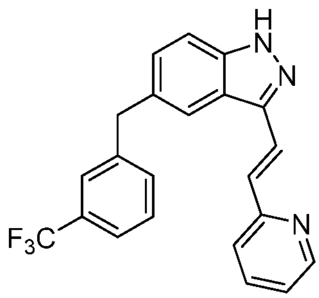
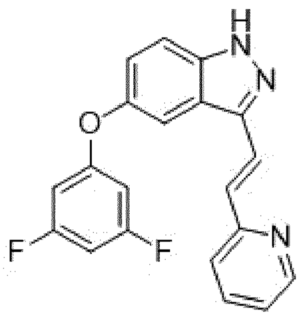
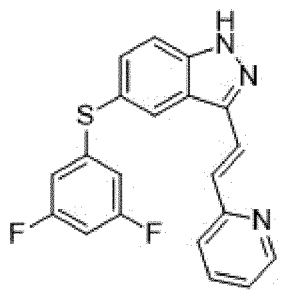

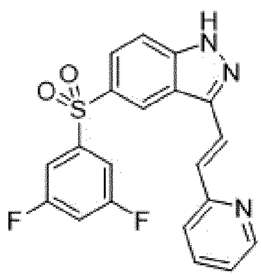
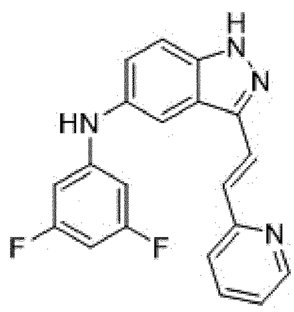
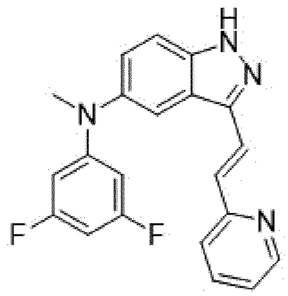

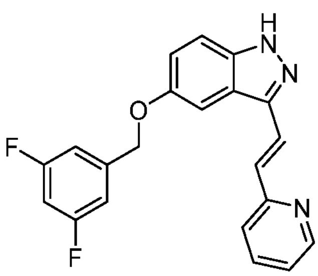
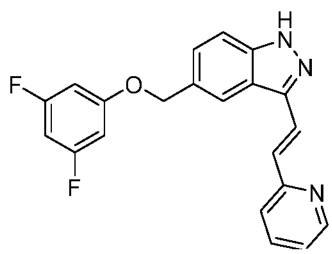
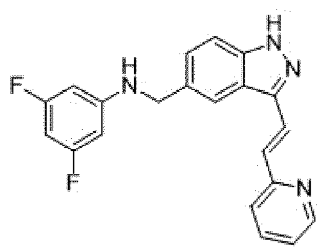

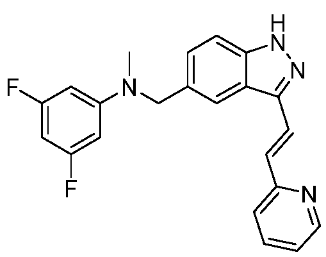
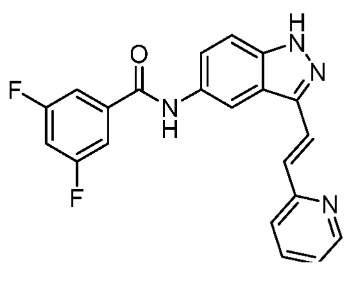
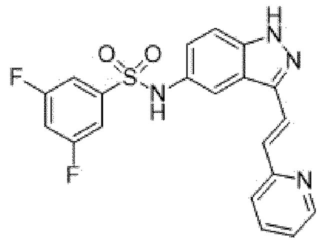

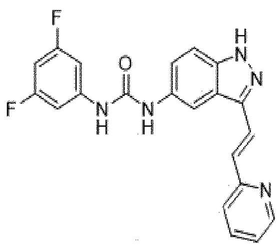

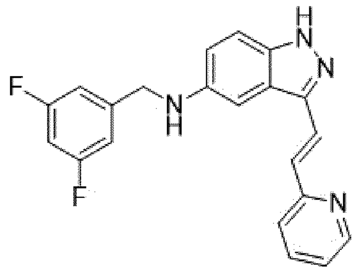

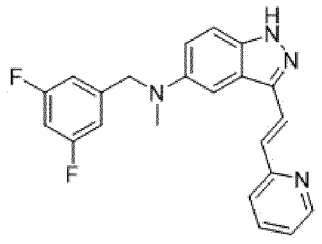

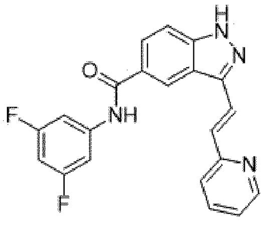

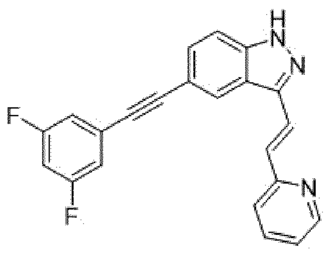

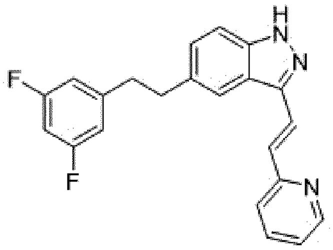

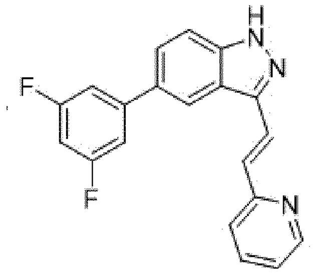

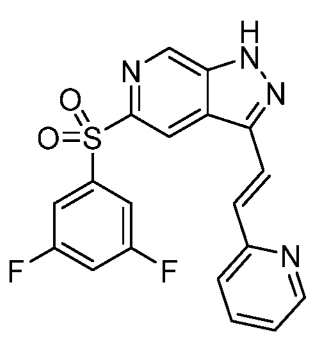

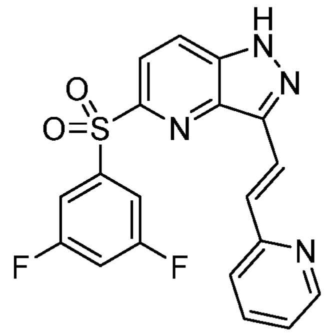

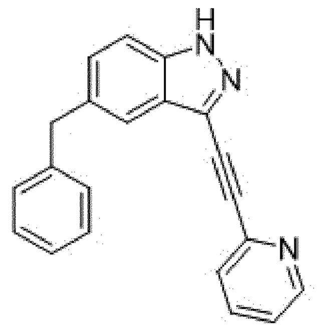

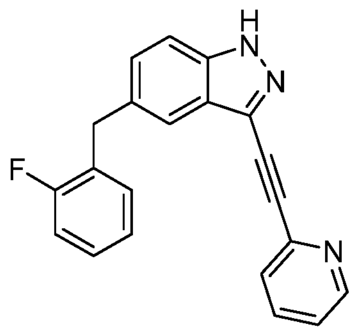

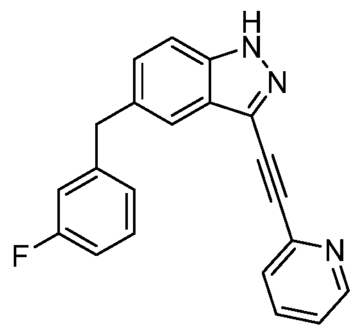

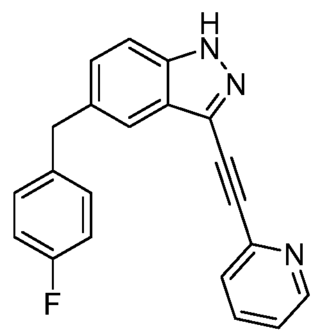

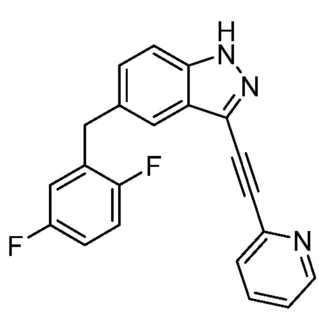

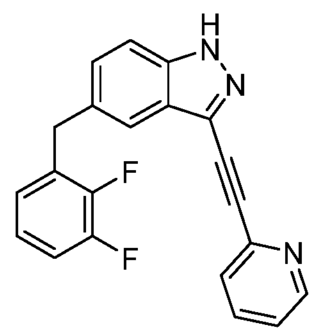

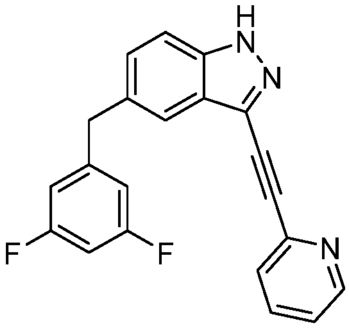
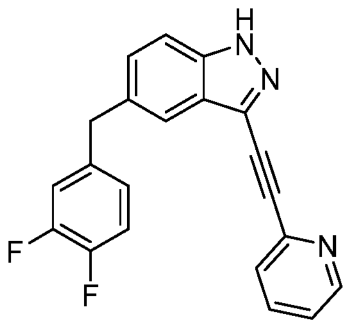
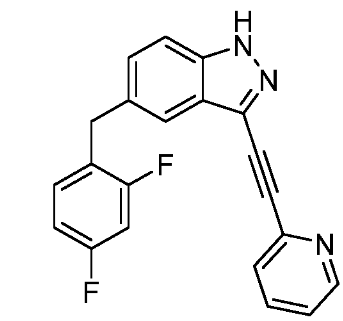

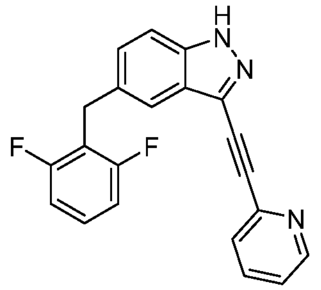
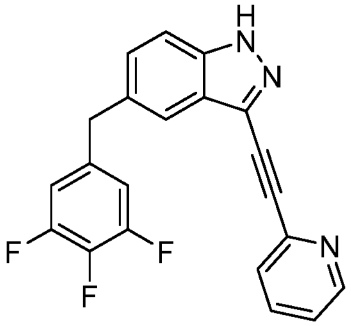
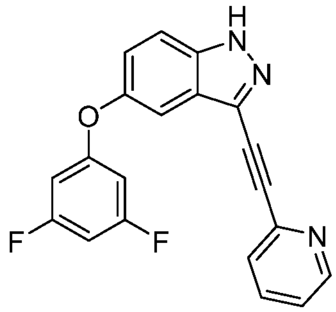

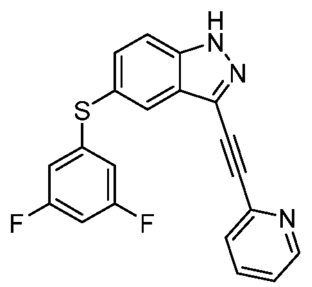
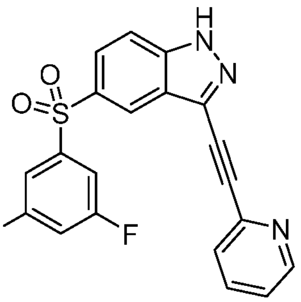
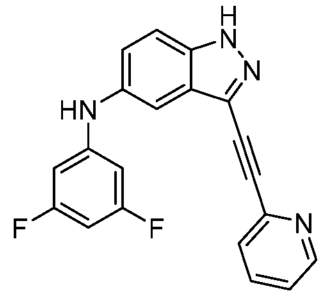

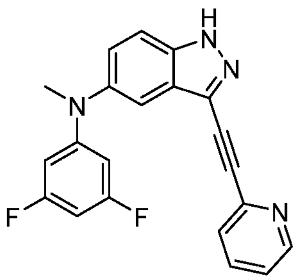
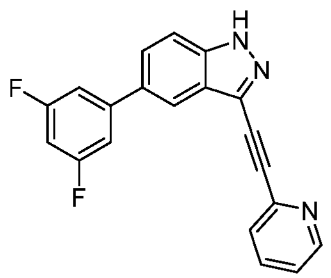
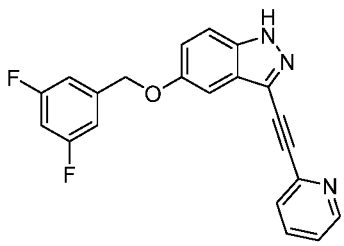

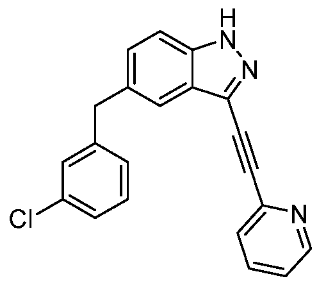
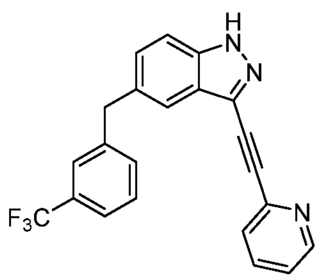
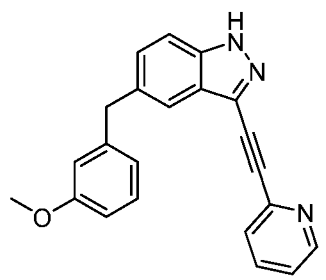

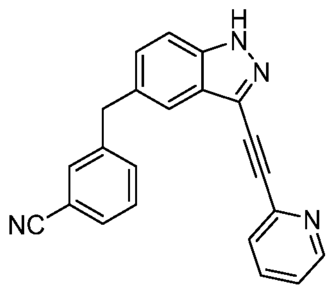
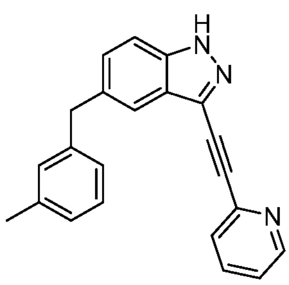
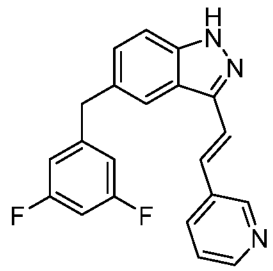

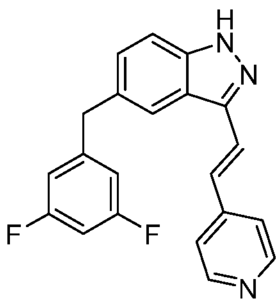
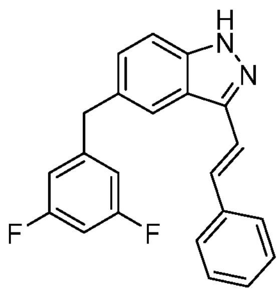
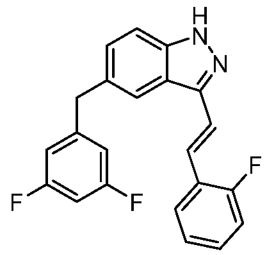

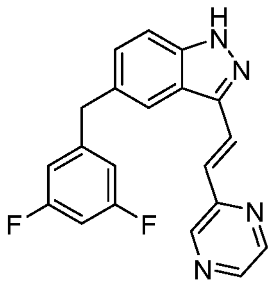
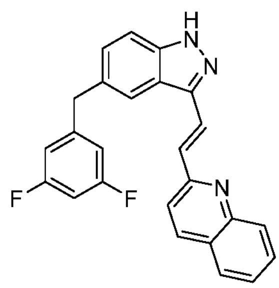
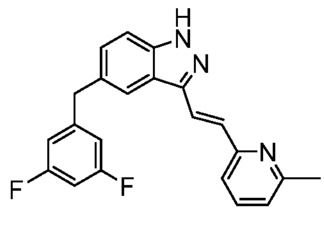

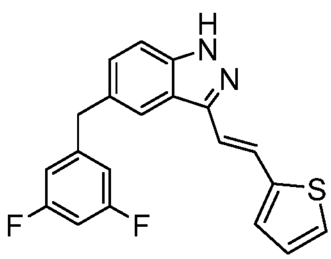
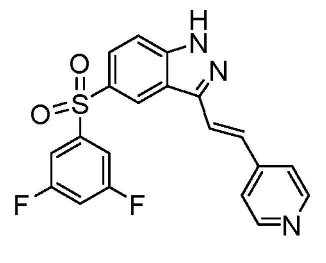
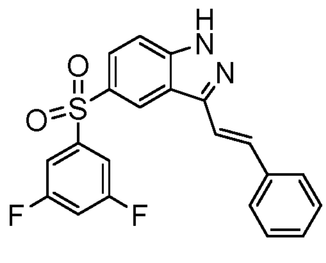

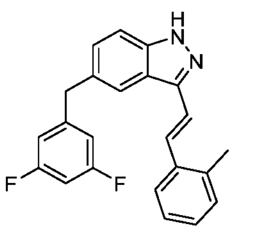
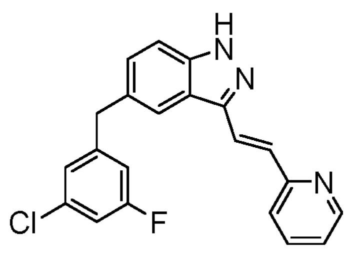
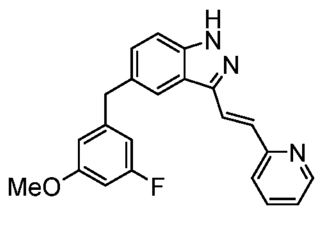

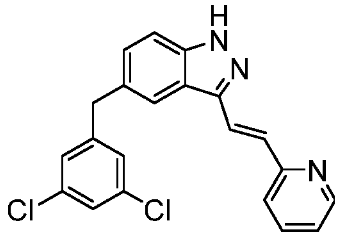
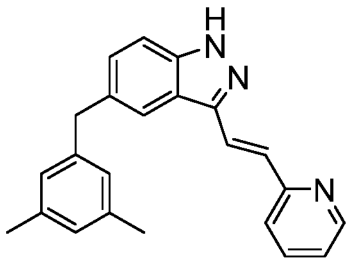
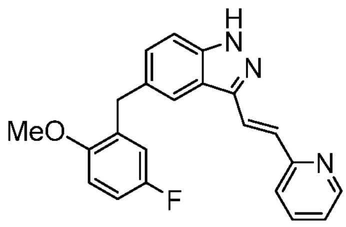

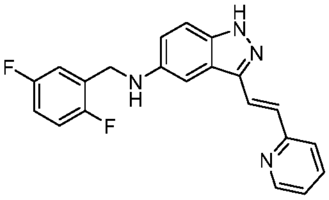
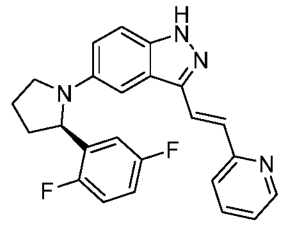
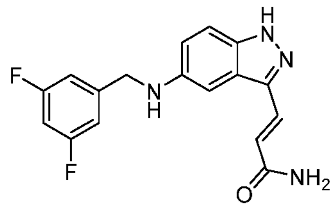

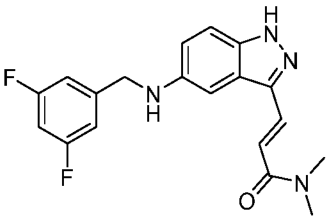
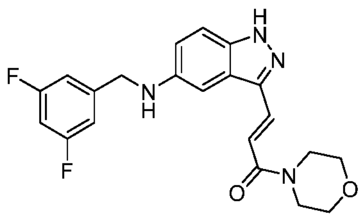
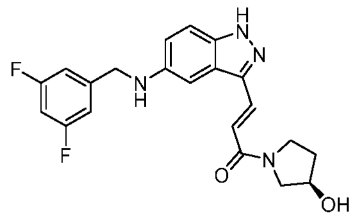

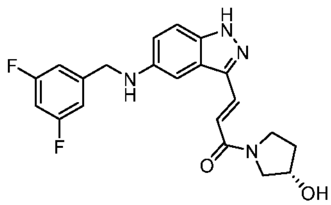
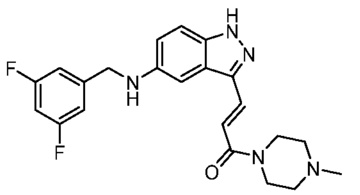
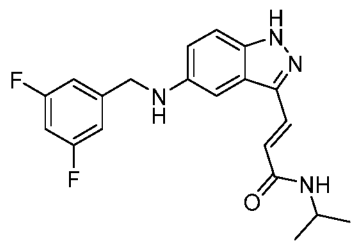

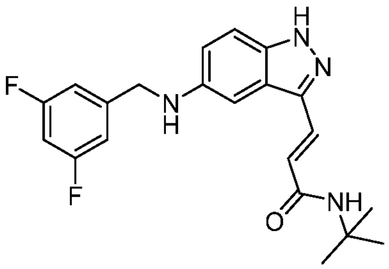
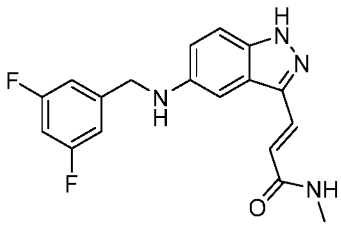
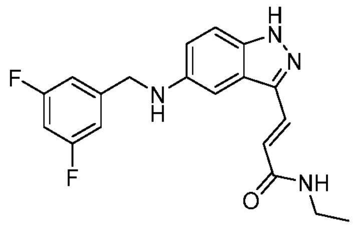

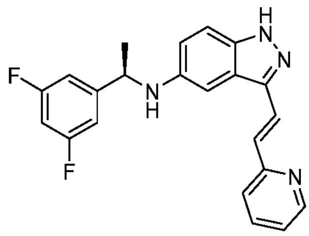
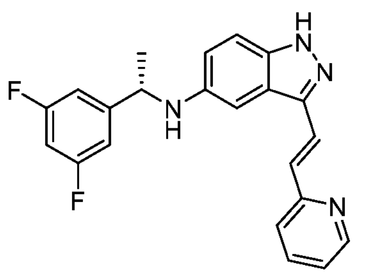
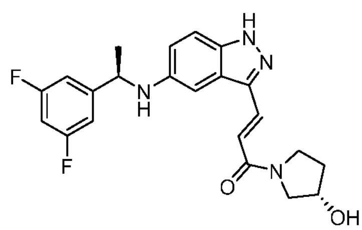

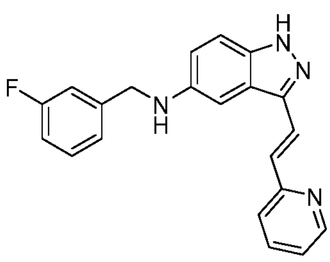
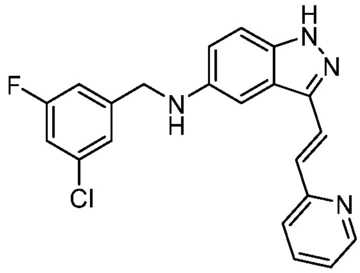
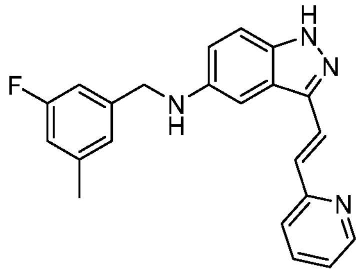

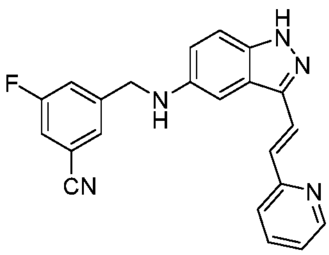
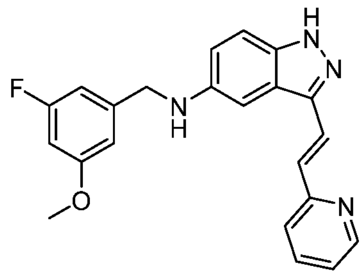
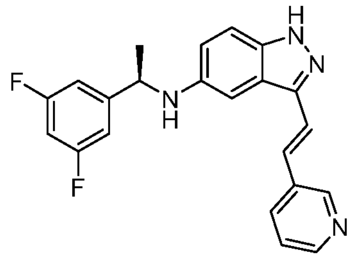

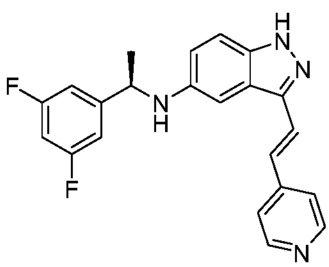
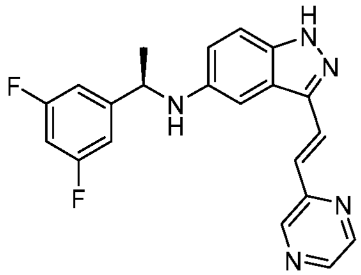
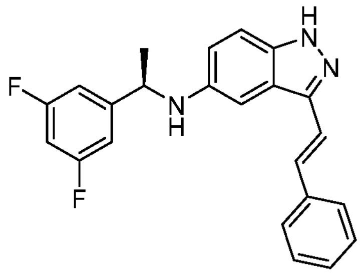

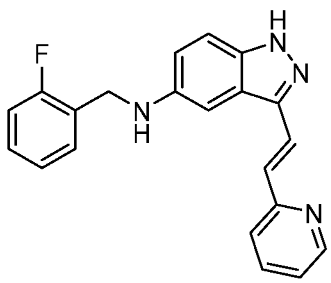
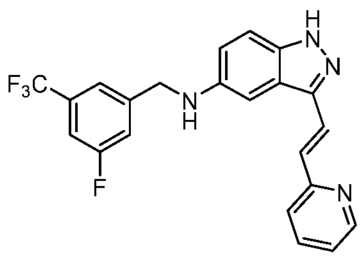
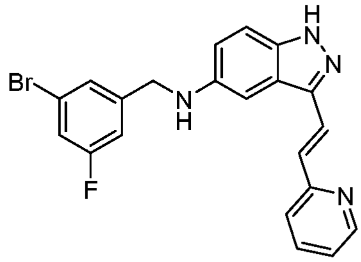

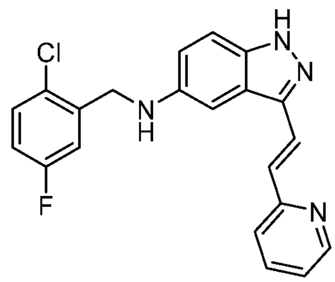
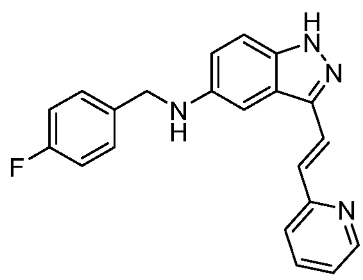
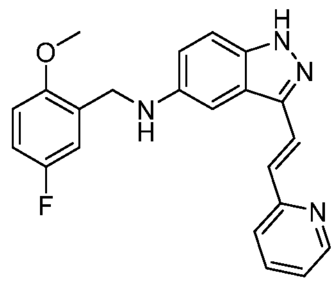

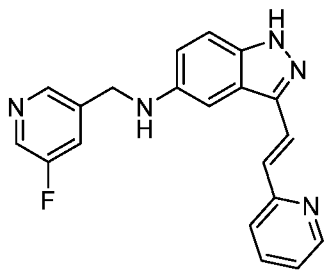
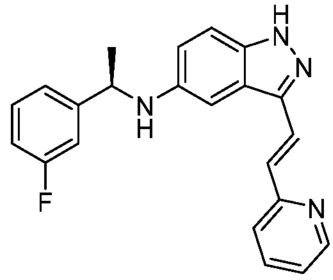
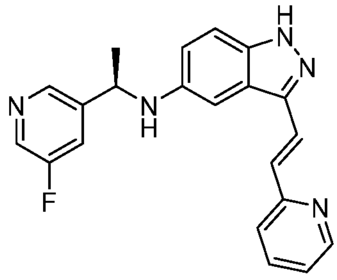

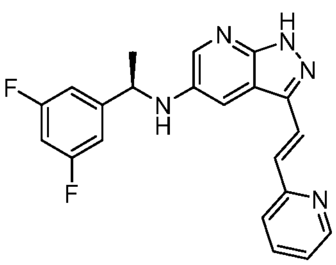
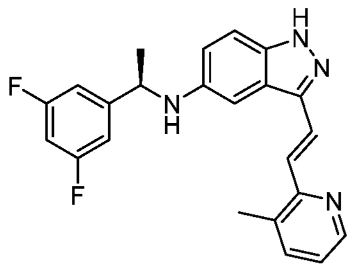
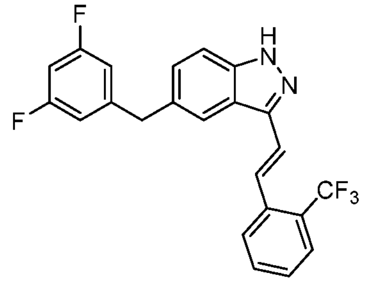

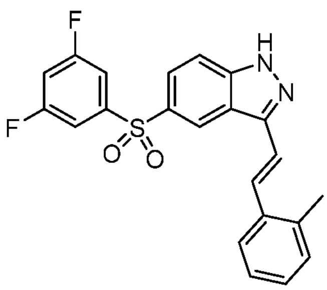
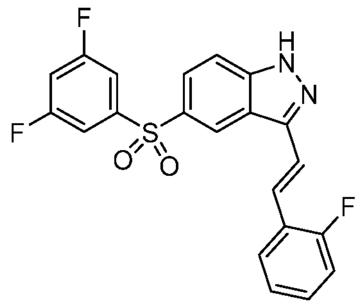
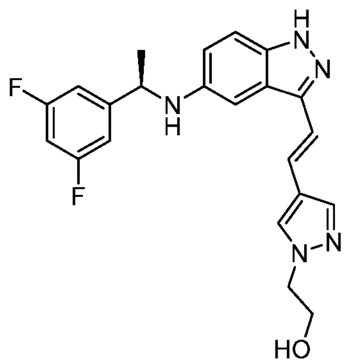

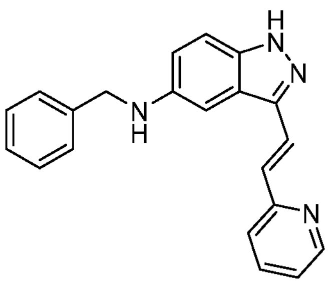
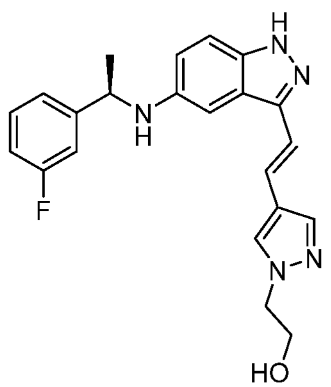
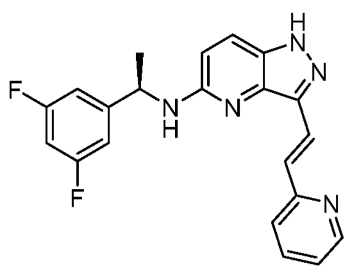

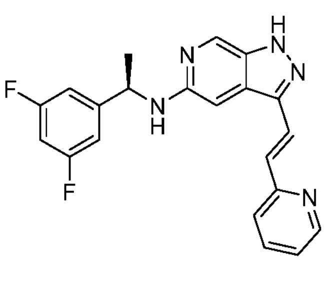
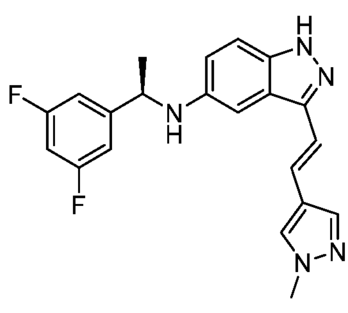
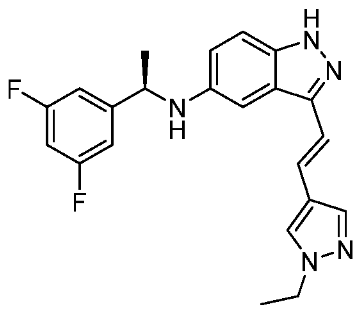

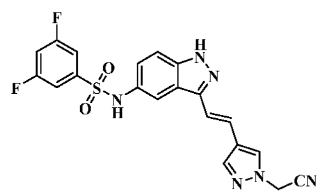
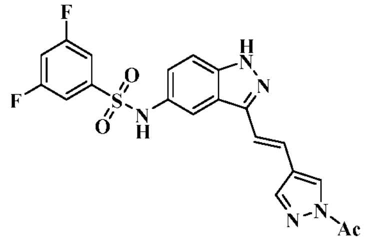
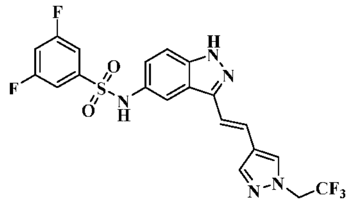
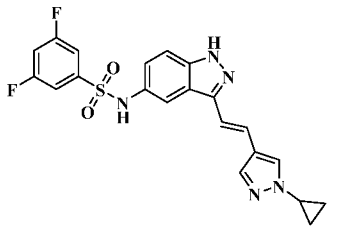
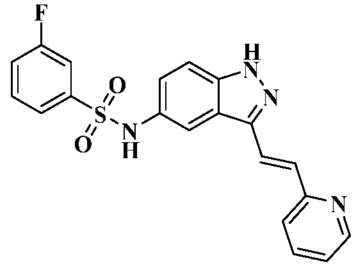
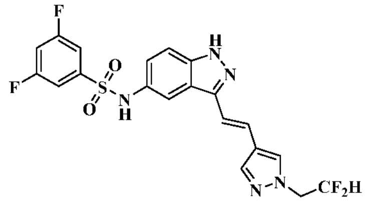
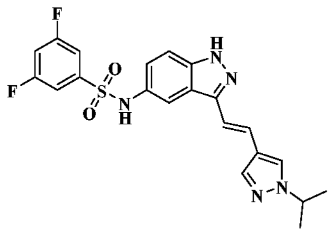
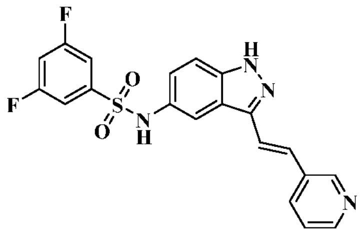
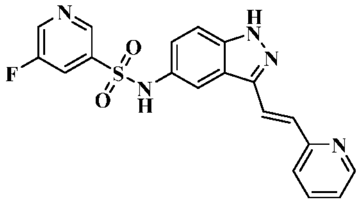
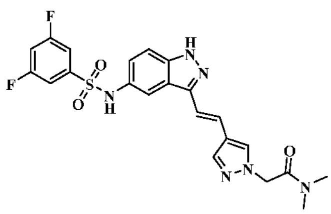
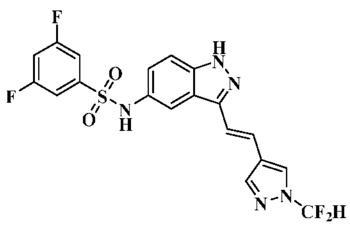
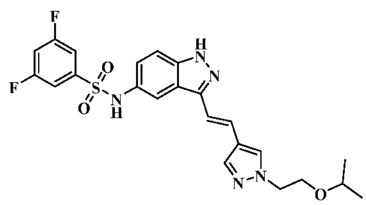
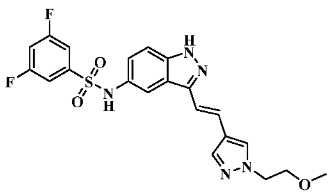
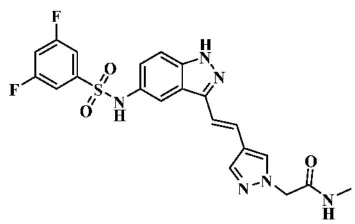
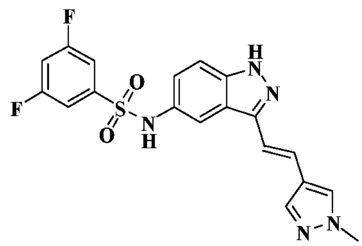
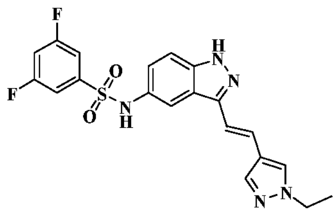
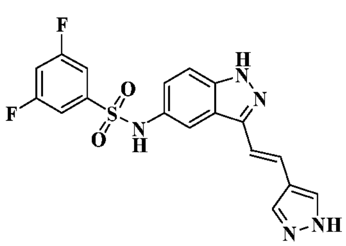
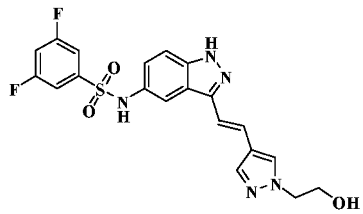

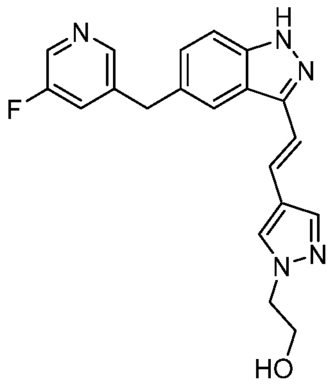
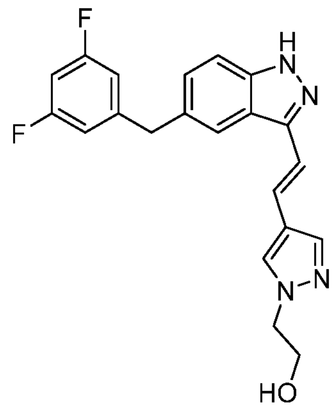
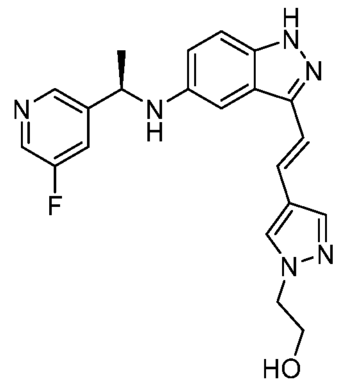
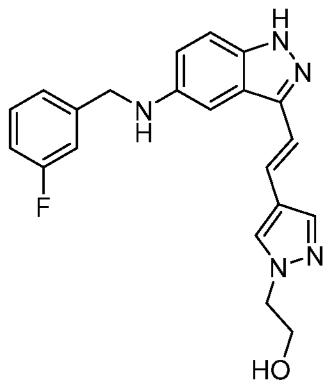
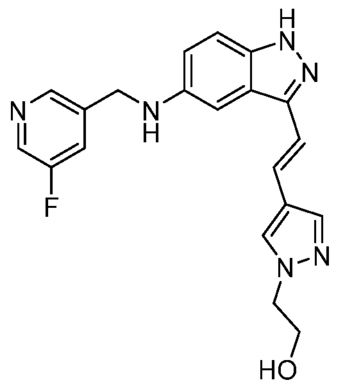
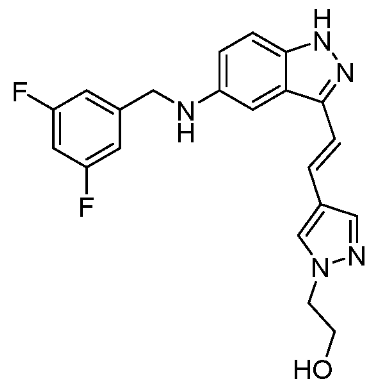
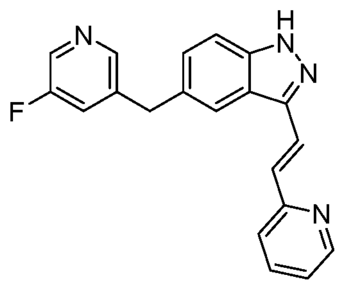
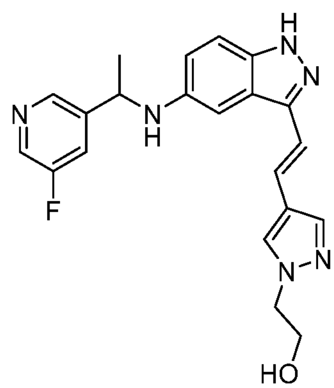

**27.** An application of the indazole derivatives or their pharmaceutically acceptable salts or stereoisomers according to any one of claims 1-26 in the preparation of tropomyosin receptor kinase (TRK) inhibitors.

**28.** An application of the indazole derivatives or their pharmaceutically acceptable salts or stereoisomers according to any one of claims 1-26 in the manufacture of a drug for preventing or treating diseases mediated by tropomyosin receptor kinase (TRK) tyrosine kinases.

**29.** The application according to claim 28, wherein the diseases mediated by TRK tyrosine kinases are tumors, preferably non-small cell lung cancer, breast cancer, colon cancer, prostate cancer, thyroid cancer, malignant melanoma, neuroblastoma and mammary analog secretory carcinoma.

**30.** A pharmaceutical composition for preventing or treating tumors prepared from an active ingredient and a pharmaceutically acceptable excipient, wherein the active ingredient includes the indazole derivatives or their pharmaceutically acceptable salts or stereoisomers according to any one of claims 1-26.

**INTERNATIONAL SEARCH REPORT**

International application No.
**PCT/CN2020/126448**

**A. CLASSIFICATION OF SUBJECT MATTER**

C07D 231/56(2006.01)i; C07D 471/00(2006.01)i; C07D 471/04(2006.01)i; C07D 487/04(2006.01)i; A61K 31/416(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 蛋白激酶, 原肌球蛋白受体激酶, 受体酪氨酸激酶, 吲唑, 暨南大学, protein kinase, tropomyosin receptor kinase, TRK, receptor tyrosine kinase, RTK, Indazole, JINAN UNIVERSITY

**C. DOCUMENTS CONSIDERED TO BE RELEVANT**

| Category* | Citation of document, with indication, where appropriate, of the relevant passages | Relevant to claim No. |
|---|---|---|
| PX | DUAN, Yunxin et al. . "Design, synthesis, and StructureeActivity Relationships (SAR) of 3-vinylindazole derivatives as new selective tropomyosin receptor kinases (Trk) inhibitors" . *European Journal of Medicinal Chemistry,* Vol. 203, 13 July 2020 (2020-07-13), pp. 1-22 | 1-30 |
| X | 王田 等 (WANG, Tian et al.). "新型TRK激酶小分子抑制剂的筛选与验证 (Screening and Verification of Small Molecular Inhibitors against Tropomyosin-Related Kinases(TRKs))" *复旦学报 (自然科学版) (Journal of Fudan University(Natural Science)),* Vol. 58, No. 2, 15 April 2019 (2019-04-15), pp. 176-182 | 1-30 |
| X | "CAS: 1348994-30-8" *STN,* 05 December 2011 (2011-12-05), pp. 1-2 | 1-20 |
| A | WU, Daichao et al. . "Crystal Structure of the FGFR4/ LY2874455 Complex Reveals Insights into the PanFGFR Selectivity of LY2874455." *PLoS One,* Vol. 11, No. 9, 12 September 2016 (2016-09-12), pp. 1-11 | 1-30 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 January 2021** | **05 February 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2020/126448

C. DOCUMENTS CONSIDERED TO BE RELEVANT

| Category* | Citation of document, with indication, where appropriate, of the relevant passages | Relevant to claim No. |
|---|---|---|
| A | CN 102421769 A (ELI LILLY AND COMPANY) 18 April 2012 (2012-04-18) abstract, and claims 1-8 | 1-30 |
| A | CN 105906621 A (SICHUAN BAILI PHARMACEUTICAL CO., LTD.) 31 August 2016 (2016-08-31) claims 1-10 | 1-30 |
| A | WO 2007058626 A1 (S. BIO PTE LTD.) 24 May 2007 (2007-05-24) claims 1-77 | 1-30 |

Form PCT/ISA/210 (second sheet) (January 2015)

**INTERNATIONAL SEARCH REPORT**
**Information on patent family members**

International application No.
**PCT/CN2020/126448**

| Patent document cited in search report | Publication date (day/month/year) | Patent family member(s) | Publication date (day/month/year) |
|---|---|---|---|
| CN 102421769 A | 18 April 2012 | JO 2860 B1 | 15 March 2015 |
| | | IL 216004 D0 | 31 January 2012 |
| | | EC SP11011441 A | 30 December 2011 |
| | | MX 2011011342 A | 18 November 2011 |
| | | SG 175909 A1 | 29 December 2011 |
| | | ME 01462 B | 20 April 2014 |
| | | UA 104756 C2 | 11 March 2014 |
| | | BR PI1013748 A2 | 05 April 2016 |
| | | DK 2427449 T3 | 22 April 2013 |
| | | PT 2427449 E | 06 May 2013 |
| | | CN 102421769 B | 02 April 2014 |
| | | EP 2427449 A1 | 14 March 2012 |
| | | AU 2010246114 B2 | 06 December 2012 |
| | | SI 2427449 T1 | 31 May 2013 |
| | | AR 078411 A1 | 09 November 2011 |
| | | TW 201105652 A | 16 February 2011 |
| | | JP 2012526126 A | 25 October 2012 |
| | | HK 1163665 A1 | 14 September 2012 |
| | | CL 2011002781 A1 | 25 May 2012 |
| | | WO 2010129509 A1 | 11 November 2010 |
| | | HN 2011002809 A | 22 July 2013 |
| | | TW I382982 B | 21 January 2013 |
| | | MY 160390 A | 15 March 2017 |
| | | US 2010286209 A1 | 11 November 2010 |
| | | CA 2760535 A1 | 11 November 2010 |
| | | ES 2408117 T3 | 18 June 2013 |
| | | IL 216004 A | 30 January 2014 |
| | | PE 20160124 A1 | 24 February 2016 |
| | | NZ 595553 A | 31 May 2013 |
| | | CR 20110580 A | 06 January 2012 |
| | | KR 101373910 B1 | 12 March 2014 |
| | | MA 33244 B1 | 02 May 2012 |
| | | CO 6450624 A2 | 31 May 2012 |
| | | EA 201171367 A1 | 30 April 2012 |
| | | JP 5555314 B2 | 23 July 2014 |
| | | KR 20120004511 A | 12 January 2012 |
| | | EA 018149 B1 | 30 May 2013 |
| | | EP 2427449 B1 | 03 April 2013 |
| | | SI EP2427449 T1 | 31 May 2013 |
| | | HR P20130323 T1 | 31 May 2013 |
| | | US 8268869 B2 | 18 September 2012 |
| | | PE 20120812 A1 | 08 July 2012 |
| | | RS 52795 B | 31 October 2013 |
| | | CA 2760535 C | 14 January 2014 |
| | | AU 2010246114 A1 | 03 November 2011 |
| | | PL 2427449 T3 | 30 August 2013 |
| CN 105906621 A | 31 August 2016 | None | |
| WO 2007058626 A1 | 24 May 2007 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERG et al.** Pharmaceutical Salts. *J. Pharm. Sci.,* 1977, vol. 66, 1-19
- **SAMBROOK et al.** Molecular Cloning: A Laboratory Manual. Cold Spring Harbor Laboratory Press, 1989